# EUROPEAN PATENT APPLICATION

(11) **EP 3 958 191 A1**
(43) Date of publication of application: **23.02.2022**
(21) Application number: 20790810.4
(22) Date of filing: 14.04.2020
(51) Int. Cl.: G06Q 10/00, G16H 10/00

(54) **SYSTEM FOR PRESENTING DAILY LIFE SCHEDULE BASED ON CIRCADIAN RHYTHM OF EACH INDIVIDUAL**

(30) Priority: 18.04.2019 JP 2019079678
(71) Applicant: Children & Future Co., Ltd., Tokyo, 158-0083 (JP); Shimura, Akiyoshi, Tokyo 158-0083 (JP)
(72) Inventor: SHIMURA, Akiyoshi, Tokyo 158-0083 (JP)
(74) Representative: Stolmár & Partner Patentanwälte PartG mbB
(86) International application number: PCT/JP2020/016435
(87) International publication number: WO 2020/213605

(57) **Abstract**

The present invention provides a system for presenting a daily life schedule based on circadian rhythm using a new index pertaining to a sleep rhythm. According to the present invention, provided is a system for presenting a daily life schedule based on circadian rhythm, the system characterized by comprising an information processing device which has a calculation unit for calculating a subjective midnight, the information processing device determines, on the basis of an answer to a question based on a sleep time zone, or measured data, whether the time-of-day data included in the answer includes time-of-day data to be excluded, the information processing device calculates a subjective midnight on the basis of the time-of-day data included in the answer or measured data, if the time-of-day data to be excluded is not included, and the information processing device calculates a subjective midnight on the basis of the time-of-day data included in the answer or measured data in which the time-of-day data to be excluded has been excluded from the time-of-day data included in the answer or measured data, if the time-of-day data to be excluded is included.

## Description

### TECHNICAL FIELD

The present invention relates to a system for representing a daily life schedule based on an individual circadian rhythm.

### BACKGROUND ART

Recent studies of Sleep Science and Chrono Biology have revealed that human beings have their respective Circadian Rhythm. "Sleep schedule" is an indicator representing the circadian rhythm. Human beings have "easy-to-sleep" and "difficult-to-sleep" times. There is a fluctuation rhythm of the outputs of the waking system nerve and the sleep maintenance system nerve associated with the circadian rhythm, which defines the timing of sleep. It is known that there is a great variety depend on the age and sex in the circadian rhythm phase (Non-Patent Literature 1), but less is known about individual differences.

To determine the phase of the circadian rhythm, there is a biological indicator such as a deep body temperature, but it is difficult to measure the deep body temperature easily. On the other hand, to grasp the circadian rhythm from the sleep-wake rhythm, it is ideal to use the intermediate time (Midpoint of Sleep) of the sleep time zone when there is no restriction in the social life, leading in a natural state, and there is no sleep debt, but it is also difficult to directly measure such time because a long holiday is required for this measurement.

A method in which the Munich ChronoType Questionnaire (MCTQ) is used as a conventional indicator of the sleep-wake rhythm for grasping a personal circadian rhythm is known (Non-Patent Literature 2). MSFsc (Sleep corrected Mid-Sleep on Free-days) is calculated using the Munich ChronoType Questionnaire, as follows.
(1) MSW (Mid-Sleep on Workdays) indicates the midpoint of a sleep time zone on weekdays.
(2) MSF (Mid-Sleep on Free-days) indicates the midpoint of a sleep time zone on holidays.
   Here, if there is an accumulation of sleep deprivation (sleep debt) on weekdays, the sleep time zone may be calculated later than the original physiological time due to an extension of the sleep time zone, and therefore, MSFsc is calculated by adjusting the sleep debt.
(3) An average sleep-time SDweek for one week is calculated. A value obtained by multiplying the number of days with work, etc. by the sleep time (SDw) of the day is added to a value obtained by multiplying the number of holidays by the sleep time (SDf) of the day, and then divided by 7. This is the SDweek.
   SDweek = (SDw × number of days with work + SDf × number of holidays)/7
(4) When SDf > SDweek, it is assumed that there is "oversleeping" due to sleep debt, and is set to MSFsc = MSF-(SDf-SDweek)/2.
(5) When SDf ≦ SDweek, it is assumed that the sleep debt is not affected, and is set to MSFsc = MSF.
   However, MSFsc has a defect described below as a method of estimating the sleep-wake schedules.

### CITATION LIST

### NON-PATENT LITERATURE

Non-patent literature1: FOSTER, Russell G.; ROENNEBERG, Till. Human responses to the geophysical daily, annual and lunar cycles. Current biology, 2008, 18.17: R784-R794.
Non-patent literature2: ROENNEBERG, Till; WIRZ-JUSTICE, Anna; MERROW, Martha. Life between clocks: daily temporal patterns of human chronotypes. Journal of biological rhythms, 2003, 18.1: 80-90.

### SUMMARY OF THE INVENTION

### TECHNICAL PROBLEM

As a result of the investigation by the present inventor, it was found that the phases of the circadian rhythm and the sleep-wake rhythm vary greatly between individuals, and have a normal distribution around the average value.

The present invention solves the above-mentioned problem, and provides a system for presenting a daily life schedule based on an individual circadian rhythm using a new index pertaining to a sleep rhythm.

### SOLUTION TO PROBLEMS

According to an embodiment of the present application, a system for presenting a daily life schedule based on circadian rhythm is provided. The system includes an information processing device having a subjective midnight calculation unit, the subjective midnight calculation unit calculating a subjective midnight; wherein the information processing device determines, on the basis of an answer to a question based on a sleep time zone, or measured data, whether time-of-day data included in the answer or the measured data includes time-of-day data to be excluded; the information processing device calculates a subjective midnight on the basis of the sleep time zone based on the time-of-day data included in the answer or measured data, if the time-of-day data to be excluded is not included; and the information processing device calculates a subjective midnight on the basis of the sleep time zone based on the time-of-day data included in the answer or measured data in which the time-of-day to be excluded has been excluded from the time-of-day data included in the answer or measured data, if the time-of-day data to be excluded is included.

The information processing device may set a flag if exception data is included in the answer or measured data, and the information processing device may calculate the subjective midnight on the basis of the sleep time zone based on the time-of-day data included in the answer or measured data which includes the exception data.

The information processing device may determine whether a time-of-day at which the least number of people fall asleep corresponding to the user's age is included in a range of the answer to the question based on a sleeping time zone, and the information processing device may issue a warning if the information processing device determines that the time-of-day at which the least number of people fall asleep is included in the answer.

The information processing device may calculate and update the time-of-data at which the least number of people fall asleep in the answer by performing aggregation using the answer or measured data.

The information processing device may determine which condition the answer for conditions corresponding to a plurality of calculation methods for calculating a required sleeping time applies to, and the information processing device may calculate the required sleeping time on the basis of the calculation method corresponding to the conditions applied to the answer.

The system for presenting the daily life schedule based on the circadian rhythm may calculate a recommended wake-up time by adding half of the required sleeping time to the subjective midnight, calculate a recommended bedtime by subtracting the required sleeping time from the recommended wake-up time, and calculate a sleep forbidden zone by adding a predetermined time to the subjective midnight on the basis of the sleep time zone.

The system for presenting the daily life schedule based on the circadian rhythm may calculate a recommended activity time for taking action for promoting falling asleep by adding or subtracting a predetermined reference value using the subjective midnight, the required sleeping time, the recommended wake-up time, or the recommended bedtime.

The information processing device may calculate the predetermined reference value by performing aggregation or regression analysis using the answer or measured data.

The information processing device may calculate a first sleepiness time zone during which sleepiness may occur by adding a first reference value having a predetermined time range at the subjective midnight on the basis of the sleep time zone, calculate a second sleepiness time zone during which strong sleepiness may occur by adding a second reference value having a narrower time range than the first reference time to the subjective midnight on the basis of the sleep time zone, and calculate a third sleepiness time zone which is likely to be a peak of sleepiness by adding a third reference value having a narrower time range than the second reference time to the subjective midnight on the basis of the sleep time zone.

The information processing device may call attention if sleep deprivation, agrypnia, or hypersomnia is determined based on the answer or measured data.

The information processing device may calculate and update the first reference value, the second reference value, and the third reference value by performing aggregation or regression analysis using the answer or measured data.

The information processing device may calculate a time zone from the time-of-day of the subjective midnight to a predetermined time as a recommended nap time zone.

The information processing device may determine whether an activity time zone is fixed to a general activity time zone, calculate a first activity time zone at which physical and mental performance improves in a first half of a day by adding a fourth reference value having a predetermined time range to the subjective midnight, if the activity time zone is fixed to the general activity time zone, calculate a second activity time zone at which physical and mental performance improves in a second half of the day by adding a fifth reference value which is set at a late time in a predetermined time range from the fourth reference value to the subjective midnight, and calculate a time zone between the first activity time zone and the second activity time zone as a recommended break time zone.

The information processing device may calculate a recommended start time at which the physical and mental performance improves by adding a sixth reference value having a predetermined time range to the subjective midnight, if the activity time zone is not fixed to a general activity time zone.

The information processing device may calculate a time zone during which a high efficacy rate of a sedative-hypnotic psychotropic drug is expected by adding a seventh reference value having a predetermined time range to the subjective midnight, calculate a time zone of taking medicine with few side effects by adding an eighth reference value that is set in a narrow time range within a predetermined time range from the seventh reference value to the subjective midnight, and calculate a first recommended time zone based on the time zone during which the high efficacy rate is expected and the time zone of taking medicine with few side effects.

The information processing device may determine whether the sedative-hypnotic psychotropic drug is an immediate release tablet, determine whether the sedative-hypnotic psychotropic drug is a delayed-action preparation, if the sedative-hypnotic psychotropic drug is not the immediate release tablet, calculate a second recommended time zone by subtracting a duration until the blood concentration or brain concentration of the drug being taken reaches an effective range or time until sleepiness actually appears after taking the drug from the first recommended time zone, if the sedative-hypnotic psychotropic drug is the delayed-action preparation, and calculate the second recommended time zone by subtracting a duration until the blood concentration or brain concentration of the drug being taken reaches an effective range or time until sleepiness actually appears after taking the drug from the first recommended time zone, if the sedative-hypnotic psychotropic drug is not the delayed-action preparation.

The information processing device may determine whether a stay period to an area with a time difference is less than 5 days, determine whether the recommended wake-up time and the recommended bedtime before making a voyage are earlier than a wake-up time and a bedtime when returning to a home country, if the stay period is less than 5 days, provide advice that the light environment at the destination may be earlier than that at a place of departure, if the recommended wake-up time and the recommended bedtime before making a voyage are earlier than the wake-up time and the bedtime when returning to the home country, and provide advice that the light environment at the destination may be later than that at the place of departure, if the recommended wake-up time and the recommended bedtime before making a voyage are later than the wake-up time and the bedtime when returning to the home country.

The information processing device may calculate a time to desire to start an activity as mealtime, calculate a time just before bedtime as a time when meals are not recommended, and set a certain fasting time in the time zone to desire to sleep.

The information processing device may determine whether there is a large deviation between the recommended wake-up time and the recommended bedtime before making a voyage and the recommended wake-up time and the recommended bedtime at the destination, calculate a first sleepiness time zone by adding a first reference value having a predetermined time range to the subjective midnight, if the deviation is determined to be large, calculate a time from the start time of the first sleepiness time zone to a predetermined time as a recommended nap time zone, and calculate a sleep forbidden zone by adding a predetermined time to the subjective midnight.

The information processing device may calculate a time in a range ±2 hours from each of the recommended bedtime and wake-up time as an allowable bedtime and wake-up time, if the deviation is determined to be small.

The information processing device may inquire to a terminal whether it makes the biological clock cycle shorter than 24 hours, if the stay period is 5 days or more, calculate a recommended light irradiation time zone based on the subjective midnight and sunrise and sunset times at the place of departure, if it is determined that making the biological clock cycle shorter than 24 hours is selected, determine whether an actual sleep-wake time zone is advanced by the light irradiation of a subjective morningness, determine whether the sleep-wake time zone is advanced enough to a range which can accommodate a schedule at the destination, if the sleep-wake time zone is actually advanced, re-calculate the subjective midnight based on the advanced sleep-wake time zone until the sleep-wake time zone is advanced enough, and re-calculate the recommended light irradiation time zone based on the re-calculated subjective midnight and the sunrise and sunset times at the place of departure.

The information processing device may calculate the recommended light irradiation time zone based on the subjective midnight and the sunrise and sunset times at the place of departure to recommend shading from the subjective midnight to sun meridian transit time and light irradiation until the subjective midnight on the basis of the sleep time zone after the sunset, if it is determined that the biological clock cycle is longer than 24 hours; determine whether the actual sleep-wake time zone has delayed; determine whether the sleep-wake time zone has delayed enough to the range which can accommodate the schedule at the destination, if the sleep-wake time zone has actually delayed; re-calculate the subjective midnight based on the delayed sleep-wake time zone until the sleep-wake time zone has delayed enough; and re-calculate the recommended light irradiation time zone based on the re-calculated subjective midnight and the sunrise and sunset times at the place of departure.

The information processing device may calculate a first recommended eating time zone which has little effect on body weight increase and relatively suppresses an increase in blood glucose levels by adding a ninth reference value having a predetermined time range to the subjective midnight; and calculate a first non-recommended eating time zone which significantly increases body weight and tends to increase blood glucose levels to a higher degree by adding a tenth reference value of a later time range than the ninth reference value to the subjective midnight.

The information processing device may calculate a second recommended eating time zone which minimizes effects on body weight increase and almost no excessive increase in blood glucose levels by adding an eleventh reference value having a predetermined time range to the subjective midnight.

The information processing device may calculate a third recommended eating time zone in which eating has little effect on body weight increase and increase in blood glucose levels by adding a twelfth reference value having a predetermined time range to the subjective midnight.

### ADVANTAGEOUS EFFECTS OF INVENTION

According to the present method, there is provided a system for presenting a daily life schedule based on the individual circadian rhythm using a new index pertaining to a sleep rhythm.

### BREF DESCRIPTION OF DRAWINGS

FIG. 1 is a schematic diagram showing a system 100 for presenting a daily life schedule based on the circadian rhythm according to an embodiment of the present invention.
FIG. 2 is a block diagram illustrating an information processing device 110 according to an embodiment of the present invention.
FIG. 3 is a flowchart illustrating processing between the information processing device 110 and a terminal according to an embodiment of the present invention.
FIG. 4 is a flowchart illustrating exclusion processing for exception data in the information processing device 110 according to an embodiment of the present invention.
FIG. 5 is a flowchart illustrating processing in the information processing device 110 based on an input error reduction method according to an embodiment of the present invention.
FIG. 6 is a schematic diagram showing an interface for preventing an input error according to an embodiment of the present invention.
FIG. 7 is a schematic diagram showing an interface for preventing an input error according to an embodiment of the present invention.
FIG. 8 is a diagram showing a relation between the deviation rate in wake-up time from SPB-SM + 3.5 hours and productivity loss according to an embodiment of the present invention.
FIG. 9 is a diagram showing an influence of light on SPB-SM of a person living in a condition without excessive light exposure at night and excessive light blocking in the morning when the person was exposed to light for one hour at every time for two consecutive days, according to an embodiment of the present invention.
FIG. 10 is a diagram showing an influence of one-hour light irradiation based on the absolute time on a sleep-wake schedule.
FIG. 11 is a flowchart illustrating calculation processing for the required sleeping time in the information processing device 110 according to an embodiment of the present invention.
FIG. 12 is a flowchart illustrating calculation processing for an optimal wake-up time/bedtime in the information processing device 110 according to an embodiment of the present invention.
FIG. 13 is a flowchart illustrating calculation processing for an optimal recommended activity time in the information processing device 110 according to an embodiment of the present invention.
FIG. 14 is a schematic diagram showing an interface 305 according to an embodiment of the present invention.
FIG. 15 (a) is a diagram showing a transition of the ratio of people who become sleepy in the daytime and the number of people who actually take a nap in the daytime relative to the absolute time (real time), and FIG. 15 (b) is a diagram showing a transition of the ratio of people who become sleepy in the daytime and the number of people who actually take a nap in the daytime relative to the relative time from SPB-SM.
FIG. 16 is a block diagram illustrating an information processing device 110A according to an embodiment of the present invention.
FIG. 17 is a flowchart illustrating calculation processing for an optimal nap time in the information processing device 110A according to an embodiment of the present invention.
FIG.18 (a) is a schematic view showing an example of an interface 401 which presents peak sleepiness to a user, and FIG. 18 (b) is a schematic view showing an example of an interface 403 which presents a recommended nap time zone to a user.
FIG. 19 is a graph showing a relation between an elapsed time from SPB-SM and the productivity loss rate (productivity loss).
FIG. 20 is a block diagram illustrating an information processing device 110B according to an embodiment of the present invention.
FIG. 21 is a flowchart illustrating calculation processing for an optimal activity time zone in the information processing device 110B according to an embodiment of the present invention.
FIG. 22 is a diagram showing elapsed time from SPB-SM and effective rates when a person with insomnia used a benzodiazepine receptor agonist.
FIG. 23 is a diagram showing elapsed time from SPB-SM and the side effect occurrence status when a person with insomnia used a benzodiazepine receptor agonist.
FIG. 24 is a block diagram illustrating an information processing device 110C according to an embodiment of the present invention.
FIG. 25 is a flowchart illustrating calculation processing for the administration time of the sedative-hypnotic psychotropic drug in the information processing device 110C according to an embodiment of the present invention.
FIG. 26 is a diagram showing a sleep diary when the light environment is kept as similar as possible to a place of departure.
FIG. 27 is a diagram showing measurement results of sleep status, activity meter, and light exposure monitor.
FIG. 28 is a block diagram illustrating an information processing device 110D according to an embodiment of the present invention.
FIG. 29 is a flowchart illustrating presentation processing for a recommended action before travel based on the circadian rhythm of each individual in the information processing device 110D according to an embodiment of the present invention.
FIG. 30 is a schematic diagram showing an interface 501 according to an embodiment of the present invention.
FIG. 31 is a schematic diagram showing an interface 503 according to an embodiment of the present invention.
FIG. 32 is a flowchart showing presentation processing for a recommended action after travel according to an embodiment of the present invention.
FIG. 33 is a schematic diagram showing an interface 505 according to an embodiment of the present invention.
FIG. 34 is a flowchart illustrating presentation processing for a recommended action based on a difference between the recommended wake-up time/bedtime calculated in the information processing device 110D and the time during which a user can rest on-the-spot.
FIG. 35 is a schematic diagram showing an interface 507 according to an embodiment of the present invention.
FIG. 36 (a) is a flowchart illustrating a method of presenting a recommended time in a case where a stay period of a user is 5 days or more, according to an embodiment of the present invention, and FIG. 36 (b) is a flowchart illustrating a method of presenting a recommended time in a case where a stay period of a user is 5 days or more, according to an embodiment of the present invention.
FIG. 37 is a flowchart illustrating presentation processing for a recommended action after traveling in a case where a stay period of a user is 5 days or more, according to an embodiment of the present invention.
FIG. 38 is a flowchart illustrating processing in a case of delaying a biological clock cycle by 24 hours and delaying a sleep-wake schedule according to an embodiment of the present invention.
FIG. 39 is a diagram showing an analysis result in which the influence of a normal subjective mealtime on body weight fluctuation is adjusted by covariance structure analysis for the correlation between mealtimes.
FIG. 40 is a diagram showing the difference between the blood glucose level at the time of ingestion and the blood glucose level 1 hour after ingestion of 40 g of carbohydrate (such as a rice ball) at any time in one day.
FIG. 41 is a block diagram illustrating an information processing device 110E according to an embodiment of the present invention.
FIG. 42 is a flowchart illustrating calculation processing for an optimal eating time in the information processing device 110E according to an embodiment of the present invention.
FIG. 43 is a schematic diagram showing an interface 601 according to an embodiment of the present invention.
FIG. 44 (a) is a diagram showing a distribution of MSFsc in the 30-39-year-old population in Japan, and FIG. 44 (b) is a diagram showing the distribution of MSFsc in a 25-29-year-old woman.

### DESCRIPTION OF EMBODIMENTS

Hereinafter, a system for presenting a daily life schedule based on an individual circadian rhythm according to the present invention will be described with reference to the drawings. The system for presenting a daily life schedule based on the individual circadian rhythm according to the present invention is not construed as being limited to the description content of the embodiment shown below. In the drawings referred to in this embodiment, the same portions or portions having similar functions are denoted by the same reference sign, and a repetitive description thereof is omitted.

### [Problem in MSFsc]

According to a survey by the present inventors, in Japanese women, MSFsc tended to advance with age: 20 to 24 years old at 4.90 hours, 25 to 29 years old at 4.62 hours, 30 to 34 years old at 4.58 hours, 35 to 39 years old at 4.20 hours, and 40 to 44 years old at 3.98 hours. These results support the conventional finding that the phase of circadian rhythm differs depending on sex and age, which is also supported by the investigation results of the present inventors.

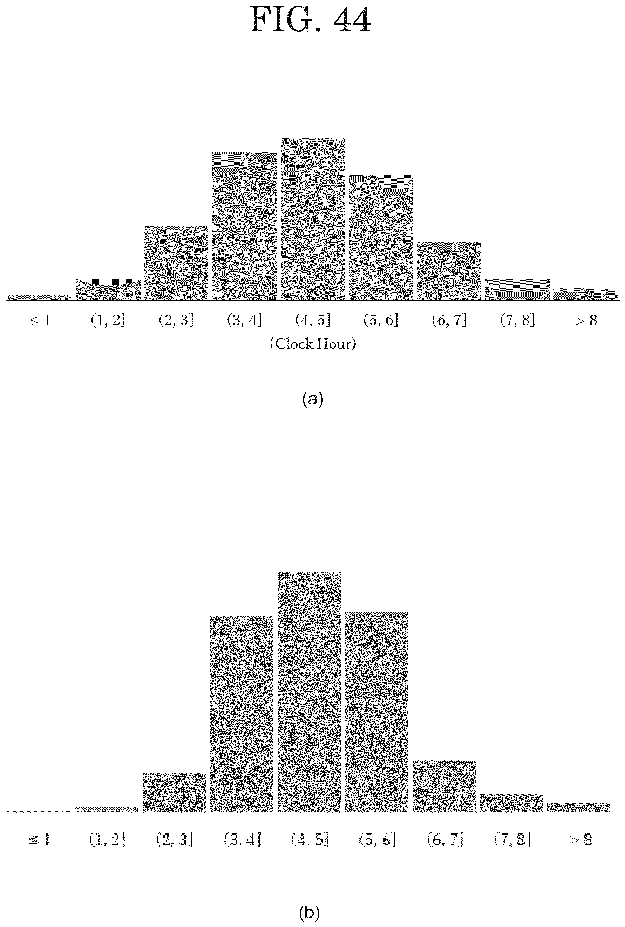

In the general population aged 30 to 39 years in Japan, the average time of MSFsc was 4.48 hours and the standard deviation was 1.59 hours (FIG. 44(a)). As shown in FIG. 44(b), the average time was 4.63 hours and the standard deviation was 1.27 hours in women aged 25 to 29 years. In other words, although there is age factor, gender factor, and individual factor among the total variation, the difference in the individual factor is larger than about a 20-year age gap or sex difference. Therefore, it is clear that the phases of the circadian rhythm and the sleep-wake rhythm are greatly different among individuals, and are normally distributed around the mean value.

MSFsc, which is one indicator of the existing circadian rhythm, has the following technical defects (A) to (C).

(A) Although MSFsc is calculated as described above, it cannot be accurately calculated in a case where there is sleep debt and bedtime is late on weekdays. For example, in a case where a person sleeps for 3 hours from 3:30 to 6:30 on weekdays and sleeps for 10 hours from 0:00 to 10:00 on holidays, MSFsc is calculated as follows.
SDweek = (3 × 5 + 10 × 2)/7 = 5 hours
MSFsc = 5:00 - (10 - 5)/2 = 2:30 because SDf>SDweek

However, although the MSW is 5:00 and the MSF is 5:00 in this case, MSFsc significantly deviates from any of these values, and exhibits a strong morningness value of 2:30. In addition, MSFsc in this case does not coincide at all with the lowest point of the deep body temperature.

(B) Currently, in a case of insomnia, i.e., in a case where sleep is severely impaired for some reason or in a case of early morning awakening, or in a case where the awaking time is pathologically delayed due to hypersomnia, the recent sleep-wake time does not necessarily indicate the sleep rhythm of the individual.

(C) Many people usually have some schedule in a holiday affecting wake-up time or bedtime, or a home environment in which they are wake up by a family member and the like. For this reason, it is impossible to assume that the recent holiday reflects the individual biological clock well.

Therefore, MSFsc simply calculated only from the most recent sleep time zone is an indicator which cannot sufficiently accurately reflect the circadian rhythm in many cases.

### [System for presenting daily life schedule based on circadian rhythm]

A system for presenting a daily life schedule based on the circadian rhythm according to an embodiment of the present invention is characterized in that it estimates the Midpoint of Sleep using a computer system or paper, and uses it as a reference for calculating various body events which occur depending on the phase of the body time or recommended activity times. In an embodiment, the system for presenting a daily life schedule based on the individual circadian rhythm calculates "subjective midnight on the basis of the sleep time zone" (referred to as SPB-SM: Sleep Phase Based Subjective Midnight).

Other representative indicators representative of circadian rhythm include circadian variations in a deep body temperature, pulse, blood pressure, parasympathetic activity (HF), and sympathetic and parasympathetic activity ratios (LF/HF), all of which fall at subjective night. As a result of the investigation by the present inventor, it was clarified that there exists the lowest point region of the deep body temperature, pulse and blood pressure in the SPB-SM, and that this SPB-SM was strongly correlated with the lowest point time region of the deep body temperature, pulse and blood pressure, and the correlative coefficient and the degree of coincidence were higher than that of the known indicator of MSFsc by the measurements using a plurality of subjects. That is, SPB-SM is extremely reliable as an indicator of circadian rhythms in the body.

In the present specification, a SBP-SM may be also defined as a "time zone" having a certain range. In this case, the time zone during which various actions are proposed based on the biological clock is presented as a distribution having a certain range from the minimum value to the maximum value.

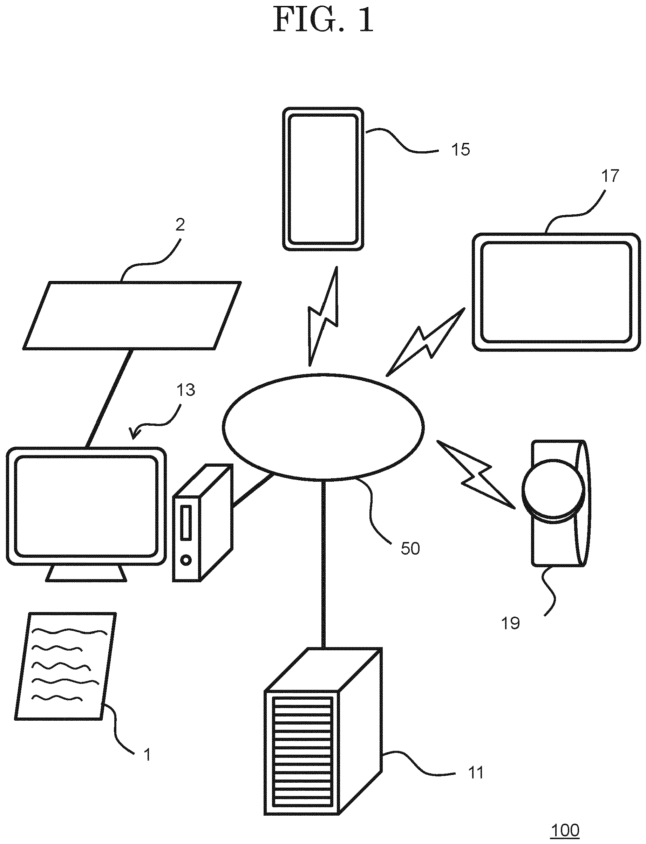

FIG. 1 is a schematic diagram showing a system 100 for presenting a daily life schedule (hereinafter, also referred to as a system 100) based on the individual circadian rhythm according to an embodiment of the present invention. The system 100 includes, for example, an information processing device 110. As the "information processing device" herein, for example, a terminal which can be directly operated by a user such as a personal computer (PC) 13, a mobile phone 15 including a smartphone, a tablet terminal 17 and a wearable device 19, and a server 11 are exemplified, but are not limited thereto, and may be a device including an input device and a display device. In an embodiment, the server 11 may be used as the information processing device 110. As described below, the PC 13 may be used to input answers set forth in a questionnaire 1. A sensor 2 may be used to transmit data to the information processing device 110 directly or via a terminal such as the PC 13. A known sensor capable of monitoring a sleep state such as, a pulse meter, a heart rate meter, or a deep body thermometer can be exemplified as the sensor 2. These sensors 2 may be arranged directly on the body surface of a user, or they may be used as a device for mounting a sensor mat having one or more sensors on bedclothes. The information processing device 110 may be connected to a network 50. The network 50 represents the so-called Internet and is a communication network which can be connected to the information processing device 110 and the terminal by wired or wireless communication. For example, one or more of the terminals described above may be connected to the server 11 via the network 50.

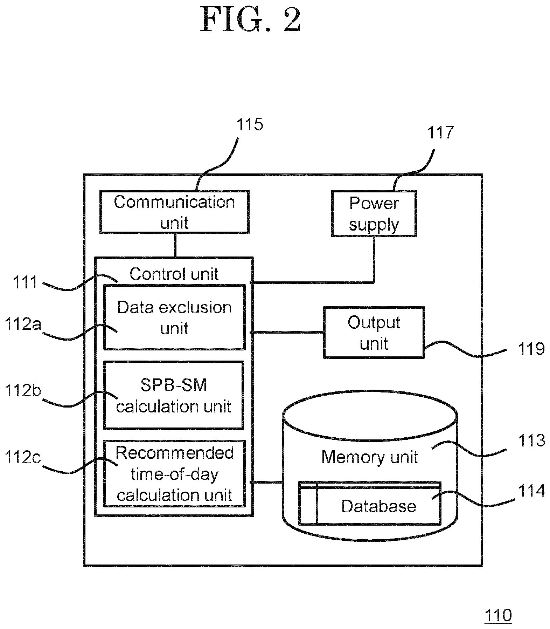

FIG. 2 is a block diagram illustrating the information processing device 110 according to an embodiment of the present invention. The information processing device 110 includes, for example, a control unit 111, a storage unit 113, a communication unit 115, and a power supply 117, and may further include an output unit 119. The control unit 111 is composed of, for example, a central processing unit (CPU), an operating system (OS), an application program or a module and the like which controls the information processing device 110. The storage unit 113 is composed of auxiliary storage device such as a hard disk and a solid-state disk (SSD). The storage unit 113 stores a database 114 containing the calculated SPB-SM. The communication unit 115 is composed of a network adapter or an on-board communication chip, and the like, and can communicate with a terminal via the network 50. The power supply 117 is a power supply device which supplies power from the outside to the respective devices in the information processing device 110, and is not particularly limited. The output unit 119 may be a display device or a printer, and a known device may be arbitrarily arranged as the output unit 119. In addition, the information processing device 110 may also include various electronic devices included in the known information processing device.

SPB-SM may be calculated by answers to the questionnaire 1 presented by the system 100 or by data obtained from a measuring device, e.g., the wearable device 19. The questionnaire 1 contains questions based on a sleep time zone such as the living environment and life rhythm of a user. The answers to the questionnaire 1 include information including SPB-SM such as the user's living environment and living rhythm and time data such as bedtime and wake-up time for calculating the recommended time based on SPB-SM. The measured data obtained from the measuring device includes data capable of monitoring the sleep state of the user and the measured time data or information of time zone.

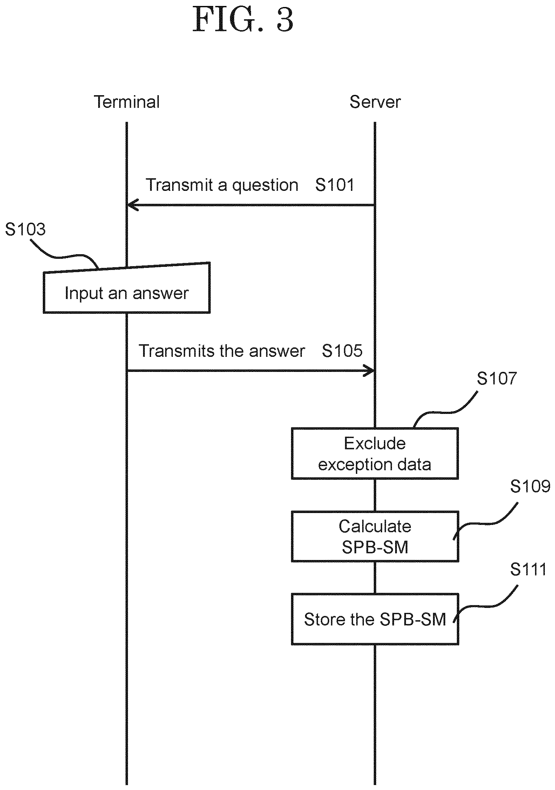

FIG. 3 is a flow diagram illustrating processing for the information processing device 110 according to an embodiment of the present invention. In each of the following embodiments, an example in which the server 11 is used as the information processing device 110 is shown, but the system 100 for presenting a daily lie schedule based on the circadian rhythm according to the present invention is not limited thereto, and the terminal may be used as the information processing device 110 and the following processing may be performed by the terminal alone. The information processing device 110 transmits a question to the terminal to calculate SPB-SM(S101). When the terminal includes a display device and an input device such as the PC 13, the mobile phone 15, the tablet terminal 17, and the wearable device 19, the terminal displays the question received from the information processing device 110 on the display device to prompt the user to answer. In the case where a printer is connected to the PC 13, the mobile phone 15, or the tablet terminal 17 by wired or wireless connection, the question received from the information processing device 110 can be printed by the printer, and the questionnaire 1 can be created.

The user inputs an answer to the terminal (S103). The terminal transmits the input answer to the information processing device 110 via the network 50 (S105). In the case where the user inputs the answer on the questionnaire 1, a person (medical professional, etc.) who made the user answering the question may input the answer wrote into the questionnaire 1 into the terminal. In the case where data is measured using a device to which a sensor mat 2 or the like is attached or a terminal to which the wearable device 19 or the like is attached, the terminal or the device transmits the measured data to the information processing device 110 via the network 50.

When the information processing device 110 receives an answer or measured data, the information processing device 110 verifies whether the received answer or measured data contains exception data, and in the case where exception data is included, excludes the exception data from the received answer or measured data (S107).

The information processing device 110 calculates SPB-SM based on the answer and the measured data excluding the exception data (S109). The information processing device 110 stores the calculated SPB-SM in the storage unit 113 (S111).

### [Exclusion of exception data]

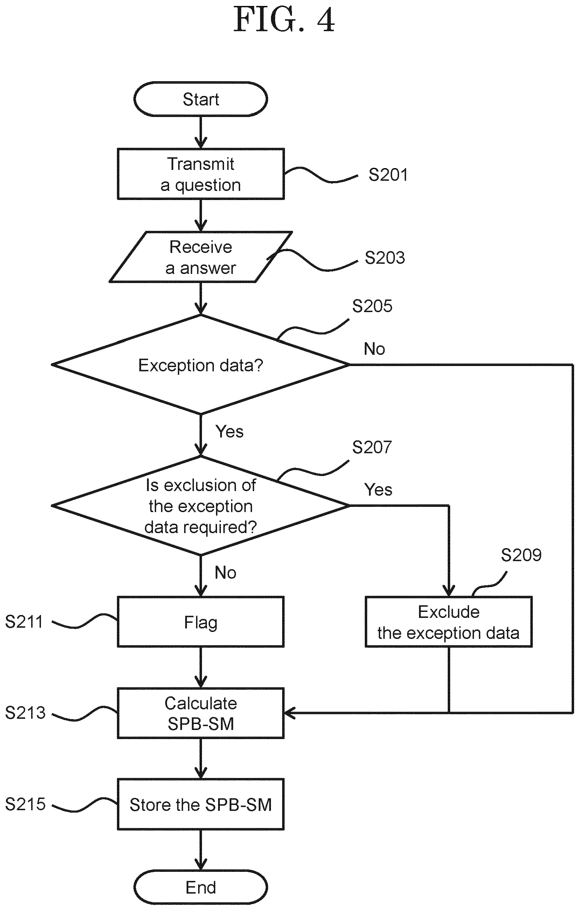

In the case where any of the following conditions (1) to (6) is satisfied, SPB-SM cannot be calculated from the sleep-wake rhythm. FIG. 4 is a flowchart illustrating exclusion processing for exception data in the information processing device 110 according to an embodiment of the present invention. The control unit 111 reads the questions regarding the conditions (1) to (6) stored in the storage unit 113, and transmits the question to the terminals such as the PC 13, the mobile phone 15, the tablet terminal 17, and the wearable device 19 from the communication unit 115 via the network 50 (S201). In the case where the questionnaire 1 is used, questions about the conditions (1) to (6) may be printed in advance. When the user inputs an answer to the question in the terminals such as the PC 13, the mobile phone 15, the tablet terminal 17, or the wearable device 19, the communication unit 115 receives the answer to the question displayed on the display device of the terminal or data obtained from the measuring device from the terminal (S203). In the case where the user answers the questionnaire 1, the person (medical professional, etc.) who made the user answer the question may input the answer wrote into the questionnaire 1 to the terminal.

The system 100 includes a data exclusion unit 112a which excludes exception data corresponding to the conditions (1) to (6). The data exclusion unit 112a is composed of an application program, module, or the like which excludes exception data from the answer to the question displayed on the questionnaire 1 or the display device of the terminal, or data obtained from the measuring device. The data exclusion unit 112a may be, for example, an application program or module stored in the storage unit 113 and executed by the control unit 111. The data exclusion unit 112a determines whether the obtained answer or measured data corresponds to any of the exception data of the conditions (1) to (6) (S205). In the case where it does not correspond to the exception data, the data exclusion unit 112a transfers the answer to the question or the data obtained from the measuring device to an SPB-SM calculation unit 112b, and the SPB-SM calculation unit 112b calculates SPB-SM (S213).

When an answer indicating that the data corresponds to any of the exception data is obtained from the terminal, the data exclusion unit 112a determines whether the exclusion of the exception data is required according to the conditions (1) to (6) (S207). In the case where it is determined that the exception data needs to be excluded, the data exclusion unit 112a excludes the answer and the measured data as the exception data from the obtained answer and the measured data (S209).

### Condition (1)

Since a phase in a human biological clock (in particular, a central clock of the suprachiasmatic nucleus) is controlled by light, in the case where the light-dark environment is excessively different from the outside world, it causes external desynchronization (so-called jet lag) in which the time of the biological clock and the time of the outside world are different. For this reason, under the condition (1) of excessive shading in the morning (e.g., not lightening the room at all after sunrise using rain doors or shading curtains and spending a long time in the day in a dark room) or excessive light exposure at night (e.g., living or working with high illumination even at midnight), the system 100 receives an answer whether the condition (1) is satisfied, and if so, the data exclusion unit 112a excludes the received answer from the data used for calculating SPB-SM (S209).

However, in the case where only data under the condition corresponding to condition (1) can be obtained due to being engaged in shift work or working at extremely early or late hours, or in the case where the majority of data is obtained under the condition corresponding to condition (1), the data exclusion unit 112a also treats the data obtained under these conditions as the data to be used for calculating SPB-SM. In this case, the data exclusion unit 112a may set a flag indicating that "a time which may differ from the original biological rhythm, is affected by the living environment and the light environment" (S211).

### Condition (2)

When having a psychiatric disorder such as a sleep or a hypersomnia-causing sleep disorder, mood disorder (depression or bipolar disorder), and psychotic symptom (such as schizophrenia), especially when these symptoms are severe, sleep and wake are less likely to be maintained by the biological clock, grasping physiological sleep time is difficult due to sleep difficulty, interrupted sleep, and waking in the early morning. For this reason, under the condition (2) of having the sleep disorder or the psychiatric disorder which causes sleep or hypersomnia which has not been remitted yet, the system 100 receives an answer whether the condition (2) is satisfied, and if so, the data exclusion unit 112a excludes the received answer from the data used for calculating SPB-SM (S209).

However, in the case where there is no data in a period which does not satisfy the condition (2), the data obtained under the condition (2) is also treated as the data used for calculating SPB-SM. In this case, the data exclusion unit 112a may set a flag indicating "a time which may be different from the original biological rhythm affected by the sleep disorder/psychiatric disorder.

### Condition (3)

In the non-24-hour type sleep-wake rhythm disorder (non-24) in which the sleep-wake rhythm does not maintain a 24-hour circadian rhythm, the sleep-wake rhythm shifts in about 25 hours, and therefore, it is impossible to apply an SPB-SM calculation method according to the present invention. Therefore, under the condition (3) of having the non-24-time type sleep-wake rhythm disorder, the system 100 receives an answer whether the condition (3) is satisfied, and the data exclusion unit 112a excludes the received answer from the data used for calculating SPB-SM (S209).

### Condition (4)

Immediately after a voyage from an area with different time zones, the biological clock does not synchronize with the local time due to jet lag. For this reason, under the condition (4) that there is a recent movement between different areas in standard times, the system 100 receives an answer whether the condition (4) is satisfied, and the data exclusion unit 112a excludes the received answer from the data used for calculating SPB-SM (S209).

### Condition (5)

In the case of ingesting psychotropic drugs (arousal drugs, antidepressants) which have an effect to delay sleep-onset time or excessive wakefulness-inducing substances such as caffeine on the day of the measurement, physiological sleep-onset time cannot be obtained. Therefore, under the condition (5) of ingesting psychotropic drugs or excessive wakefulness-inducing substances, the system 100 receives an answer whether the system 100 satisfies the condition (5), and the data exclusion unit 112a excludes the received answer from the data used for calculating SPB-SM (S209).

However, in the case where there is no data in a period which does not satisfy the condition (5), the data obtained under the condition (5) is also treated as the data used for calculating SPB-SM. In this case, the data exclusion unit 112a may set a flag indicating "a time which may be different from the original biological rhythm affected by a stimulating substance" (S211).

### Condition (6)

In the case of ingesting psychotropic drugs (hypnotics and sedatives, antidepressants, anxiolytic agents, and antipsychotic agents) which advance sleep-onset time or excessive alcohol on the day of the measurement, physiological sleep-onset time cannot be obtained. Therefore, under the condition (6) of ingesting psychotropic drugs or excessive alcohol, the system 100 receives an answer whether the system 100 satisfies the condition (6), and the data exclusion unit 112a excludes the received answer from the data used for the calculating SPB-SM (S209).

However, in the case where there is no data in a period which does not satisfy the condition (6), the data obtained under the condition (6) is also treated as the data used for calculating SPB-SM. In this case, the data exclusion external 112a may set a flag indicating "a time which may be different from the original biological rhythm affected by a substance which has a sleep-onset effect" (S211).

The data excluding the exception data may be transferred from the data exclusion unit 112a to the SPB-SM calculation unit 112b, or may be temporarily stored in the storage unit 113.

### [SPB-SM calculation method]

The SPB-SM calculation unit 112b is composed of an application program, module, or the like which calculates SPB-SM based on the data obtained from the data exclusion unit 112a. The SPB-SM calculation unit 112b may be, for example, an application program or module stored in the storage unit 113 and executed by the control unit 111. The SPB-SM calculation unit 112b calculates SPB-SM based on the data obtained from the data exclusion unit 112a (S213). The storage unit 113 stores the calculated SPB-SM in the database 114 (S215).

### [SPB-SM calculation method 1]

The SPB-SM calculation unit 112b calculates, from the answers to the questionnaire 1 or the data obtained from the measuring device, a distribution of the Midpoint of Sleep at the elapse of two weeks or the Midpoint of Sleep after the elapse of two weeks or the average time thereof as SPB-SM if there is a period when there is no event or social schedule which affects the sleeping or waking for two weeks or more and the sleep during the period is sufficiently achieved (S213).

### [SPB-SM calculation method 2]

A phenomenon of "over-slept" as a solution to sleep debt lasts for 2 weeks but is particularly intense in the first 3 days and greatly alleviated after day 4 (Kitamura, Shingo, et al. "Estimating individual optimal sleep duration and potential sleep debt." Scientific reports 6 (2016): 35812). For this reason, the SPB-SM calculation unit 112b calculates, from the answer to the questionnaire 1 or the data obtained from the measuring device, a distribution of the Midpoint of Sleep on day 4 or the Midpoint of Sleep after day 4, or the average time thereof as SPB-SM if there is a recent period with no event or social schedule which affects the sleeping or waking continuously for two weeks or more and the period of sufficient sleep during the period is less than two weeks, but there are consecutive holidays for four days or more with no social schedule which affects the sleeping or waking (S213).

### [SPB-SM calculation method 3]

In the case where there are no recent consecutive holidays for 4 days or more with no social schedule that affects the sleeping or waking, the SPB-SM calculation unit 112b calculates SPB-SM at the age of the person at which the answer was obtained based on an SPB-SM calculation method 1-1 from the answers to the questionnaire 1 or the data obtained from the measuring device in the range that can be traced back, including when the person was young, while in school, or the like.

Alternatively, the SPB-SM calculation unit 112b calculates SPB-SM at the age of the person at which the answer was obtained, based on a SPB-SM calculation method 1-2, from the answer to the questionnaire 1 or the data obtained from the measuring device in the range that can be traced back, including when the person was young, while in school, or the like.

In the late period of adolescence, the phase of the human biological clock and the Midpoint of Sleep tend to be the most delaying (eveningness), and thereafter to be the progression (morningness) with age. The inventor's investigation here has revealed that individual differences, which are the deviation rate from the distributions of the average SPB-SM defined by a given age point and sex, are maintained even after changes due to aging. That is, the deviation-value score (Z-score) for the average SPB-SM of the age at a certain age does not change significantly with aging.

Therefore, the SPB-SM calculation unit 112b may convert SPB-SM at that time calculated by SPB-SM calculation method 1-1 or the SPB-SM calculation method 1-2 to a deviation value based on the most recent answer to the questionnaire 1 or the data obtained from the measuring devices based on the age and gender, and may use this as SPB-SM at the present time calculated from the average SPB-SM based on the age and gender at the present time (S213).

For example, in a 30-year-old woman residing in Tokyo, Japan, in the case where the day on which the woman took a holiday for one week consecutively, which existed during the summer vacation at a university at the age of 20, meets the most recent above-mentioned condition, the SPB-SM calculation unit 112b calculates SPB-SM for that period, then converts it to a deviation value, and calculates the present SPB-SM. If the time obtained from the SPB-SM calculation method 1-2 at the age of 20 for this woman was 6.3 o'clock (6:18), this corresponds to a value deviated from 1 standard deviation (+1σ), so the deviation is 60. If this is applied at the age of 30, SPB-SM is 5.9 o'clock (5:54).

However, the average time of this SPB-SM varies from region to region. Even in the same country, in the eastern part of the country, the average time of SPB-SM advances due to the influence of early sunrise and sunset, and in the western part of the country, the average time of SPB-SM delays due to the influence of late sunrise and sunset. Therefore, the deviation value may be calculated for each region, or the information processing device can update the average time which is the basis of the calculation by storing the data.

In the SPB-SM calculation methods 1-3, a plurality of SPB-SM candidates may be obtained by answering sleep-wake time zones at a plurality of ages. In such cases, it is preferable to use the distribution thereof or the average time thereof as SPB-SM.

### [SPB-SM calculation method 4]

In the case where there is a sleep time zone of each day estimated by a mobile terminal equipped with an acceleration sensor or a device (e.g., the wearable device 19 such as an actigraph or a smart watch, a mobile phone terminal 15, or the sensor mat 2) to be attached to a human body, clothes, and bedding, or in the case where there is a measured value of a pulse and a heart rate, or a measured value of a deep body temperature or an estimated value thereof, the SPB-SM calculation unit 112b calculates SPB-SM by the following calculation methods (S213).

In the case where there is data for grasping whether the day (wearing date) when the user has worn the wearable device 19 or the like is a scheduled day (weekday, etc.) or a holiday, the SPB-SM calculation unit 112b can collate the data and calculate SPB-SM according to the SPB-SM calculation method 1-1 or the SPB-SM calculation method 1-2.

In the case where there is no data for grasping whether the day (wearing date) when the user has worn the wearable device 19 or the like is a scheduled day (weekday, etc.) or a holiday, the SPB-SM calculation unit 112b may set the average time of the Midpoint of Sleep in the obtainable period as SPB-SM.

In the case where the pulse and the heart rate have been measured by the sensor 2, the SPB-SM calculation unit 112b determines the time at which the minimum value of the pulse and the heart rate occurs during the obtainable period as SPB-SM. In the case where there is a range in the time zone in which the minimum value of the pulse and the heart rate occurs, the SPB-SM calculation unit 112b determines the intermediate time thereof as SPB-SM.

In the case where the deep body temperature or its estimated value has been measured by the sensor 2, the SPB-SM calculation unit 112b determines the time at which the minimum value of the deep body temperature occurs during the obtainable period as SPB-SM. In the case where there is a range in the time zone during which the minimum value occurs, the SPB-SM calculation unit 112b determines the intermediate time thereof as SPB-SM.

### [SPB-SM calculation method 5]

A plurality of SPB-SMs may be obtained by the above SPB-SM calculation method 1-1, the SPB-SM calculation method 1-2, the SPB-SM calculation method 1-3, and the SPB-SM calculation method 1-4. In this case, the SPB-SM calculation unit 112b may determine SPB-SM by any one of the following methods (1) to (5) (S213).
(1) The SPB-SM calculation unit 112b determines SPB-SM as a distribution of ranges from its minimum value to the maximum value.
(2) The SPB-SM calculation unit 112b determines the average time of SPB-SM as SPB-SM.
(3) The SPB-SM calculation unit 112b determines an average (trim mean) obtained by excluding the time as the exclusion data as SPB-SM.
(4) The SPB-SM calculation unit 112b calculates the physiological wake-up time based on the user's personal biological clock by the optimal wake-up time/bedtime calculation method based on the individual circadian rhythm, which will be described later, from the respective SPB-SM, method of (2), method of (3), and time of SFsc, and causes the user to answer at which time the user may naturally wake-up. In the case where the user inputs an answer indicating that a single time matches, the SPB-SM calculation unit 112b determines the answered single time as SPB-SM. On the other hand, in the case where the user inputs an answer indicating that a plurality of times match, the SPB-SM calculation unit 112b determines the average of the answered plurality of times as SPB-SM.
(5) The SPB-SM calculation unit 112b calculates the "time at which sleepiness in the daytime occurs" by a method of calculating the sleepiness timing based on the individual circadian rhythm, which will be described later, from the respective SPB-SM, the method of (2), the method of (3), and time of MSFsc, and causes the user to answer which of them matches the most present actual situation. In the case where the user inputs an answer indicating that a single time matches, the SPB-SM calculation unit 112b determines the answered single time as SPB-SM. On the other hand, in the case where the user inputs an answer indicating that a plurality of times match, the SPB-SM calculation unit 112b determines the average of the answered plurality of times as SPB-SM.

### [SPB-SM calculation method 6]

In the case where neither the data nor the result of any of the above-described SPB-SM calculation method 1-1 to the above-described SPB-SM calculation method 1-5 is obtained, the SPB-SM calculation unit 112b determines SPB-SM by any one or a plurality of methods described below (S213). In this case, to indicate that the value is inaccurate, the SPB-SM calculation unit 112b may set a flag indicating that the value is a temporary value and store the calculated SPB-SM in the database 114 (S215).
(1) MSFsc is defined as SPB-SM.
(2) The average time of the Midpoints of Sleep during the measurement/answer period is defined as SPB-SM.
(3) The Midpoint of Sleep on holiday is defined as SPB-SM.
(4) The system 100 may display the following options on the terminal: "If anything, you are (a) Morningness, (b) Neither, (c) Eveningness". In the case where (a) is selected, SPB-SM may be 2:30, in the case where (b) is selected, SPB-SM may be 4:00, and in the case where (c) is selected, SPB-SM may be 5:30. Although the respective specified times can be changed by a system administrator, it is preferable that (a) is within the range of 0:00 to 4:00, (b) is within the range of 3:00 to 5:00, and (c) is within the range of 4:00 to 8:00.
(5) The system 100 may display the following options on the terminal: "If anything, you are (a) Very morningness, (b) morningness, (c) Neither, (d) Eveningness, (e) Very eveningness". In the case where (a) is selected, SPB-SM may be 2:00, in the case where (b) is selected, SPB-SM may be 3:00, in the case where (c) is selected, SPB-SM may be 4:00, in the case where (d) is selected, SPB-SM may be 5:00, and in the case where (e) is selected, SPB-SM may be 6:00. Although the respective specified times can be changed by the system administrator, it is preferable that generally (a) and (b) are within the range of 22:00 to 4:00, (c) is within the range of 3:00 to 5:00, and (d) and (e) are within the range of 4:00 to 10:00.

### [SPB-SM calculation method 7]

The answerer (user) or the person who made the user answer the question (healthcare professional, etc.) may specify which data is preferentially used to calculate as an index of the biological clock. The system 100 may present the person with which of "(a) No particular specification", "(b) The original biological rhythm", or "(c) The biological rhythm in the most recent life" is prioritized. In a case where the system 100 did not let the user select anything, or nothing was selected by the user, the SPB-SM calculation unit 112b selects data (a). The SPB-SM calculation unit 112b calculates the data (a) to (c) by the following calculation methods (S213).
(a) Calculates SPB-SM according to SPB-SM calculation methods 1-1 to 1-6.
(b) The circadian rhythm is defined by light. For this reason, the SPB-SM calculation unit 112b instructs the user to input the answers to be used for the SPB-SM calculation methods 1-1 to 1-6 when the user could be exposed to the morning sunlight and the user has not been exposed to artificial light after sunset or spent in environments with very low illuminance after sunset.
(c) The SPB-SM calculation unit 112b causes the user to input how much the most recent period is desired to measure the rhythm and requests the terminal to input the answers during that period to be used in the SPB-SM calculation methods 1-1 to 1-6.

The SPB-SM calculation unit 112b calculates SPB-SM by any of the above means or combinations thereof, and the SPB-SM calculation unit 112b stores the calculated SPB-SM in the memory of the control unit 111 or in the database 114 of the storage unit 113 (S215). In the present embodiment, the data exclusion unit 112a and the SPB-SM calculation unit 112b are described as separate programs or modules, but the present invention is not limited to this, and the SPB-SM calculation unit 112b may include the data exclusion unit 112a, and the SPB-SM calculation unit 112b may perform exclusion processing for the exception data.

### [Input error reduction method]

In the conventional system or program for measuring the time related to sleep and wake-up similar to the system 100 according to the embodiment of the present invention, when the bedtime or wake-up time is input to the system or program, an error frequently occurs because humans input the wake-up time, bedtime, or alarm time to the system or program via an interface, rather than being automatically input from some device.

For example, even if any user goes to bed at 22:00 at night (10 p.m.), the user may input that the user went to bed at 10:00 (10 a.m.) in the interface. In some cases, the user may attempt to input a non-existent time (e.g., 30:00).

As a result of the investigation by the present inventor, in an input method which causes the user just to input numerical values, it has been clarified that about 5% of general adults (n=10,016) generate the above-mentioned erroneous answers, and about 6% of high school students (n=360) generate the above-mentioned erroneous answers.

If an erroneous answer occurs, it is impossible to grasp the correct sleep time zone. In the case where an alarm function is used, the system or program cannot be activated at the time originally desired by the user. Thus, in an embodiment, the system 100 preferably includes a means for reducing input errors.

The present inventor investigated the proportion of people sleeping (excluding sleep breaks and daytime naps) at that time in the general population. As a result, around 16 o'clock was the time when the number of people sleeping by the main sleep was the smallest on weekdays and holidays in the whole population. However, this time varies according to age: 15 o'clock at age 9 or younger, 16 o'clock at age 10 to 14, 17 o'clock at age 15 to 24, 16.5 o'clock at age 25 to 34, 16 o'clock at age 35 to 44, 15.5 o'clock at age 45 to 54, 15 o'clock at age 55 to 64, and 14.5 o'clock at age 65 or older.

Therefore, in the case where the age of the user who inputs the sleep time is known in advance, if the sleep time zone in which the user answered or the time zone in which sleep is scheduled (e.g., when using the alarm function) includes 15 o'clock at age 9 or younger, 16 o'clock at age 10 to 14, 17 o'clock at age 15 to 24, 16.5 o'clock at age 25 to 34, 16 o'clock at age 35 to 44, 15.5 o'clock at age 45 to 54, 15 o'clock at age 55 to 64, and 14.5 o'clock at age 65 or older, it is highly likely an erroneous answer. When the system 100 receives an answer to a question displayed on the display device of the terminal or data obtained from the measuring device from the terminal, and the data exclusion unit 112a determines that the data is proper data, the SPB-SM calculation unit 112b may perform an aggregation or regression analysis using the data determined as proper data, and calculate the time at which the answer is likely to be an erroneous answer. Alternatively, when the answer to the question displayed on the display device of the terminal or the data obtained from the measuring device is received from the terminal and the data exclusion unit 112a determines that the data is proper data, the storage unit 113 may store the data determined as proper data, and the data exclusion unit 112a may periodically perform an aggregation or regression analysis using the stored data to calculate the time at which the answer is likely to be an erroneous answer. The aggregation or regression analysis may be configured to be performed by the data exclusion 112a or the control unit 111 may include an application program or module (aggregation or regression analysis unit) or the like to perform the aggregation or regression analysis.

In the system 100 according to the present embodiment, when such a time is input to the data exclusion unit 112a, the SPB-SM calculation unit 112b issues an alert (warning) for confirming "whether the user really wants to go to sleep at the time" or "whether the user really wants to wake up at the time".

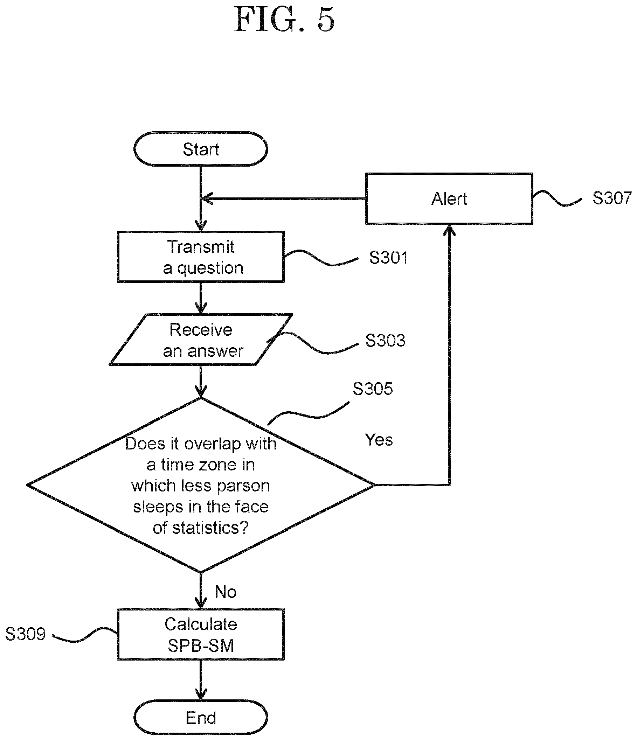

FIG. 5 is a flowchart illustrating processing in the information processing device 110 based on the input error reduction method according to an embodiment of the present invention. As described in the above embodiment, the control unit 111 reads the questions stored in the storage unit 113, and transmits the questions to the terminals such as the PC 13, the mobile phone 15, the tablet terminal 17, and the wearable device 19 from the communication unit 115 via the network 50 (S301). When the user inputs an answer to a question to the terminals such as the PC 13, the mobile phone 15, the tablet terminal 17, or the wearable device 19, the communication unit 115 receives the answer to the question displayed on the display device of the terminal or data obtained from the measuring device from the terminal (S303). In the case where the user answers the questionnaire 1, the person who made the user answer the question (medical professional, etc.) may input the answer wrote into the questionnaire 1 to the terminal.

The data exclusion unit 112a determines whether the received answer to the question or the data obtained from the measuring device includes a time that is likely to be an erroneous answer described above (S305). In a case where the received answer to the question or the data obtained from the measuring device includes a time that is likely to be an erroneous answer, the SPB-SM calculation unit 112b issues an alert for confirming "whether the user really wants to go to sleep at the time" or "whether the user really wants to wake up at the time" (S307). In the case where the received answer to the question or the data obtained from the measuring device does not include a time that is likely to be an erroneous answer, the SPB-SM calculation unit 112b calculates SPB-SM based on the received answer to the question or the data obtained from the measuring device (S309).

In the case where the age of the user who inputs the sleep time is not known in advance, and if the answered sleep time zone or the time zone during which sleep is scheduled includes any time from 15 o'clock to 17 o'clock, the data exclusion unit 112a and the SPB-SM calculation unit 112b may issue an alert for confirming "whether the user really wants to go to sleep at the time" or "whether the user really wants to wake up at the time".

### [Interface to prevent input errors]

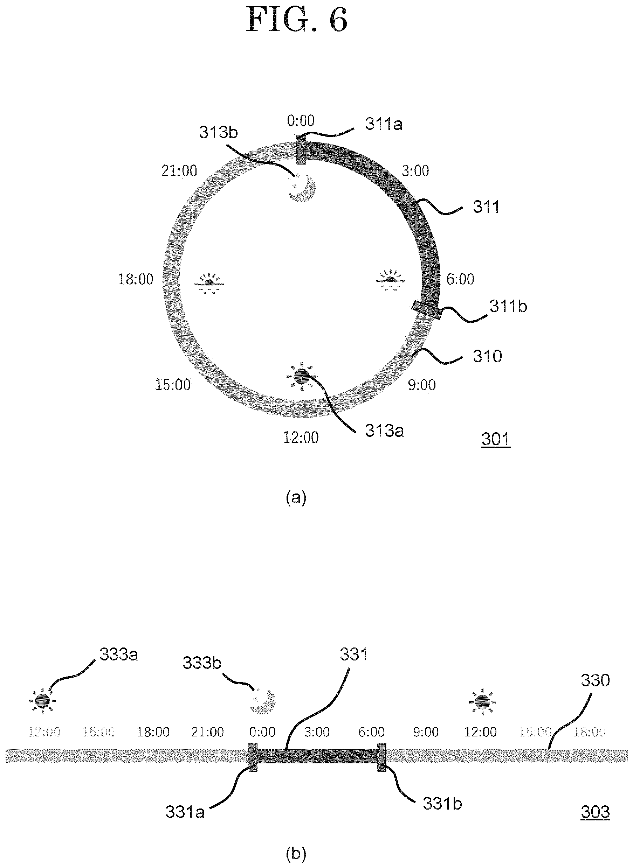

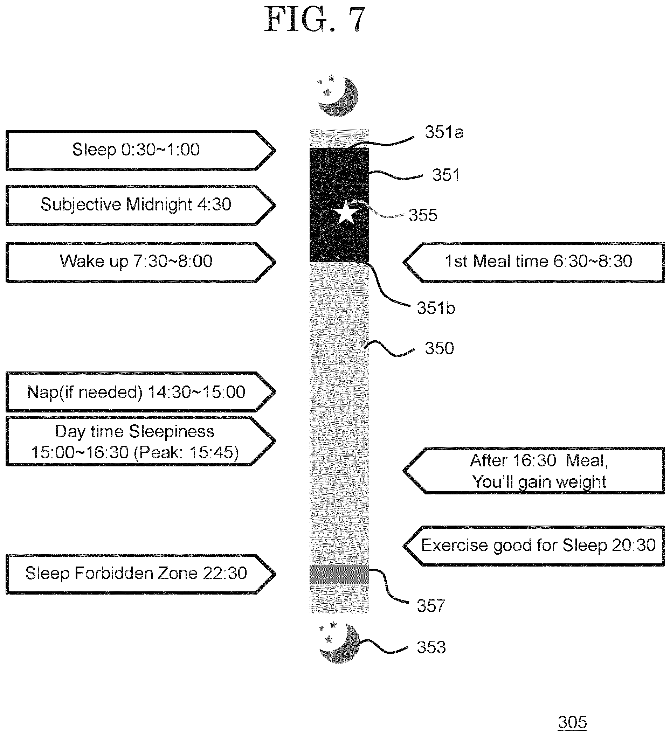

As described above, in the case where the bedtime or wake-up time is input as numbers, there is a possibility that input errors occur. In an embodiment, to prevent the above-described input errors, the system 100 preferably displays an interface to prevent input errors on the terminals such as the PC 13, the mobile phone 15, the tablet terminal 17, or the wearable device 19, and causes the user to input a bedtime or wake-up time. FIGS. 6 and 7 are schematic diagrams showing an interface for preventing an input error according to an embodiment of the present invention. In the system 100, an application program in conjunction with the information processing device 110 is installed in the terminals such as the PC 13, the mobile phone 15, the tablet terminal 17, or the wearable device 19. FIG. 6 (a) is a schematic diagram showing an interface 301 having a circular indicator 310 provided by the application program. The interface 301 includes a slider 311 for moving a selection button or an icon 311a set as the bedtime and a selection button or an icon 311b set as the wake-up time on the circular indicator 310. The interface 301 has an icon/illustration 313a, such as the sun, which means the day, and an icon/illustration 313b, such as the moon or star, which means the night adjacent to the indicator 310.

FIG. 6 (b) is a schematic diagram showing an interface 303 for moving a slider on a linear indicator. The interface 303 having a linear indicator has a slider 331 for moving a selection button or an icon 331a set as the bedtime and a selection button or an icon 331b set as the wake-up time on a linear indicator 330. The interface 303 has an icon/illustration 333a, such as the sun, which means the day, and an icon/illustration 333b, such as the moon or star, which means the night adjacent to the indicator 330.

FIG. 7 is a schematic diagram showing an interface 305 for moving a slider on a linear indicator. The interface 305 having a linear indicator has a slider 351 for moving a selection button or an icon 351a set as the bedtime and a selection button or an icon 351b set as the wake-up time on a linear indicator 350. The interface 305 has an icon/illustration 353, such as the moon, which means the night adjacent to the indicator 350. The interface 305 has an icon/illustration 355, such as a star, which indicates SPB-SM. The interface 305 may also present "time estimated to be difficult to fall asleep (Sleep Forbidden Zone)" as a zone 357.

In the case where the user directly inputs the time to the interface displayed on the terminal, the user can input the bedtime and the wake-up time to the terminal by moving the slider on the interface 301 having the circular indicator, the interface 303 having the linear indicator, or the interface 305 having the linear indicator.

In the case of using the method of moving the slider on the linear indicator for selecting, the slider may be arranged around 3 to 4 a.m. or around that time as a center, which is the time zone during which many people fall asleep in the whole population. Normally, by lightening the color of the display before 15:00, which is rarely bedtime, and after 15:00 of the next day, which is rarely wake-up time, it is possible to further reduce input errors by the user.

In an embodiment, when a sleep-wake time is input regardless of whether it is a circular indicator, a straight indicator, or an input in text format, input errors by the user are further reduced by setting the bedtime to about 0:00, which is the most frequent value, and the wake-up time to 7:00 am, which is the most frequent value, as initial values.

### [Method for calculating optimal wake-up time/bedtime based on individual circadian rhythm]

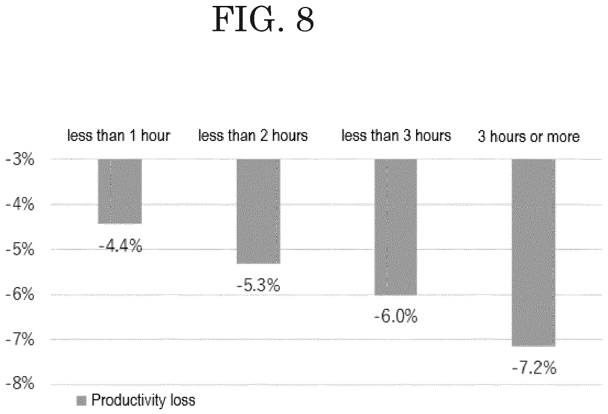

As a result of the research of the present inventors, it was clarified that the mental and physical health (Physical component summary and Mental component summary in the QOL scale) and the performance (labor productivity score and presenteeism score) decrease as the ratio of deviation of the actual wake-up time and bedtime increase from the "optimal wake-up time and bedtime" estimated from the individual circadian rhythm. FIG. 8 is a diagram showing that productivity decreases as the ratio of the deviation of the actual individual wake-up time from the "optimal wake-up time" calculated from the above-described SPB-SM increases. The time shown in FIG. 8 indicates the deviation rate in the wake-up time from SPB-SM + 3.5 hours, and the vertical axis indicates the ratio of productivity loss. The data shown in FIG. 8 is n=2905.

Human beings have "times easy to fall asleep" and "times difficult to fall asleep" in a day. The "times difficult to fall asleep" is called "Sleep Forbidden Zone", and it is known that it is difficult for a human being to fall asleep at a certain time zone before bedtime, even though it is at night (LAVIE, Peretz. Ultrashort sleep-waking schedule. III. 'Gates' and 'forbidden zones' for sleep. Electroencephalography and clinical neurophysiology, 1986, 63.5: 414-425. Literature: STROGATZ, STEVEN H.; KRONAUER, Richard E.; CZEISLER, Charles A. Circadian pacemaker interferes with sleep onset at specific times each day: role in insomnia. American Journal of Physiology-Regulatory, Integrative and Comparative Physiology, 1987, 253.1: R172-R178.). It is said that the secretion and the like of thyroid hormones are involved in Sleep Forbidden Zone.

However, for the time corresponding to Sleep Forbidden Zone, only an ambiguous conclusion of "one to three hours before a customary bedtime" was made, and the time varied in several studies, and it was not known at specifically what time. Furthermore, in the case of a person who has a social life, it is difficult to grasp the customary bedtime itself because it fluctuates and varies.

As a result of the investigation by the present inventor, it was revealed that Sleep Forbidden Zone was weakly associated with weekday customary bedtime, while using SPB-SM indicated that Sleep Forbidden Zone was present around 18 hours after (6 hours before) SPB-SM. The optimal wake-up time/bedtime calculation method based on the individual circadian rhythm according to an embodiment of the present invention can prevent a loss of time by an attempt to fall asleep at a time when it is supposed to be intrinsically difficult to sleep or prevent an induced insomnia (psychophysiological insomnia) caused by sleep difficulty, by using Sleep Forbidden Zone existing around 18 hours after (6 hours before) SPB-SM as a reference.

Since human beings control the center of the biological clock by light, it is possible to control SPB-SM by themselves by controlling light. At this time, blue light of 420 nm to 480 nm is particularly involved in the adjustment of the biological clock by light. For example, see the following references.

Newman, Lucy A., et al. "Melanopsin forms a functional short-wavelength photopigment." Biochemistry 42.44 (2003): 12734-12738.

Brainard, George C., et al. "Sensitivity of the human circadian system to short-wavelength (420-nm) light." Journal of biological rhythms 23.5 (2008): 379-386.

Berson DM, Dunn FA. et al. Phototransduction by retinal ganglion cells that set the circadian clock. Science. 2002;295(5557):1070-3.

Dacey DM, Liao HW. et al. Melanopsin-expressing ganglion cells in primate retina signal colour and irradiance and project to the LGN. Nature.2005;433(7027): 749-54.

Tu DC, Zhang D. et al. Physiologic diversity and development of intrinsically photosensitive retinal ganglion cells. Neuron. 2005;48(6):987-99.

Emanuel, Alan Joseph, and Michael Tri Hoang Do. "Melanopsin tristability for sustained and broadband phototransduction." Neuron 85.5 (2015): 1043-1055. http://www.cell.com/neuron/pdfExtended/S0896-6273(15)00100-2.

Pilorz, Violetta, et al. "Melanopsin Regulates Both Sleep-Promoting and Arousal-Promoting Responses to Light." PLoS Biol 14.6 (2016): e1002482. http://journals.plos.org/plosbiology/article?id=10.1371/journal.pbio.1002482.

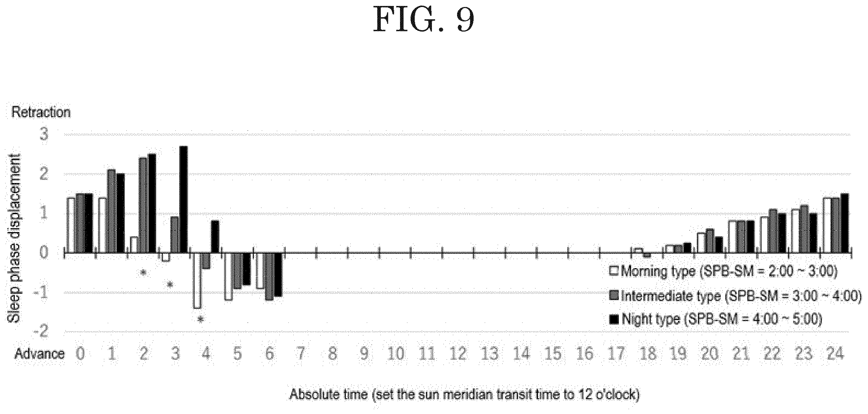

FIG. 9 is a diagram showing an effect of one hour of light irradiation on the sleep-wake schedule. In FIG. 9, the effect of light on SPB-SM of a person living in a condition without excessive light exposure at night and excessive light blocking in the morning was examined at every time when the person was exposed to light for one hour for two consecutive days. This investigation was conducted in September when sunrise was 6:00 and sunset was around 18:00, and the data of people who are exposed to excessive light at night and blocking light in the morning and day in daily life were excluded. Since the outside world is bright from 7:00 to 17:00, no data is collected for light irradiation. In FIG. 9, p < 0.05 (one-way ANOVA).

The biological clock advances when exposed to light from a time earlier than the actual SPB-SM time. The human biological clock delays when exposed to light from after sunset to SPB-SM. For this reason, it has been clarified in the present invention at what time the biological clock is able to operate, and it is possible to operate the biological clock by using the time and presenting the time using lighting or operating the lighting by the system.

Human beings recognize the "start of the morning" by starting light irradiation, recognize the "continuation of the daytime" by maintaining light irradiation, and recognize the "start of the night" by continuing darkness (approximately 50 Ix or less, or approximately 10 Ix or less in the case of blue light) to the extent that melatonin secretion occurs.

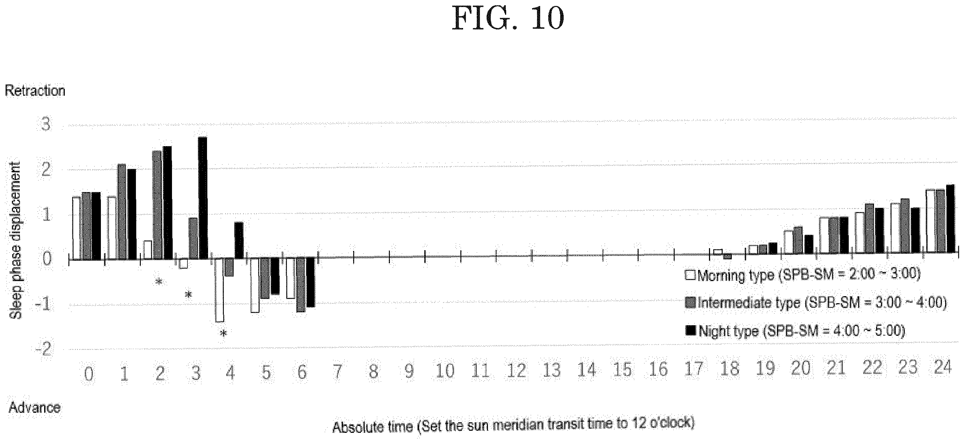

Here it is known that short-time light irradiation with reference to the peak value of a melatonin concentration has an effect on the adjustment of the biological clock (KHALSA, Sat Bir S., et al. A phase response curve to single bright light pulses in human subjects. The Journal of physiology, 2003, 549.3: 945-952.). On the other hand, in the present embodiment, as a result of performing the light exposure for each SPB-SM based on the absolute time, the result shown in FIG. 10 was obtained. FIG. 10 is a diagram showing an influence of one-hour light irradiation on the sleep-wake schedule based on the absolute time (mid-south time of the sun).

In other words, the biological clock advances by exposure to light earlier than the actual dawn, while the biological clock delays by exposure to light earlier than SPB-SM. The time zone when the morningness person (SPB-SM = 2:00 to 3:00) can advance the biological clock by exposure to light starts at around 3:00, while the time when the eveningness person (SPB-SM = 4:00 to 5:00) can advance the biological clock starts at around 5:00. As described above, whether the biological clock is easily advanced or retracted is different according to the type of the biological clock of the individual, and even if the individuals are exposed to light at the same time, the influence of the light on the biological clock differs between individuals. For this reason, it is also important to be able to select whether to advance or retract the biological clock in the case where the phase of the biological clock is to be displaced in accordance with the activity time, for example, in the case where the biological clock is to be engaged in shift work or moved to an area where a time difference occurs, and it is possible to promote early adaptation to the activity time by adjusting the "from when" or "how long time" when exposed to light according to the biological clock of the individual.

Although it does not have a direct effect on the central clock (suprachiasmatic nucleus) of the biological clock, there are diet, exercise, bathing, and the intake of articles of taste as factors that act on the peripheral clock and affect the degree of arousal and ease of falling asleep. In addition, ingestion of melatonin or melatonin receptor agonists is an example of a drug induced synchronization factor of the biological clock. In an embodiment, the system 100 can present an optimal time to implement factors affecting the degree of arousal and ease of falling asleep.

As a result of the examination by the inventor, it was clarified that, if a fasting time of 12 hours or more is set, the sleep onset probability in the time zone increases and sleepiness increases after the next day. It is thought that this is because the body recognizes the time zone of the body time as a dormant period.

Referring to FIG. 1, the system 100 further includes a recommended time-of-day calculation unit 112c in the control unit 111 to execute an optimal wake-up time/bedtime calculation method according to an embodiment of the present invention. The recommended time-of-day calculation unit 112c is composed of an application program or module or the like for calculating the optimal wake-up time/bedtime of the user from the answers to the questions displayed on the questionnaire 1 or the display device of the terminal, or from the data obtained from the measuring device. The recommended time-of-day calculation unit 112c may be, for example, an application program or module stored in the storage unit 113 and executed by the control unit 111.

The optimal wake-up time/bedtime based on the individual circadian rhythm is calculated based on the SPB-SM of the user, which is calculated by the SPB-SM calculation methods of the above-described embodiments and stored in the database 114, and a required sleeping time. Instead of SPB-SM, it may be calculated based on the intermediate time of the required sleeping time of the original physiological sleep time zone obtained by other methods and the required sleeping time. The required sleeping time of the user can be calculated using the following method.

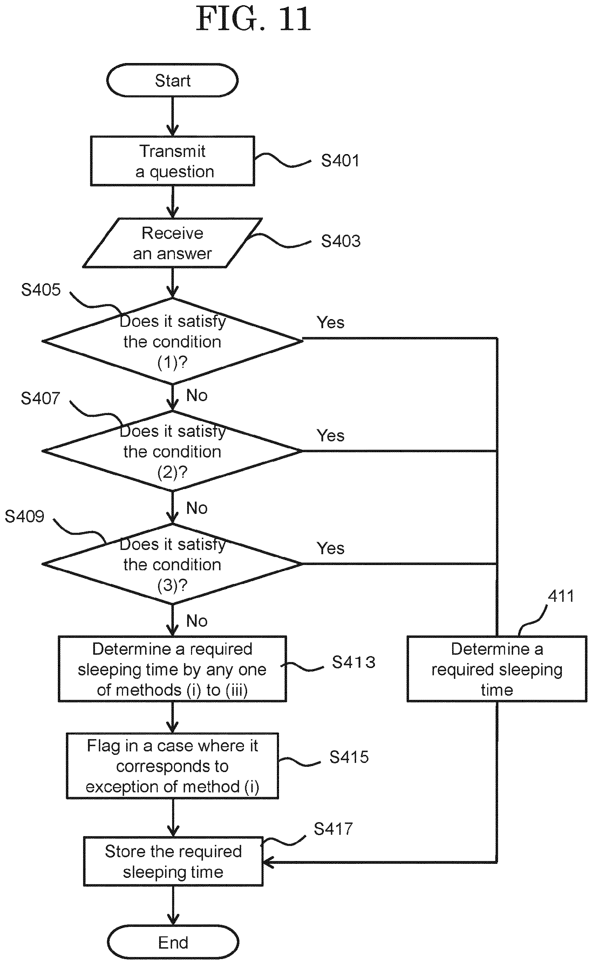

FIG. 11 is a flowchart illustrating calculation processing for the required sleeping time in the information processing device 110 according to an embodiment of the present invention. The control unit 111 reads the questions regarding the conditions (1) to (4) corresponding to the following required sleeping time calculation methods 1 to 4 stored in the storage unit 113, and transmits the questions to the terminals such as the PC 13, the mobile phone 15, the tablet terminal 17, and the wearable device 19 from the communication unit 115 via the network 50 (S401). In a case where the questionnaire 1 is used, questions about the conditions (1) to (4) may be printed in advance.

When the user inputs the answers to the question to the terminals such as the PC 13, the mobile phone 15, the tablet terminal 17, or the wearable device 19, the communication unit 115 receives the answer to the question displayed on the display device of the terminal or the data obtained from the measuring device from the terminal (S403). In the case where the user answers the questionnaire 1, the person who made the user answer the question (medical professional, etc.) may input the answer wrote into the questionnaire 1 to the terminal.

The recommended time-of-day calculation unit 112c sequentially determines whether the received data satisfies the conditions (1) to (4). That is, the recommended time-of-day calculation unit 112c determines whether the received data satisfies the condition (1) (S405), and in the case where the received data does not satisfy the condition (1), determines whether the received data satisfies the condition (2) (S407). In the case where the received data does not satisfy the condition (2), the recommended time-of-day calculation unit 112c determines whether the received data satisfies the condition (3) (S409). In the case where the received data does not satisfy the condition (3), the recommended time-of-day calculation unit 112c determines that the received data satisfies the condition (4).

The recommended time-of-day calculation unit 112c sequentially determines which of the conditions (1) to (4) the received data satisfies, and in the case where the received data satisfies any of the conditions (1) to (3), determines the required sleeping time based on the corresponding methods 1 to 3 for determining the required sleeping time (S411). The storage unit 113 stores the calculated SPB-SM in the database 114 (S417). Alternatively, the calculated SPB-SM may be temporarily stored in the main memory device (memory) to calculate the next recommended wake-up time and recommended bedtime.

### [Method 1 for determining required sleeping time]

The recommended time-of-day calculation unit 112c determines whether there is a sleep time after the elapse of two weeks in the case where natural sleep onset/natural wake-up without forcibly going to bed or awaking is continued for two weeks or more in the light-dark environment in a natural state by an isolation experiment of the user, hospitalization, or the like (condition (1)) (S405). In the case where it is determined that there is a sleep time after the elapse of two weeks, the recommended time-of-day calculation unit 112c sets the sleep time after the elapse of two weeks as the required sleeping time (S411).

### [Method 2 for determining required sleeping time]

In the case where there is no sleep time corresponding to the condition (1) in the received answer, the recommended time-of-day calculation unit 112c determines whether there is a sleep time after the elapse of two weeks in a period where there is no social schedule that affects bedtime or wake-up continuously for two weeks or more (condition (2)) (S407). In the case where it is determined that there is a sleep time after the elapse of two weeks, the recommended time-of-day calculation unit 112c sets the sleep time after the elapse of two weeks as the required sleeping time (S411).

### [Method 3 for determining required sleeping time]

In the case where there is no sleep time corresponding to the condition (2) in the received answer, the recommended time-of-day calculation unit 112c determines whether there is a sleep time after day 4 when sleeping and waking freely for four consecutive days or longer (condition (3)) (S409). In the case where it is determined that there is a sleep time after day 4, the recommended time-of-day calculation unit 112c sets the sleep time after day 4 as the required sleeping time (S411). This is because the phenomenon of "over-slept" as a solution to sleep debt lasts for 2 weeks but is particularly intense in the first 3 days and greatly alleviated after day 4 (Kitamura, Shingo, et al. "Estimating individual optimal sleep duration and potential sleep debt." Scientific reports 6 (2016): 35812.).

### [Method 4 for determining required sleeping time]

In the case where the data of condition (3) cannot be obtained in the received answer, the recommended time-of-day calculation unit 112c may use the time obtained by any one of the following methods (i) to (iii) as the required sleeping time (S413).

### Method (i)

The recommended time-of-day calculation unit 112c reads the questions stored in the storage unit 113, such as "How long do you need to sleep to act without feeling sleepiness in the daytime?", "How long do you need to sleep to act in good physical condition in the daytime?", and "How long do you sleep if no schedule of school, work, or the like in particular is continued?", and transmits the questions to the terminals such as the PC 13, the mobile phone 15, the tablet terminal 17, and the wearable device 19 from the communication unit 115 via the network 50. In a case where the questionnaire 1 is used, questions about the conditions (1) to (4) may be printed in advance.

When the user inputs the answer to the question in the terminals such as the PC 13, the mobile phone 15, the tablet terminal 17, and the wearable device 19, the communication unit 115 receives the answer to the question displayed on the display device of the terminal or the data obtained from the measuring device from the terminal. In the case where the user answers the questionnaire 1, the person who made the user answer the question (medical professional, etc.) may input the answer wrote into the questionnaire 1 to the terminal. The recommended time-of-day calculation unit 112c sets the maximum value of the above-mentioned time obtained from the received answer as the required sleeping time (S413). However, at this time, the following two exception procedures are performed.

### Exception procedure (i-1)

The recommended time-of-day calculation unit 112c sets 6 hours as the required sleeping time in the case where the user is a worker generation and a time less than 6 hours is input from the terminal (S413). This is because sleep times less than 6 hours are known to significantly increase mortality (AMAGAI, Yoko, et al. Sleep duration and mortality in Japan: The Jichi medical school cohort study. Journal of epidemiology, 2004, 14.4: 124-128.) and greatly increase the risk of psychological problems including suicide (GOODWIN, Renee D.; MARUSIC, Andrej. Association between short sleep and suicidal ideation and suicide attempt among adults in the general population. Sleep, 2008, 31.8: 1097-1101.).

### Exception procedure (i-2)

The recommended time-of-day calculation unit 112c sets 8 hours as the required sleeping time in the case where a time less than 8 hours is input in children and adolescent generations (adolescents) (S413). This is because sleep times of less than 8 hours are known to increase suicide rates (LIU, Xianchen. Sleep and adolescent suicidal behavior. Sleep, 2004, 27.7: 1351-1358.).

In this case, because the minimum sleep time required for healthy health is 8 hours, the recommended time-of-day calculation unit 112c set a flag to indicate that the time is set as the sleep time (S415).

However, in a few percent of the population, there are "short sleepers" who do not suffer from any sleepiness or disorder even if they sleep for less than 6 hours. The recommended time-of-day calculation unit 112c questions the user whether the sleep time was always short, including in childhood. In the case where the user inputs an answer indicating that the sleep time was always short for the lifetime including in childhood, the recommended time-of-day calculation unit 112c sets the input time as the required sleeping time (S413). In the case where the user inputs an answer indicating that the sleep time was not always short for the lifetime, because the minimum sleep time required for healthy health is 6 hours for a worker generation, and 8 hours for children and adolescent generations, the recommended time-of-day calculation unit 112c sets a flag to indicate that the time is set as the sleep time (S415).

### Method (ii)

In the case where the age data of the user is stored in the storage unit 113, the recommended time-of-day calculation unit 112c reads the age data of the user. In the case where the age data of the user does not exist, the recommended time-of-day calculation unit 112c transmits a request for inputting the age of the user to the terminal. When the user inputs the age to the terminals such as the PC 13, the mobile phone 15, the tablet terminal 17, or the wearable device 19, the communication unit 115 receives the user's age from the terminal. In the case where the user answers the questionnaire 1, the person who made the user answer the question (medical professional, etc.) may input the answer wrote into the questionnaire 1 to the terminal. The recommended time-of-day calculation unit 112c sets the average time of sleep in each age indicated by the previous study (ROFFWARG, Howard P.; MUZIO, Joseph N.; DEMENT, William C. Ontogenetic development of the human sleep-dream cycle. Science, 1966.) as the required sleeping time based on the received user's age (S413).

### Method (iii)

The recommended time-of-day calculation unit 112c sets 7 hours, which is the minimum required sleeping time of a worker, which is indicated by the academic institution (HIRSHKOWITZ, Max, et al. National Sleep Foundation's sleep time duration recommendations: methodology and results summary. Sleep Health, 2015, 1.1: 40-43.), as the required sleeping time (S413).

The required sleeping time of the user determined by any of the above processing methods may be stored in the storage unit 113 (S417).

### [Method of calculating recommended wake-up time and recommended bedtime based on individual required sleeping time]

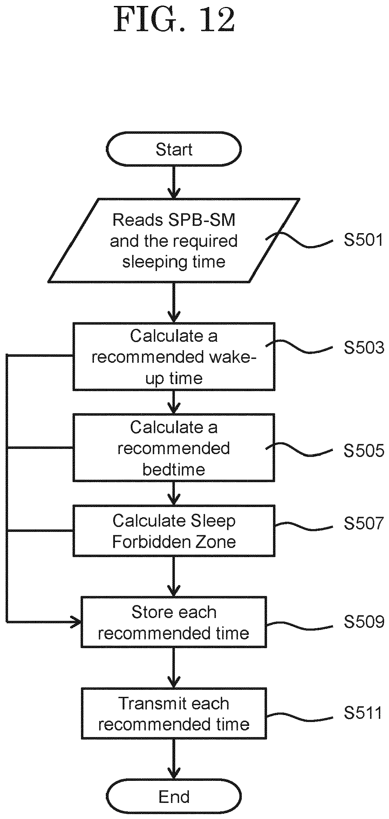

The wake-up time at which the individual's mental and physical health, QOL, and performance are maximized or accidents and incidents such as traffic accidents are minimized is in a time zone between a time obtained by adding half of the required sleeping time of the individual to SPB-SM and a time about 30 minutes before that time. FIG. 12 is a flowchart illustrating calculation processing for an optimal wake-up time/bedtime in the information processing device 110 according to an embodiment of the present invention. The recommended time-of-day calculation unit 112c reads SPB-SM and the required sleeping time of the user stored in the storage unit 113 (S501). The recommended time-of-day calculation unit 112c calculates the time or time zone obtained by adding half of the required sleeping time of the user to SPB-SM of the user as the "recommended wake-up time" (S503). For example, if the user's SPB-SM is 4:30 and the required sleeping time is 8 hours, the user's recommended wake-up time is around 8:00 to 8:30. The recommended time-of-day calculation unit 112c stores the recommended wake-up time in the storage unit 113 (S509). The recommended wake-up time may be transmitted from the communication unit 115 to the terminal via the network 50 (S511), and the terminal may display the received recommended wake-up time.

The recommended time-of-day calculation unit 112c subtracts the required sleeping time of the user from the recommended wake-up time to calculate an optimal bedtime (S505). For example, in the above example, since SPB-SM of the user is 4:30, the required sleeping time is 8 hours, and the recommended wake-up time is around 8:00 to 8:30, the recommended bedtime is around 0:00 to 0:30. The recommended time-of-day calculation unit 112c stores the recommended bedtime in the storage unit 113 (S509). The recommended bedtime may be transmitted from the communication unit 115 to the terminal via the network 50 (S511), and the terminal may display the received recommended bedtime.

As described above, the time of 18 hours after (6 hours before) SPB-SM is Sleep Forbidden Zone during which falling asleep is difficult, and it is not recommended to go to bed at this time. Therefore, the recommended time-of-day calculation unit 112c calculates Sleep Forbidden Zone as "time difficult to fall asleep" (S507). For example, in the above example, SPB-SM is 4:30 and Sleep Forbidden Zone is calculated to be around 22:30. The recommended time-of-day calculation unit 112c stores Sleep Forbidden Zone in the storage unit 113 (S509). Sleep Forbidden Zone may be transmitted from the communication unit 115 to the terminal via the network 50 (S511), and the terminal may display the received Sleep Forbidden Zone as a time difficult to fall asleep. Therefore, even if the user wants to go to bed early, the recommended bedtime is after this time.

### [Method of presenting sleep-onset-related behaviors based on individual circadian rhythm]

Even if the optimal wake-up time/bedtime based on the biological clock is known as described above, it may be difficult to stay at the wake-up time/bedtime in social life, and how the biological clock is adjusted is important. Therefore, it is also important to communicate the method for adjusting the biological clock based on the calculated recommended wake-up time and recommended bedtime, or the possibility of disease.

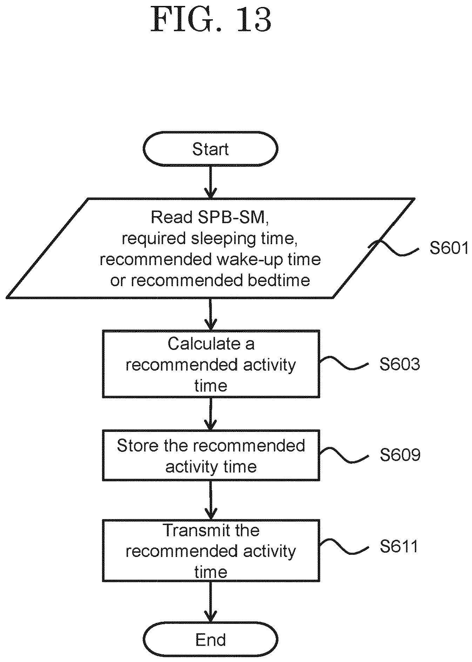

In an embodiment, the system 100 may present a lifestyle habit to the user to smoothen sleep. For example, the relationship between bathing and exercise that raises body temperature and sleep is known (Shiro Oda, Effects of temperature fluctuations before sleep on sleep (general), Lifelong learning studies and practice: The research bulletin of the Institute of Lifelong Studies, Hokkaido Asai Gakuen University,2003, 4: 223-231.), and it is known that bathing about 2 to 6 hours before sleep effectively lowers the deep body temperature at sleep and promotes falling asleep. FIG. 13 is a flowchart illustrating calculation processing for an optimal recommended activity time in the information processing device 110 according to an embodiment of the present invention. The recommended time-of-day calculation unit 112c reads the recommended bedtime of the user stored in the to adjust unit 113 (S601). The recommended time-of-day calculation unit 112c calculates 2 to 6 hours before the recommended bedtime as a recommended bathing time for promoting sleep (S603). The recommended time-of-day calculation unit 112c stores the recommended bathing time in the to adjust unit 113 (S609). The recommended bathing time is transmitted from the communication unit 115 to the terminal via the network 50 (S611), and the terminal may display the received recommended bathing time and recommend bathing to the user.

It is known that exercise at the end of high body temperature period in daytime hyperthermia (corresponding to 16 hours after SPB-SM) effectively lowers the deep body temperature at bedtime, promotes sleep onset, and increases deep sleep. The recommended time-of-day calculation unit 112c reads the user's SPB-SM stored in the to adjust unit 113 (S601). Based on the user's SPB-SM, the recommended time-of-day calculation unit 112c calculates around SPB-SM +16 hours as a recommended exercise time for improving sleeping (S603). The recommended time-of-day calculation unit 112c stores the recommended exercise time in the to adjust unit 113 (S609). The recommended exercise time is transmitted from the communication unit 115 to the terminal via the network 50 (S611), and the terminal may display the received recommended exercise time and recommend exercise to the user.

In the case where an application program which functions in the system 100 is installed in the terminals such as the PC 13, the mobile phone 15, or the tablet terminal 17, the terminals can sense the operation of the terminal by the user. In an embodiment, if the terminals sense that the terminal is operated by the user even though it is already the recommended bedtime, the system 100 may notify the user that the time is already the recommended bedtime and prompt the user to go to bed via the terminal.

Light exposure within 2 hours of bedtime significantly inhibits sleep onset. For this reason, an application program functioning in the system 100 may be installed in a terminal having a function of an illuminometer. The system 100 may recommend the user to reduce the ambient illuminance via the terminal if the terminal senses 50 Ix or more of light or 10 Ix or more of blue wavelength light after the time two hours before the recommended bedtime.

Since human beings control the center of the biological clock by light, it is possible to control SPB-SM by themselves by controlling light. Therefore, when the user wants to move the time presented by the system 100 back and forth, it may be displayed on the terminal that it is possible. For example, in the case where the user wishes to advance SPB-SM, the system 100 may recommend the user to actively be exposed to the morning light, avoid nighttime light, and the like by displaying these on the terminal. On the contrary, in the case where the user wants to delay SPB-SM, the system 100 may recommend the user to avoid the morning light, actively be exposed to the light at night, and the like by displaying these on the terminal.

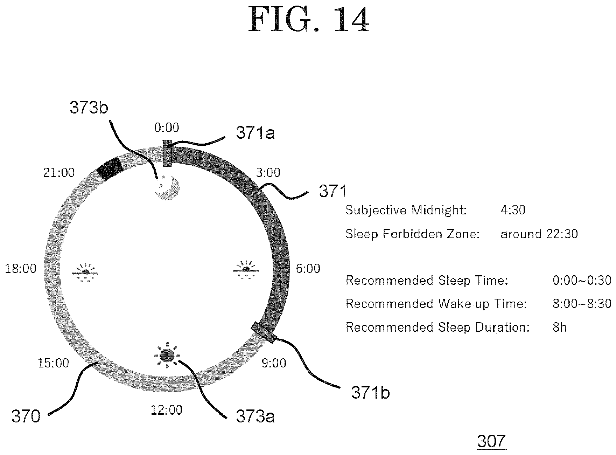

These displays may be performed, for example, by an interface 307 as shown in FIG. 14. The interface 307 has a mark 371a indicating the recommended bedtime, a mark 371b indicating the recommended wake-up time, and a bar 371 indicating the recommended sleeping time on a circular indicator 370. The interface 307 has an icon/illustration 373a, such as the sun, which means the day, and an icon/illustration 373b, such as the moon or star, which means the night adjacent to the indicator 370. The system 100 can provide various recommended times as visually easy-to-understand displays for the user via the interface 307 of such terminals.

It is preferable that the above-mentioned recommended times are continuously or periodically corrected based on the results of aggregation or regression analysis by the SPB-SM calculation unit 112b. The reference values to be subtracted or added for calculating the respective recommended times may also be updated by aggregation or regression analysis using data obtained from the user by the SPB-SM calculation unit 112b or the recommended time-of-day calculation unit 112c.

### [Lighting control system]

In an embodiment, a lighting control system may be configured by connecting the system 100 for presenting a daily life schedule based on the individual circadian rhythm with lighting equipment. For example, the system 100 for presenting a daily life schedule based on the individual circadian rhythm may reduce the illumination intensity or reduce the blue light component of the connected lighting equipment, or turn off the lighting equipment.

As described above, since human beings control the center of the biological clock by light, it is possible to control the phase of the biological clock and SPB-SM by themselves by controlling light. Therefore, when the user wants to move the time presented by the system 100 for presenting a daily life schedule based on the individual circadian rhythm advance or delay, the system 100 for presenting a daily life schedule based on the individual circadian rhythm may display that the presented time can be moved back and forth.

In the case where the user wants to advance the phase of the biological clock and SPB-SM, it is possible to display a message recommending "actively be exposed to the morning light and avoid nighttime light". In the case where the system 100 for presenting a daily life schedule based on the individual circadian rhythm is connected to lighting equipment, the system 100 for presenting a daily life schedule based on the individual circadian rhythm can advance SPB-SM by starting light irradiation containing a large amount of blue light components of 420 nm to 480 nm after SPB-SM.

In the case where the user wants to delay the phase of the biological clock and SPB-SM, a message recommending "avoiding the morning light and actively be exposed to light at night" can be displayed. In the case where the system 100 for presenting a daily life schedule based on the individual circadian rhythm is connected to lighting equipment, the system 100 for presenting a daily life schedule based on the individual circadian rhythm can delay SPB-SM by irradiating light containing a large amount of blue light components of 420 nm to 480 nm until SPB-SM.

### [Method of calculating sleepiness timing based on the individual circadian rhythm and method of presenting optimal nap time]

Human beings may experience reduced performance due to accumulation of sleep debt in the case where they are unable to secure adequate sleep time (VAN DONGEN, Hans, et al. The cumulative cost of additional wakefulness: dose-response effects on neurobehavioral functions and sleep physiology from chronic sleep restriction and total sleep deprivation. Sleep, 2003, 26.2: 117-126.). This decline in performance and sleepiness can be improved somewhat by a brief nap of about 20 to 30 minutes (Mitsuo Hayashi and Tadao Hori. A short nap as a countermeasure against afternoon sleepiness; Physiological psychology and Psychophysiology, 2007, 25.1: 45-59.).

On the other hand, there is currently no technology for knowing the time suitable for a nap. There is expert opinion that a brief nap after 12:00 is advisable because of increased sleepiness at daytime (12:00 to 15:00), and there is only a recommendation that nap within 8 hours before bedtime, i.e., on average, nap after 15:00, is undesirable because it inhibits adequate sleep inducer accumulation (Makoto Uchiyama, et al. Response and Treatment Guidelines for Sleep Disorders, 2002.).

As a result of the investigation by the present inventor, it was clarified that there were many people who were caused to feel sleepiness at around 12:00 to 16:00 in the whole population, but that there were differences depending on the individual circadian rhythm, and it was possible to predict the time at which the sleepiness appeared extremely acutely in a case where SPB-SM was used as a reference.

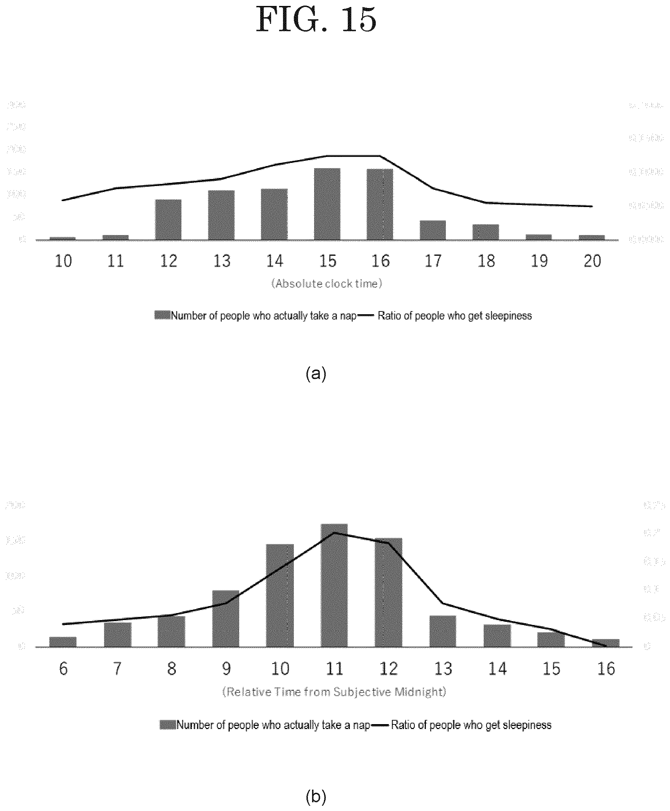

FIG. 15 is a graph showing a transition of the ratio of people who become sleepy in the daytime (the percentage of people who answer that they "may feel intense sleepiness during that time") and the number of people who actually take a nap in the daytime. FIG. 15 (a) is a diagram showing a transition between the ratio of people who become sleepy in the daytime and the number of people who actually take a nap in the daytime relative to the absolute time (actual time). FIG. 15 (b) is a diagram showing the transition of the ratio of people who become sleepy in the daytime and the number of people who actually take a nap in the daytime relative to the relative time from SPB-SM. The "ratio of people who become sleepy" in FIG. 15 (a) matches with the results of Multiple Sleep Latency Test (MSLT), which is a known objective measurement method for sleepiness (BIELIC, Toni; ZEC, Damir. Influence of Ship Technology and Work Organization on Fatigue. Pomorski zbornik, 2004, 42.1: 263-276.).

From the results in FIG. 15 (b) it is understood that sleepiness in the daytime/performance decline and nap appear according to the individual circadian rhythm rather than the absolute time of the outside world, that there is a time zone of intense sleepiness at SPB-SM +10.5 to 12 hours, peaking around SPB-SM +11.25 hours, and that sleepiness abruptly disappears after SPB-SM +13 hours. In other words, excessive sleepiness in the daytime and deterioration of performances can be suppressed by taking a brief nap from around SPB-SM +10 hours. On the other hand, even if a nap is attempted after SPB-SM +13 hours, in the first place, it is difficult to fall sleep at that time, and it is difficult to take a nap. The system for predicting and visualizing the timing at which the nap is recommended and the timing at which the sleepiness occurs is highly important in that productivity on the job can be improved and an accident or the like due to the sleepiness can be prevented.

The sleepiness timing based on the individual circadian rhythm and the optimal nap time are calculated by the above-described calculation method of SPB-SM of the user, and can be calculated by the following process based on SPB-SM of the individual stored in the main storage device or in the database 114, or the intermediate time of the original physiological sleep time zone obtained by another method (hereinafter also referred to as SPB-SM for convenience) and displayed on the display device of the terminal.

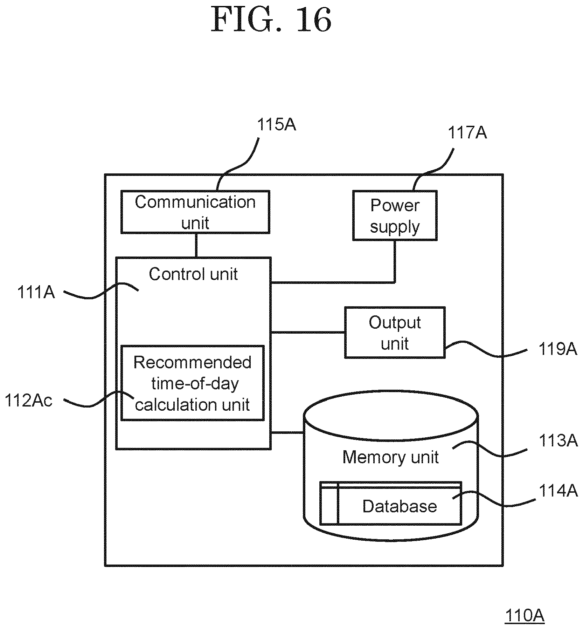

FIG. 16 is a block diagram illustrating an information processing device 110A according to an embodiment of the present invention. In this embodiment, the system 100 includes the information processing device 110A in place of the information processing device 110 to predict sleepiness timing based on an individual circadian rhythm and to present an optimal nap time. The information processing device 110A may include, for example, a control unit 111A, a storage unit 113A, a communication unit 115A, and a power supply 117A, and may further include an output unit 119A. The basic configuration of the control unit 111A is similar to that of the control unit 111, but differs from that of the control unit 111 in that it includes a recommended time-of-day calculation unit 112Ac which presents the optimal nap time based on the individual circadian rhythm and predicts sleepiness timing. The configurations of the storage unit 113A, the communication unit 115A, and the output unit 119A may be the same as those of the storage unit 113, the communication unit 115, and the output unit 119, respectively, and detailed descriptions thereof are omitted. In addition, the information processing device 110A may include various types of electronic devices included in a known information processing device.

The recommended time-of-day calculation unit 112Ac is composed of an application program, module, and the like for predicting sleepiness timing based on the individual circadian rhythm and presenting an optimal nap time. The recommended time-of-day calculation unit 112Ac may be, for example, an application program or module stored in the storage unit 113A and executed by the control unit 111A.

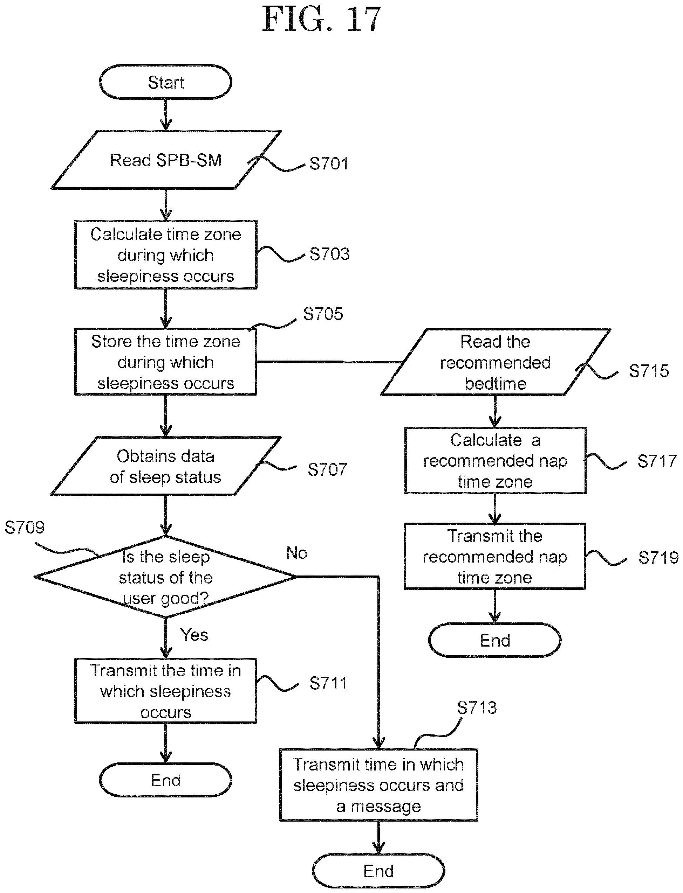

FIG. 17 is a flowchart illustrating calculation processing for an optimal nap time in the information processing device 110A according to an embodiment of the present invention. The recommended time-of-day calculation unit 112Ac reads the user's SPB-SM stored in a database 114A of the storage unit 113A (S701). The recommended time-of-day calculation unit 112Ac calculates a first sleepiness time zone during which sleepiness may occur by adding a first reference value having a predetermined time range to the user's SPB-SM, calculates a second sleepiness time zone during which strong sleepiness may occur by adding a second reference value having a narrower time range than the first reference value to SPB-SM, and calculates a third sleepiness time zone which is likely to be peak sleepiness by adding a third reference value having a narrower time range than the second reference value to SPB-SM. For example, the recommended time-of-day calculation unit 112Ac calculates SPB-SM +9 to 12 hours as the "time zone during which sleepiness occurs", particularly SPB-SM +10.5 to 12 hours as the "time zone during which strong sleepiness may occur", and SPB-SM +11.25 hours as "peak sleepiness" (S703). The recommended time-of-day calculation unit 112Ac stores the time zone during which sleepiness occurs in the storage unit 113A (S705).

The recommended time-of-day calculation unit 112Ac obtains data of the sleep status of the user (S707). The data of the sleep status of the user is obtained by receiving the answers to the questions displayed on the display device of the terminal or the data obtained from the measuring device from the terminal. The recommended time-of-day calculation unit 112Ac determines whether the sleep status of the user is good based on the obtained sleep status of the user (S709). In the case where the sleep status of the user is good, the recommended time-of-day calculation unit 112Ac may transmit the time in which sleepiness occurs from the communication unit 115A to the terminal via the network 50 (S711), and the terminal may display the received time zone during which sleepiness occurs. In the case where the user has had insufficient sleep, insomnia, or hypersomnia, the recommended time-of-day calculation unit 112Ac may transmit a message prompting the user to pay attention to sleepiness while transmitting a time in which sleepiness occurs from the communication unit 115A to the terminal via the network 50 (S713), and the terminal may display the received time in which sleepiness occurs and a message prompting the user to pay attention to sleepiness.

It is preferable that the above-mentioned "time zone during which sleepiness may occur", "time zone during which strong sleepiness may occur" and "peak sleepiness (time zone during which sleepiness is likely to be at a peak)" are continuously or periodically corrected based on the result of aggregation or regression-analysis by the recommended time-of-day calculation unit 112Ac. Therefore, the respective reference values to be subtracted or added for calculating the "time zone during which sleepiness may occur", "time zone during which strong sleepiness may occur" and "peak sleepiness" may also be updated by aggregation or regression-analysis using the data obtained from the user by the recommended time-of-day calculation unit 112Ac.

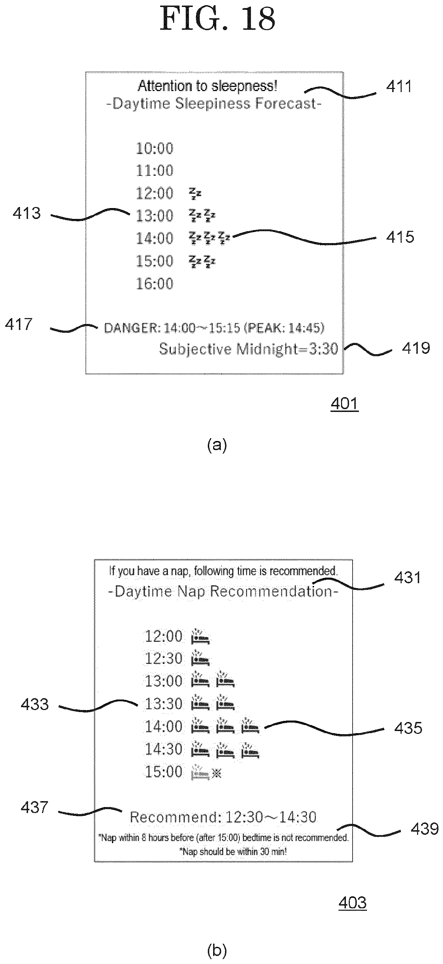

In an embodiment, these displays may be provided by an interface 401 of the terminal as shown in FIG. 18, for example. FIG. 18 (a) is a schematic diagram showing an example of the interface 401 which presents peak sleepiness to a user. The interface 401 may include, for example, a title 411, a time display 413, an icon 415, a display for calling attention 417, and an SPB-SM display 419. The title 411 may clarify that the interface 401 is to present peak sleepiness to the user. The time display 413 and the icon 415 visually display the "time zone during which sleepiness occurs", "time zone during which strong sleepiness may occur" and "peak sleepiness" calculated by the recommended time-of-day calculation unit 112Ac by the number of icons 415 arranged for the time display 413. The time display 413 and the icon 415 may be realized by other known display means such as a bar graph with respect to the time axis. The display for calling attention 417 may display, for example, a time zone in which sleepiness peaks and calls attention to the user. The SPB-SM display 419 displays SPB-SM of the user used to calculate peak sleepiness. The interface 401 may include other known displays to present peak sleepiness to the user.

In the case of working in the "time zone during which sleepiness occurs", the possibility of occurrence of an accident by the user increases. Therefore, in an embodiment, when the above time zone during which the sleepiness occurs is transmitted from the communication unit 115A to the terminal via the network 50, the time zone may be displayed on the terminal as a "time zone during which a miss/incident/accident is likely to occur". In the case where the user is a driver of a vehicle (train, bus, taxi, truck, etc.) or a worker in dangerous work, advice to call attention such as "do not engage in work in this time zone or pay careful attention in this time zone" may be presented to the terminal in the time zone during which sleepiness occurs. Further, in the time zone during which sleepiness peaks, the recommended time-of-day calculation unit 112Ac may transmit advice such as "take a break" to the terminal and cause the terminal to present the advice.

To suppress sleepiness in the "time zone during which sleepiness occurs", it is desirable to take a nap in the first half of the time. In an embodiment, the recommended time-of-day calculation unit 112Ac reads SPB-SM of the user described in the above embodiment from the storage unit 113A (S715). The recommended time-of-day calculation unit 112Ac calculates the time from the start time of the above-mentioned "time zone at which sleepiness occurs" to a predetermined time as a recommended nap time zone. For example, the recommended time-of-day calculation unit 112Ac calculates SPB-SM +9 to 11:00 as a recommended nap time zone in the case where sleepiness is likely to occur (S717). The recommended time-of-day calculation unit 112Ac may transmit the recommended nap time zone from the communication unit 115A to the terminal via the network 50 (S719), and the terminal may display the received recommended nap time zone.

At this time, since the nap within 8 hours before bedtime is not recommended, in an embodiment, the recommended time-of-day calculation unit 112Ac may calculate the recommended nap time -8 hours or later as the time zone during which the nap is not recommended (S717).

FIG. 18 (b) is a schematic diagram showing an example of the interface 403 which presents the recommended nap time zone to the user. The interface 403 may include, for example, a title 431, a time display 433, an icon 435, a recommended time zone display 437, and a non-recommended time zone display 439. The title 431 may clarify that the interface 403 is to present a recommended nap time zone to the user. The time display 433 and the icon 435 visually display the recommended nap time zone calculated by the recommended time-of-day calculation unit 112Ac by the number of icons 435 arranged for the time display 433. The time display 433 and the icon 435 may be realized by other known display means such as a bar graph with respect to the time axis. The recommended time zone display 437 may display, for example, a time zone during which a nap is recommended, and may recommend nap to the user. The non-recommended time zone display 439 presents the user with a time zone during which a nap is not recommended. It may be displayed at the same time as displaying that the nap is to be taken preferably within 30 minutes. The interface 403 may include other known displays to present the user with a time zone during which a nap is recommended.

As described above, sleepiness is present at SPB-SM +9 to 12 hours, sleepiness is intense after SPB-SM +10.5 hours, and sleepiness peaks toward SPB-SM +11 hours. In an embodiment, the interface 403 displays a star in the range of SPB-SM +9 to 12 hours that sleepiness occurs, and because it is desirable to have a nap at the time before the sleepiness peaks, that is, at SPB-SM +10 to 10.5 hours, which is before feeling intensely sleepy, a nap may be recommend to the user by increasing the number of stars.

It is preferable that each of the above-mentioned recommended times is continuously or periodically corrected based on the result of aggregation or regression-analysis by the SPB-SM calculation unit 112b. The reference values to be subtracted or added for calculating the respective recommended times may also be updated by aggregation or regression-analysis using the data obtained from the user by the SPB-SM calculation unit 112b or the recommended time-of-day calculation unit 112c.

### [Method of presenting optimal time zones for starting work, attending school, working, studying, and exercising based on individual circadian rhythm]

In a company, it is common for a single shift to start at 9:00 and finish at 17:00 or around these times. On the other hand, in the case of a variable working system or shift work, the working hours are set with considerable differences depending on the organization. However, these are not established with specific rationales from the viewpoints of productivity and health maintenance, but are customary.

Recent studies of Sleep Science and Chrono Biology have shown that each individual has a Circadian Rhythm and time zones in which they can work in good physical and mental condition. For example, early morning waking of a young person whose rhythm as a whole has become eveningness rhythm has an adverse effect on their mind and body. For example, in schools, changing the starting time to around 10 o'clock am has been found to improve mental and physical health, reduce absenteeism, and improve performance such as grades (KELLEY, Paul, et al. Is 8: 30 am Still Too Early to Start School? A 10: 00 am School Start Time Improves Health and Performance of Students Aged 13-16. Frontiers in human neuroscience, 2017, 11: 588.).

As a result of investigation by the present inventor, it has been clarified that there is the following relationship between presentism which express how much original productivity of an individual is lowered due to a mental and physical disorder and the circadian rhythm.

This presentism also includes physical performance, and in an embodiment of the present invention, performance in physical work with an exercise load, training, and sports can also be predicted.

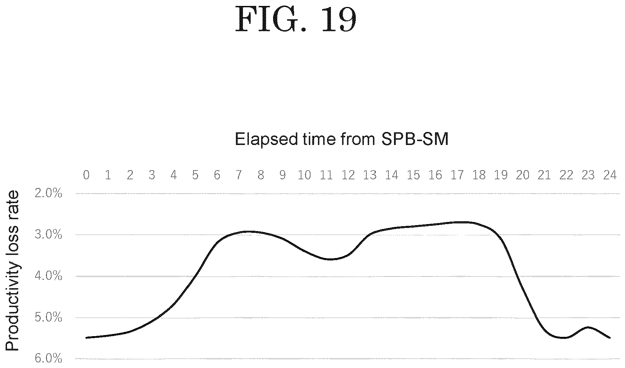

FIG. 19 is a graph showing a relationship between an elapsed time from SPB-SM and the productivity loss rate (productivity loss). As is apparent from FIG. 19, human productivity is the lowest at SPB-SM +0 hours. On the other hand, the high-productivity time zones are bimodally distributed, indicating that productivity increases in time zones of SPB-SM +6.5 to 8.5 hours and SPB-SM +13 to 18.5 hours, and that the highest productivity in a day is around SPB-SM +17 hours.

These findings can be used to present the optimal time to start work, attend school, and engage in athletic activities and sports. In addition, it is possible to present a time zone in which efficiency can be expected to increase when the individual performs work or study. In addition, in the case where a person is to work on a shift, it becomes clear which time zone is desirable for the individual to work. In the case where the length of time to be worked is fixed in advance, the integration of labor productivity according to the area under the curve (AUC) of the working hours can be calculated by calculating the AUC of FIG. 19, and efficient working hours can be derived.

The method of presenting an optimal activity time zone for attending school, starting work, and working based on the individual circadian rhythm can be calculated by the following processing method based on the SPB-SM of the individual calculated by the above-described calculation method of the SPB-SM of the user, and stored in the main memory device or the database 114, or the intermediate time of the original physiological sleeping time zone obtained by another method (hereinafter also referred to as SPB-SM for convenience) and displayed on the display device of the terminal.

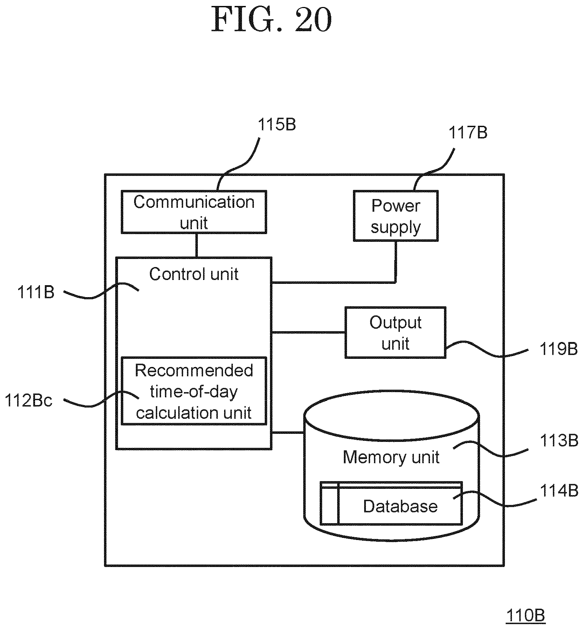

FIG. 20 is a block diagram illustrating an information processing device 110B according to an embodiment of the present invention. In this embodiment, the system 100 includes the information processing device 110B in place of the information processing device 110 to perform presentation of an optimal nap time based on the individual circadian rhythm and prediction of a sleepiness timing. The information processing device 110B includes, for example, a control unit 111B, a storage unit 113B, a communication unit 115B and a power supply 117B, and may further include an output unit 119B. The basic configuration of the control unit 111B is similar to that of the control unit 111, but differs from that of the control unit 111 in that it includes a recommended time-of-day calculation unit 112Bc for calculating an optimal activity time zones for starting work, attending school, working, studying, and exercising, and the like. The configurations of the storage unit 113B, the communication unit 115B, and the output unit 119B may be the same as those of the storage unit 113, the communication unit 115, and the output unit 119, respectively, and detailed descriptions thereof are omitted. In addition, the information processing device 110B may include various types of electronic devices included in a known information processing device.

The recommended time-of-day calculation unit 112Bc is composed of an application program or module, and the like for calculating an optimal activity time zone for starting work, attending school, working, studying, exercising, and the like based on the circadian rhythm of each individual. The recommended time-of-day calculation unit 112Bc may be, for example, an application program or module stored in the storage unit 113B and executed by the control unit 111B.

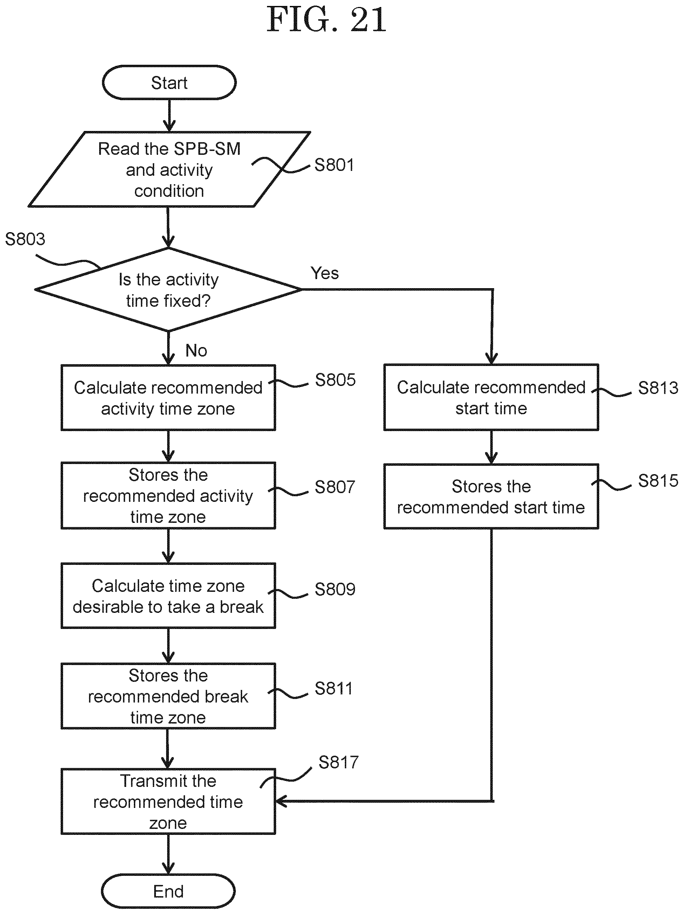

FIG. 21 is a flowchart illustrating calculation processing for an optimal activity time zone in the information processing device 110B according to the embodiment of the present invention. The recommended time-of-day calculation unit 112Bc reads SPB-SM of the user and activity conditions of the user for calculating an optimal activity time zone stored in a database 114B of the storage unit 113B (S801). The recommended time-of-day calculation unit 112Bc determines whether the activity time zone of the user is fixed to an activity time accompanied by a variable working system or shift work, and the activity time is a condition differing from a general condition (S803). In the case where the user is active in a general activity time zone, the recommended time-of-day calculation unit 112Bc calculates a first activity time zone during which physical and mental performance improves in the first half of the day by adding a fourth reference value having a predetermined time range to SPB-SM, and calculates a second activity time zone during which physical and mental performance improves in the second half of the day by adding a fifth reference value which is set at a late time in a predetermined time range from the fourth reference value to SPB-SM. For example, the recommended time-of-day calculation unit 112Bc calculates a time zone of SPB-SM +6.5 to 10 hours and a time zone of SPB-SM +12 to 18 hours as recommended activity time zones which are desirable for time zones for starting work, attending school, working, and studying (S805). The recommended time-of-day calculation unit 112Bc stores the recommended activity time zones in the storage unit 113B (S807).

The recommended time-of-day calculation unit 112Bc calculates a time zone between the first activity time zone and the second activity time zone as a recommended break time zone. For example, the recommended time-of-day calculation unit 112Bc calculates SPB-SM +10 to 12 hours as the "desirable time zone to take a break" (the recommended break time zone) (S809). The recommended time-of-day calculation unit 112Bc stores the recommended break time zone in the storage unit 113B (S811). The recommended time-of-day calculation unit 112Bc may transmit the recommended activity time zone and/or the recommended break time zone from the communication unit 115B to the terminal via the network 50 (S817), and the terminal may display the received recommended activity time zone and/or the recommended break time zone. In the case where work is set to a time including the subjective midnight due to shift work or the like, around SPB-SM may be calculated as the "desirable time zone to take a break" (the recommended break time zone).

In an embodiment, in a case where the length of working and schooling hours is fixed, the optimal start/end times can be presented by calculating AUC (Area Under the Curve). The recommended time-of-day calculation unit 112Bc calculates a recommended start time at which physical and mental performance improves by adding a sixth reference value having a predetermined time range to SPB-SM. For example, when the working hours of the user can be freely set and binding hours of 8-hours of is scheduled as in the variable working system, the productivity loss is minimized in the case of starting from SPB-SM +6 to 13 hours, and therefore the recommended time-of-day calculation unit 112Bc calculates SPB-SM +6 to 13 hours as the recommended start time (S813). On the other hand, in shift work, in the case of selecting actual working hours from the fixed time zone, it is possible to recommend which time zone is more appropriate for working by calculating the AUC. The recommended time-of-day calculation unit 112Bc stores the recommended start time in the storage unit 113B (S815). The recommended time-of-day calculation unit 112Bc may transmit the recommended start time from the communication unit 115B to the terminal via the network 50 (S817), and the terminal may display the received recommended start time.

In an embodiment, the terminal may provide the user with an estimated performance height for each time zone represented by a numerical value or an icon.

In an embodiment, the system may not only return the results to the individual, but may also output to an organization a list of which users (students or employees) are considered desirable to start work, attend school, work, study, and exercise at a certain time.

### [Method for calculating time of use of sedative-hypnotic psychotropic drug based on individual circadian rhythm]

Sedative-hypnotic psychotropic drugs, including hypnotics, are used to promote sleep onset. However, its effects are not reliable, and it is known that there are side effects called paradoxical reactions, such as intoxication-like reactions, disinhibition and abnormal behaviors, and memory deficits.

It is important to maximize the efficacy of drugs, prevent side effects, and prevent the wastage of medical resources and the abuse of drugs due to taking them at a time when it is ineffective.

As a result of the investigation by the present inventor, it has been clarified that there is a time zone during which the efficiency rate is increased by its administration, time zone where it is difficult to fall asleep due to poor effects even if the drug is taken, and time zone where the side effects are likely to occur, by the administration of the sedative-hypnotic psychotropic drug.

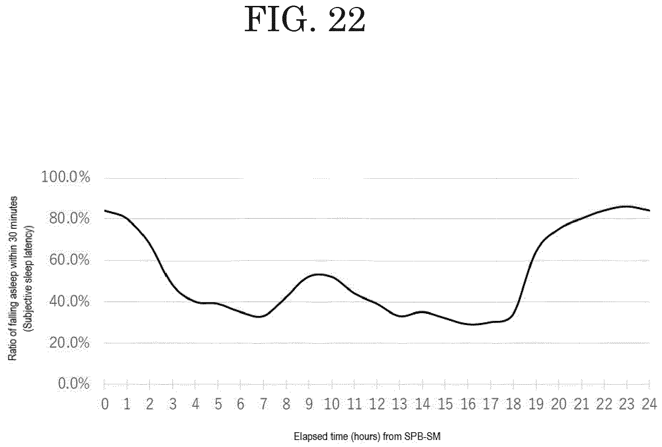

As a result of the investigation by the present inventor, it was found that there is a following clear difference depending on the time starting from SPB-SM calculated by the above-mentioned SPB-SM calculation method in the efficiency rate (subjective degree of improvement and percentage of people for who it was possible to fall asleep within 30 minutes after taking the drug) in a case where a person having insomnia symptoms uses a benzodiazepine receptor agonist, as shown in FIG. 22.

That is, from the time when approximately 4 hours have elapsed (in most cases, it corresponds to the morning) to the time when approximately 18 hours have elapsed (in most cases, it corresponds to the night) from SPB-SM of the user, the efficiency rate of the sedative-hypnotic psychotropic drug (the percentage of those who could fall asleep within 30 minutes after taking a sedative-hypnotic psychotropic drug) is less than 50% (there is a time zone that temporarily exceeds 50% after 10 to 11 hours from SPB-SM).

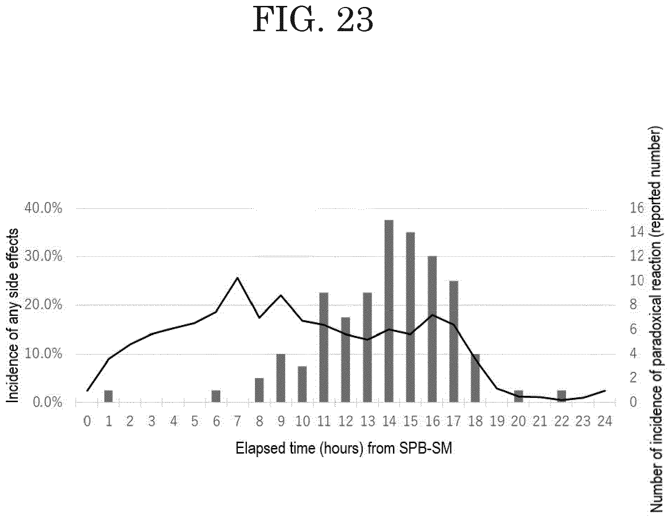

The incidence of any side effects (dizziness, lightheadedness, malaise, hallucinations, paresthesia, sleepiness carry-over to the next day, nausea, etc.) that occurred when a person having insomnia symptoms uses a benzodiazepine receptor agonist, and the number of cases of serious side effects that resulted from the use of a benzodiazepine receptor agonist, such as intoxication-like reactions, disinhibition, abnormal behavior, memory deficiency, agitation, easy anger, and personality transformation, which are called paradoxical reactions, are shown in FIG. 23.

Side effects occur at a frequency of 10% or more from the time about 2 hours after SPB-SM to the time about 17 hours after SPB-SM. In the first half, there were many sleepiness carry-over symptoms, and in the second half, there were many autonomic symptoms such as dizziness and nausea, and paradoxical reactions.

It was clear that the effects of sedative-hypnotic psychotropic drugs other than a benzodiazepine receptor agonist had a pattern similar to those described above. That is, in the case where the timing of the hypnotic effect corresponds to the "Forbidden Zone" (in the above embodiment, it was shown to correspond to around SPB-SM +18 hours), the hypnotic-sedative psychotropic drug could not exert its effect, and conversely, it was considered that side effects were likely to occur.

This revealed that there are times when the administration of sedative-hypnotic psychotropic drugs is recommended and not recommended, for each individual. Based on these findings, the system 100 according to an embodiment of the present invention displays whether and to what extent it is recommended that the user take the hypnotic drug at any given time.

The administration time of the sedative-hypnotic psychotropic drug based on the individual circadian rhythm can be calculated by the following process based on SPB-SM of the individual, which is calculated by the above-described calculation method of SPB-SM of the user and stored in the main memory device or the database 114, or the intermediate time of the original physiological sleeping time zone obtained by another method (hereinafter also referred to as SPB-SM for convenience) and displayed on the display device of the terminal.

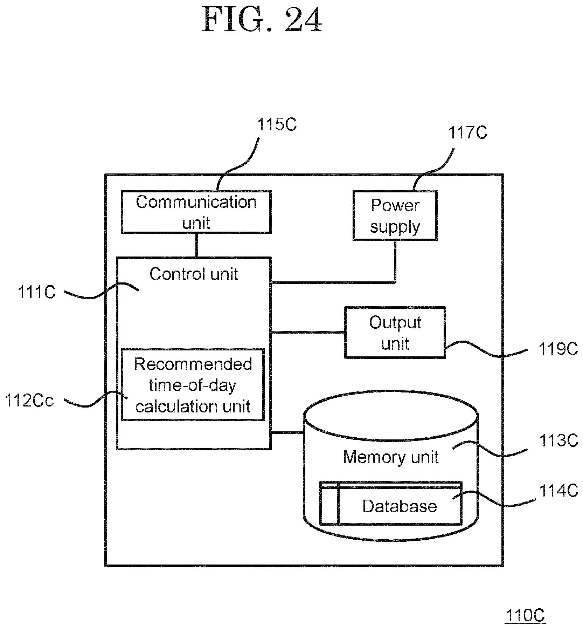

FIG. 24 is a block diagram illustrating an information processing device 110C according to an embodiment of the present invention. In the present embodiment, the system 100 includes the information processing device 110C in place of the information processing device 110 to calculate the administration time of sedative-hypnotic psychotropic drugs based on the individual circadian rhythm. The information processing device 110C includes, for example, a control unit 111C, a storage unit 113C, a communication unit 115C, and a power supply 117C, and may further include an output unit 119C. The basic configuration of the control unit 111C is similar to that of the control unit 111, but differs from that of the control unit 111 in that it includes a recommended time-of-day calculation unit 112Cc for calculating the administration time of sedative-hypnotic psychotropic drugs based on the individual circadian rhythm. The configurations of the storage unit 113C, the communication unit 115C, and the output unit 119C may be the same as those of the storage unit 113, the communication unit 115, and the output unit 119, respectively, and detailed descriptions thereof are omitted. In addition, the information processing device 110C may include various types of electronic devices included in a known information processing device.

The recommended time-of-day calculation unit 112Cc is composed of an application program, module, and the like for calculating the administration time of sedative-hypnotic psychotropic drugs based on the individual circadian rhythm. The recommended time-of-day calculation unit 112Cc may be, for example, an application program or module stored in the storage unit 113C and executed by the control unit 111C.

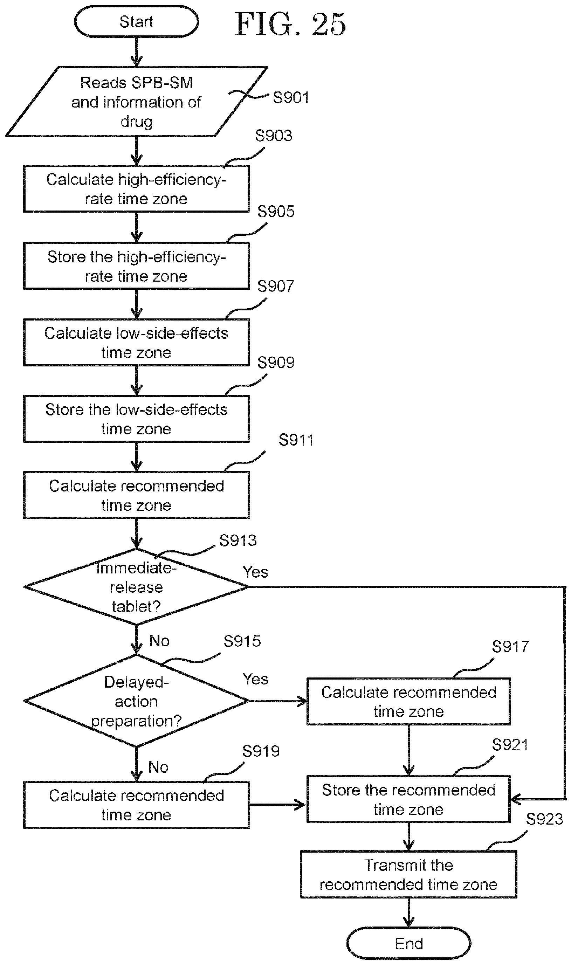

FIG. 25 is a flowchart illustrating calculation processing for the administration time of the sedative-hypnotic psychotropic drug in the information processing device 110C according to an embodiment of the present invention. The recommended time-of-day calculation unit 112Cc reads SPB-SM of the user and information of the drug to be taken, which are stored in a database 114C of the storage unit 113C (S901). In a case where the information of the drug is not stored in the database 114C, the information processing device 110C may transmit an inquiry about the drug to be taken to the terminal, and may receive an answer of the user from the terminal.

The recommended time-of-day calculation unit 112Cc calculates the time zone from SPB-SM +19 hours to SPB-SM +27 hours (+3 hours) as the time zone during which the high efficiency rate is expected (high-efficiency-rate time zone (seventh reference value)), based on the case where the user is taking an immediate-release tablet of a drug acting on a GABA receptor, represented by benzodiazepine receptor agonists, among sedative-hypnotic psychotropic drugs (S903). The recommended time-of-day calculation unit 112Bc stores the high-efficiency-rate time zone in the storage unit 113B (S905). The recommended time-of-day calculation unit 112Cc calculates the time zone from SPB-SM +18 hours to SPB-SM +25 hours (+1 hour) as a time zone of taking medicine with few side effects (low-side-effects time zone (eighth reference value)) (S907). The recommended time-of-day calculation unit 112Bc stores the low-side-effects time zone in the storage unit 113B (S909). In the case where the action time of the drug to be taken is long, the end time of the low-side-effects time zone may be appropriately shortened in accordance with the action time of the drug to be taken.

The recommended time-of-day calculation unit 112Cc integrates the high-efficacy-rate time zone and the low-side-effects time zone, and calculates the time zone from SPB-SM +19 hours to SPB-SM +25 hours (+1 hour) (however, the latter time is advanced to prevent sleepiness carry-over to the next day in the case where the effect time is long due to a long half-life) as a time zone (first recommended time zone) during which the effect of the drug to be taken is high and the side effect is low (S911). The recommended time-of-day calculation unit 112Cc determines whether the drug taken by the user is an immediate-release tablet of a drug acting on a GABA receptor (S913). In the case where the drug taken by the user is an immediate-release tablet of a drug acting on a GABA receptor, the recommended time-of-day calculation unit 112Bc stores the recommended time zone in the storage unit 113B (S921).

in the case where the drug being taken by the user is not an immediate-release tablet of the drug acting on a GABA receptor, the recommended time-of-day calculation unit 112Cc determines whether the drug being taken by the user is a delayed-action preparation of the drug acting on a GABA receptor, represented by the benzodiazepine receptor agonist, or a time-release preparation, or a drug that does not reach Cₘₐₓ immediately after taking the same among the sedative-hypnotic psychotropic drugs (S915). In the case where the drug being taken by the user is a delayed-action preparation of the drug acting on a GABA receptor, or a time-release drug, or a similar drug that does not reach Cₘₐₓ immediately after taking the same, the recommended time-of-day calculation unit 112Cc subtracts a duration until the blood concentration or brain concentration of the drug being taken reaches an effective range or time until sleepiness actually appears after taking the drug from the recommended time zone, and calculates it as the time zone (second recommended time zone) during which the effect of the drug being taken is high and the side effect is low (S917). The recommended time-of-day calculation unit 112Cc stores the recommended time zone in the storage unit 113B (S921).

In the case where the drug being taken by the user is not a delayed-action preparation, that is, in the case where the drug being taken by the user is a drug that exerts a sedative-hypnotic effect by acting on receptors other than a GABA receptor, such as an orexin receptor antagonist, a serotonin receptor 2 or 3 (5-HT₂ receptor or 5-HT₃ receptor) antagonist, or a histamine receptor antagonist, the recommended time-of-day calculation unit 112Cc subtracts a duration until the blood or brain concentration of the drug being taken reaches an effective range or time until sleepiness actually appears after taking the drug from the recommended time zone, and calculates it as the time zone (third recommended time zone) during which the effect of the drug being taken is high and the side effect is low (S919). For example, in the case where the user takes an orexin receptor antagonist, it is preferable that the recommended time zone starts from SPB-SM +18 hours. The recommended time-of-day calculation unit 112Cc stores the recommended time zone in the storage unit 113B (S921).

The recommended time-of-day calculation unit 112Cc may transmit the recommended time zone from the communication unit 115C to the terminal via the network 50 (S923), and the terminal may display the received recommended time zone.

### [Method of calculating alcohol intake time based on individual circadian rhythm]

Alcohol also has similar pharmacological effects as the above-mentioned benzodiazepine receptor agonists because GABA_{A} receptors are the main site of action that provides acute psychological effects. That is, the time when alcohol use is likely to cause psychological or intoxication reactions is equivalent to the results of studies on benzodiazepine receptor agonists. Sleepiness due to alcohol intake is likely to occur between SPB-SM +19 hours to +27 hours (+3 hours). Intoxication reaction due to alcohol intake is more likely to occur between SPB-SM +6 to +9 hours and SPB-SM +11 to +18 hours, particularly between SPB-SM +14 to +16 hours.

In a case where alcohol is used, the recommended time-of-day calculation unit 112Cc may determine that the time zone from SPB-SM +19 hours to SPB-SM +27 hours (+3 hours) is a time zone during which there is a high possibility of sleepiness due to alcohol intake, and may display the time zone on the interface such as the display. The recommended time-of-day calculation unit 112Cc may determine that the time zone from SPB-SM +11 to 18 hours is a time zone during which a user is likely to be intoxicated by alcohol intake. In particular, it may indicate that the possibility of sleepiness due to alcohol intake is remarkable between SPB-SM +14 to +16 hours.

### [Method for presenting action before travel and while moving at high speed for preventing jet lag based on the individual circadian rhythm]

When moving to a country with different time zones in a short time, a so-called jet lag occurs. Jet lag is caused by external desynchronization between the biological clock and the social time of the outside world (External Dys-synchronization: academic terminology of chronobiology/sleep science) which is caused by trying to act on the social schedules of the destination despite the biological clock remaining at the place of departure. Jet lag causes various mental and physical disorders and performance deterioration due to external desynchronization of biological clocks of various organs, in addition to disturbances of sleep and wake schedules that cause sleep failure at night of the local time, waking failure at morning of the local time, and intense sleepiness during daytime of the local time.

As a result of the examination by the inventor, it is considered that there are mainly two methods for preventing sleep problems and poor physical condition due to jet lag and maintaining the performance depending on the travel pattern.
1. Short-term (approximately within 5 days) travel
2. Long-term (approximately 5 days or more) travel

### 1. Short-term travel

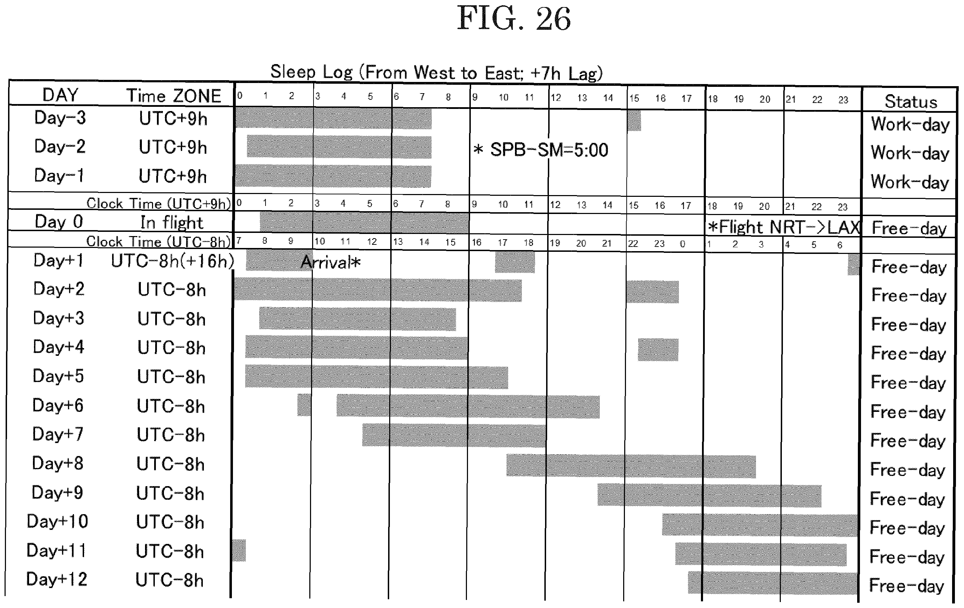

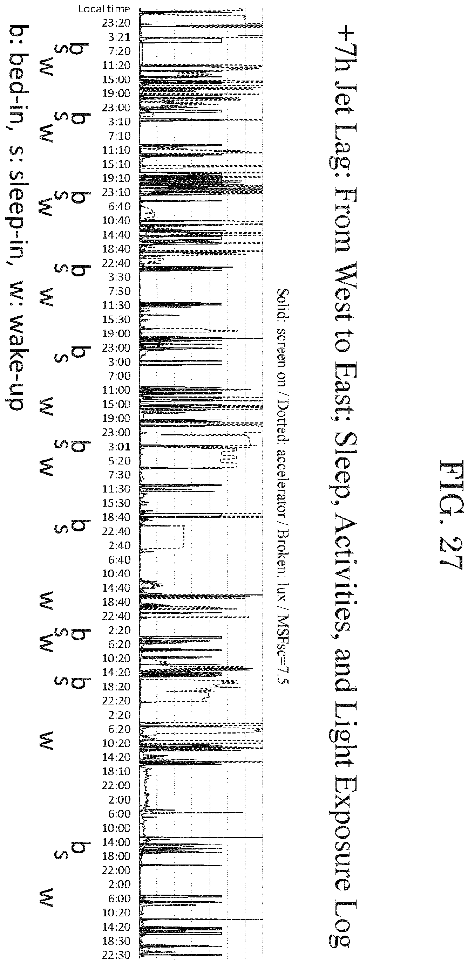

As a result of experiments by the inventor, it was revealed that the rhythm of the biological clock at the time of departure was maintained for about 5 days if light exposure in accordance with sunrise and sunset at the time of departure was taken into consideration. FIG. 26 shows a sleep diary performed by the present inventor when the light environment is kept as similar as possible to the place of departure. FIG. 27 shows measurement results of sleep status, activity meter, and light exposure monitor.

The sleep-wake time zone, sleepiness time zone, and time zone expected to exhibit high performance, which are defined in accordance with the biological clock of the individual shown in the above-described embodiment, are maintained for 4 to 5 days after travel if the light environment at the place of departure is maintained in the present embodiment. Since sudden fluctuations in the biological clock cause malfunctions and poor performance due to External/Internal Dys-synchronization, it is desirable to maintain the biological clock at the place of departure if short-term travel is scheduled to take place for approximately 5 days or less. Otherwise, they will return to the place of departure at the timing when they are about to be synchronized with the local time, which will have a serious adverse effect on their mental and physical health and performance.

In an embodiment, the system 100 can present the sleep-wake time zone, sleepiness time, and time zone expected to exhibit high performance defined by the biological clock of the individual and present a recommendation for the action while moving at high speed, such as on a passenger plane, and a recommendation for the action after arriving at the destination, so that the sleep-wake time zone, sleepiness time, and time zone expected to exhibit high performance may be utilized at the destination.

### 2. For long-term (approximately 5 days or more) travel

After 5 days of stay, significant synchronization with the local time occurs. In the example of FIG. 26, the sleep phase is synchronized with the local time in a form of delay, but the synchronization is not always synchronized by delaying. In the case of moving to a region with large time zone differences, the sleep phase is often synchronized by advancing in the case of a strong morningness person with SPB-SM < 1:30. In addition, the sleep of some people does not delay or advance, but temporarily divides and subdivides, destabilizes it, and then synchronizes with the local time. In any case, in the case of traveling for a long period of time, it is possible to alleviate the problem of jet lag by immediately synchronizing the biological clock with the local time.

At this time, the following three methods can be used as methods for adjusting the biological clock.
a) Adapt to local time by delaying the biological clock
b) Adapt to local time by advancing the biological clock
c) Adapt to local time by temporarily completely desynchronizing and destabilizing and then resetting

Which occurs in the natural state is defined by the responsiveness of each individual to light and the cycle length (tau=τ) of the chronotype and biological clock. However, it is possible to create delays or advances by irradiating light at a time matched to the biological clock of the individual.

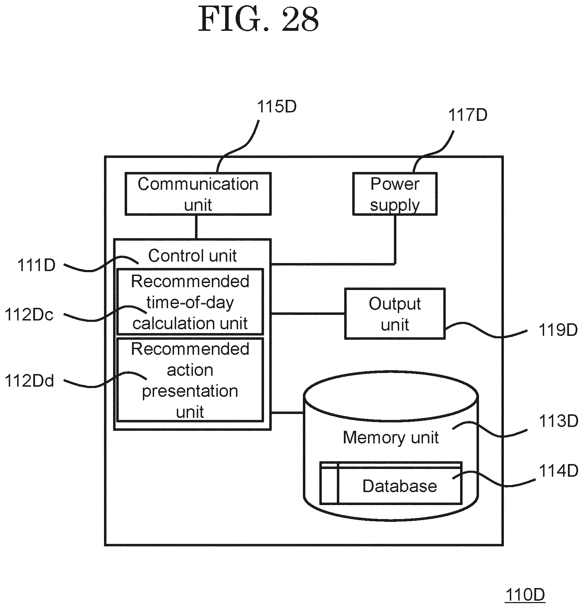

FIG. 28 is a block diagram illustrating an information processing device 110D according to an embodiment of the present invention. In the present embodiment, the system 100 includes the information processing device 110D in place of the information processing device 110 to present the action before travel and while moving at high speed for preventing jet lag based on the individual circadian rhythm. The information processing device 110D includes, for example, a control unit 111D, a storage unit 113D, a communication unit 115D and a power supply 117D, and may further include an output unit 119D. The basic configuration of the control unit 111D is similar to that of the control unit 111, but differs from that of the control unit 111 in that it includes a recommended time-of-day calculation unit 112Dc for calculating the optimal wake-up time/bedtime based on the individual circadian rhythm, presenting the optimal nap time, and predicting the sleepiness timing, and a recommended action presentation unit 112Dd for presenting the recommended action before travel and while moving at high speed for preventing jet lag based on the individual circadian rhythm. The configurations of the storage unit 113D, the communication unit 115D, and the output unit 119D may be the same as those of the storage unit 113, the communication unit 115, and the output unit 119, respectively, and detailed descriptions thereof are omitted. In addition, the information processing device 110D may include various types of electronic devices included in a known information processing device.

The recommended time-of-day calculation unit 112Dc includes an application program or module for calculating an optimal wake-up time/bedtime of a user from the answer to the question displayed on the questionnaire 1 or the display device of the terminal, or data obtained from the measuring device, and an application program or module for predicting sleepiness timing based on the individual circadian rhythm and presenting an optimal nap time. The recommended time-of-day calculation unit 112Dc may be, for example, an application program or module stored in the storage unit 113D and executed by the control unit 111D. The recommended time-of-day calculation unit 112Dc can calculate the optimal wake-up time/bedtime by the same processing method as the method of calculating the optimal wake-up time/bedtime described in the above embodiment. The recommended time-of-day calculation unit 112Dc can predict the sleepiness timing and present the optimal nap time by the same processing method as the method of predicting the sleepiness timing and the method of presenting the optimal nap time based on the individual circadian rhythm described in the above embodiment. Therefore, the detailed explanation of the recommended time-of-day calculation unit 112Dc is omitted. In an embodiment, in the case where the system 100 includes the information processing device 110 and the information processing device 110A described above, the information processing device 110D may have a configuration in which the recommended time-of-day calculation unit 112Dc is omitted.

The recommended action presentation unit 112Dd is composed of an application program or module or the like that presents the recommended action before travel and while moving at high speed for preventing jet lag based on the individual circadian rhythm. The recommended action presentation unit 112Dd may be, for example, an application program or module stored in the storage unit 113D and executed by the control unit 111D.

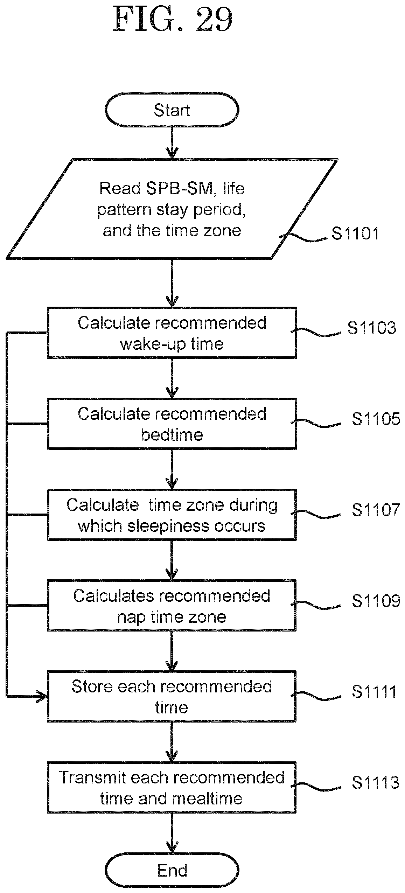

FIG. 29 is a flowchart illustrating presenting processing for a recommended action before travel based on the individual circadian rhythm in the information processing device 110D according to an embodiment of the present invention. Based on the SPB-SM and life pattern of the user, the information processing device 110D calculates an optimal wake-up time/bedtime based on the individual circadian rhythm, predicts the sleepiness timing, presents an optimal nap time, and provide the user with the living condition before travel.

The recommended time-of-day calculation unit 112Dc reads the SPB-SM and life pattern of the user, information of the stay period and the time zone at the destination, which are stored in a database 114D of the storage unit 113D (S1001). In the case where the life pattern of the user, the information of the stay period and the time zone at the destination are not stored in the database 114D, the information processing device 110D may transmit questions regarding the life pattern, stay period, and the time zone at the destination to the terminal, and may receive the answer of the user from the terminal. The information processing device 110D may prompt the user to input the destination location information by prompting the user to answer the country name of the destination or by displaying an interface in which a global map is displayed on the display device provided with a touch panel, for example.

The recommended time-of-day calculation unit 112Dc calculates the optimal wake-up time based on the read SPB-SM of the user and the required sleeping time (S1103). The recommended time-of-day calculation unit 112Dc subtracts the required sleeping time of the user from the recommended wake-up time to calculate an optimal bedtime (S1105). Further, based on the read SPB-SM of the user, the recommended time-of-day calculation unit 112Dc calculates a time zone during which sleepiness occurs (S1107). The recommended time-of-day calculation unit 112Dc calculates the recommended nap time zone based on the read SPB-SM of the user (S1109). The recommended time-of-day calculation unit 112Dc stores the calculated recommended times in the storage unit 113D (S1111). The recommended time-of-day calculation unit 112Dc may transmit each recommended time and mealtime from the communication unit 115D to the terminal via the network 50 (S1113), and the terminal may display the recommended life pattern before travel including each received recommended time and mealtime.

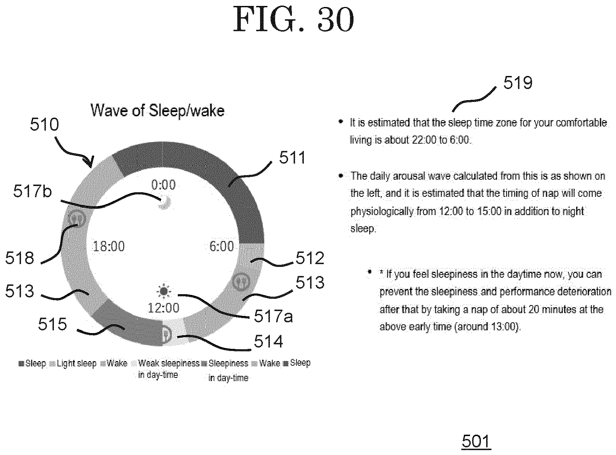

In an embodiment, these displays may be provided, for example, by an interface 501 of the terminal shown in FIG. 30. The interface 501 includes, for example, a circular indicator 510, and displays a sleep time zone 511, a light sleep time zone 512, an awaking time zone 513, a time zone 514 during which weak sleepiness occurs in the daytime, and a time zone 515 during which strong sleepiness is likely to occur in the daytime. The light sleep time zone 512 indicates a time zone from the start of the time zone during which the sleepiness occurs to the start of the time zone during which strong sleepiness is likely to occur as described in the above embodiment. Adjacent to the indicator 510, the interface 501 has an icon/illustration 517a, such as the sun, which means the day, an icon/illustration 517b, such as the moon or star, which means the night, and an icon/illustration 518, which indicates the mealtime. The interface 501 may display a comment 519 to alert the user of sleepiness and recommend a nap, and the like.

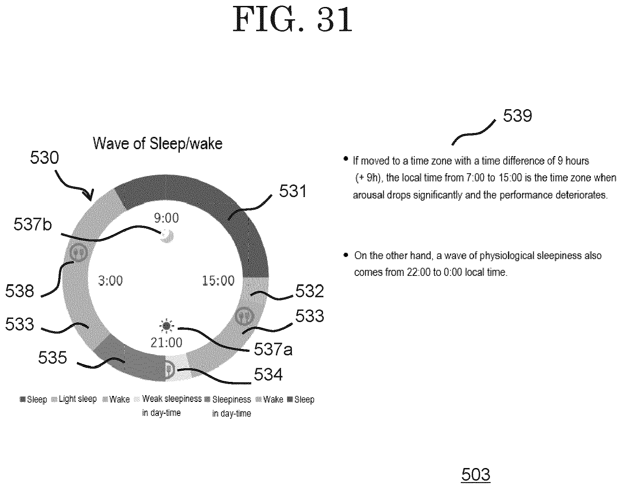

The recommended action presentation unit 112Dd can present the recommended life pattern before travel to the user together with the time sets of the time zone after the travel. In an embodiment, this may be done, for example, by an interface 503 of the terminal as shown in FIG. 31. The interface 503 includes, for example, a circular indicator 530, and displays a sleep time zone 531 before traveling, a light sleep time zone 532 before traveling, an awaking time zone 533 before traveling, a time zone 534 during which weak sleepiness occurs before traveling, and a time zone 535 during which strong sleepiness occurs before traveling, by applying them to the time display of the destination. Adjacent to the indicator 530, the interface 503 has an icon/illustration 537a, such as the sun, which means the day, an icon/illustration 537b, such as the moon or star, which means the night, and an icon/illustration 538, which indicates the mealtime. The interface 503 may display a comment 539 to alert the user of poor performance, sleepiness, or the like. As described above, in the system 100, the user can visually understand the difference between the optimal life pattern of the user before traveling and the life pattern after traveling.

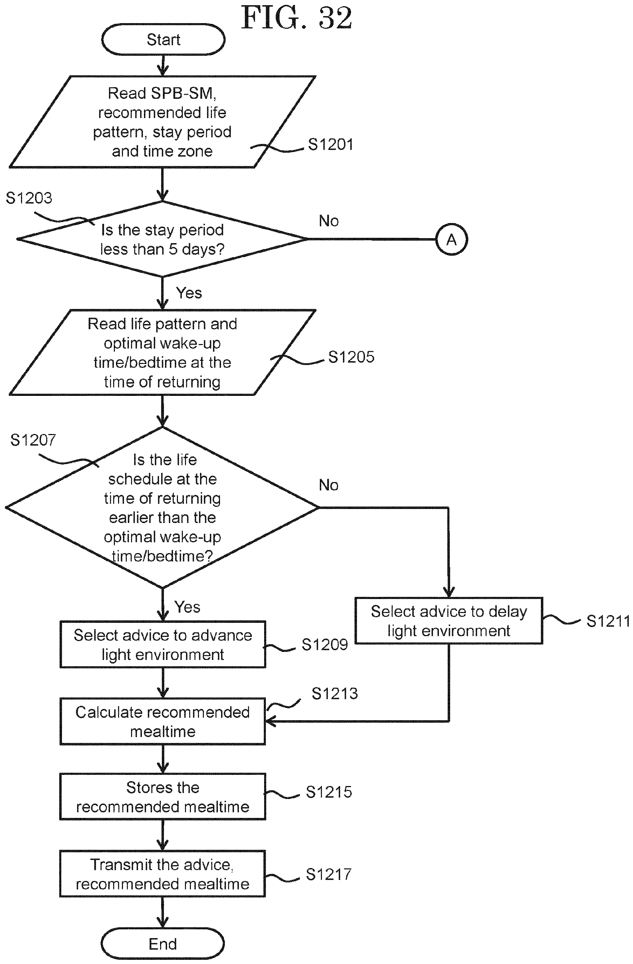

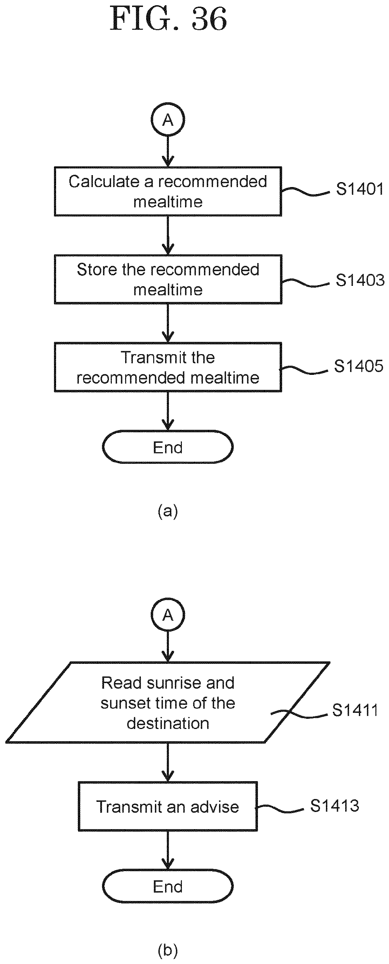

FIG. 32 is a flowchart showing presentation processing for a recommended action after travel according to an embodiment of the present invention. The recommended action presentation unit 112Dd reads the SPB-SM of user, the recommended life pattern before traveling, stay period, and data of the time zone at the destination, which are stored in the database 114D of the storage unit 113D (S1201). The recommended action presentation unit 112Dd determines whether the read stay period is less than 5 days (within 4 to 5 days) (S1203). For a short-term trip of less than 5 days, it is important to adapt to the local conditions without significantly displacing the user's biological clock at the place of departure and to return to the place of departure while maintaining that state. Therefore, the recommended action presentation unit 112Dd transmits advice for maintaining the recommended life pattern (phase of the biological clock at the place of departure) before traveling and the performance to the terminal (S1217). The recommended action presentation unit 112Dd transmits advice to the terminal to make a light environment that imitated the place of departure place as much as possible (to lighten at the time of the daytime at the place of departure and darken at the time of nighttime at the place of departure). In the case where the read stay period is 5 days or more, the recommended action presentation unit 112Dd performs the processing shown in FIG. 36.

The recommended action presentation unit 112Dd transmits a request for inputting "sleep/activity schedules assumed or desired at the time of returning to the place of departure" to the user to the terminal. When the user inputs "sleep/activity schedules assumed or desired at the time of returning to the place of departure" to the terminals such as the PC 13, a mobile phone 105, the tablet terminal 17, the wearable device 19, or the like, the communication unit 115D receives the sleep/activity schedules assumed or desired at the time of returning to the place of departure of the user from the terminal (S1205). The stay period and data of the time zone at the destination, and the sleep/activity schedules assumed or desired at the time of returning to the place of departure may be input by the user who travels to the terminal, or may be input by the service provider which sets up the system 100. In the case where the user answers the questionnaire (questionnaire 1), the person who made the user answer the question (service provider) may input the answer wrote into the questionnaire to the terminal.

The recommended action presentation unit 112Dd determines whether the schedule at the time of returning to the place of departure received from the terminal is earlier than the recommended life pattern before traveling (S1207). If the schedule at the time of returning to the place of departure is earlier than the recommended life pattern based on the circadian rhythm before traveling, the recommended action presentation unit 112Dd transmits advice to the terminal that the light environment at the destination can be advanced than that at the place of departure (S1209). On the other hand, if the schedule at the time of returning to the place of departure is later than the recommended life pattern based on the circadian rhythm before traveling, the recommended action presentation unit 112Dd transmits advice to the terminal that the light environment at the destination can be delayed than that at the place of departure (S1211). However, as described above, even if it is desired to advance the schedule of the user, the recommended action presentation unit 112Dd transmits advice to the terminal to prevent the start of the light irradiation from being earlier than SPB-SM (based on the time at the place of departure) of the user based on the examination result of the inventor on the effect of the personal biological clock and the corresponding light irradiation on the sleep-wake schedule (S1217). The information processing device 110D may be connected to a lighting device used by the user at the destination or while moving (in an airplane, etc.) via the network 50, and operate to automatically turn on, turn off, or dim the lighting at that time. As described above, the lighting control system can be configured by connecting the system 100 for presenting a daily life schedule based on the individual circadian rhythm to the lighting equipment. The light stimulation at this time may be continuous light or may be pulsed light (irradiate only for a short time once or a plurality of times). At this time, only the 420 nm to 480 nm blue light that affects the internal biological clock may be turned on, off, or dimmed.

Ingestion of food is known to be an arousal stimulus in terms of a sleep-wake rhythm. In an embodiment, the recommended action presentation unit 112Dd transmits advice to the terminal to prevent eating food around the time at which the user desires to start an activity and not just before bedtime (S1217). For this reason, the recommended action presentation unit 112Dd calculates the time at which the user desires to start an activity as mealtime, and calculates the time just before the user goes to bed as the time when meals are not recommended (S1213). The recommended time-of-day calculation unit 112Dc stores the calculated recommended mealtime in the storage unit 113D (S1215). The recommended time-of-day calculation unit 112Dc transmits the recommended mealtime from the communication unit 115D to the terminal via the network 50. Since providing a fasting time of approximately 12 hours or more increases the probability of falling asleep after the next day at that time, the recommended action presentation unit 112Dd transmits advice to the terminal to extend the meal interval before and after the time zone at which the user desires to rest and sleep at the destination and to make the meal interval at least 12 hours if feasible (S1217).

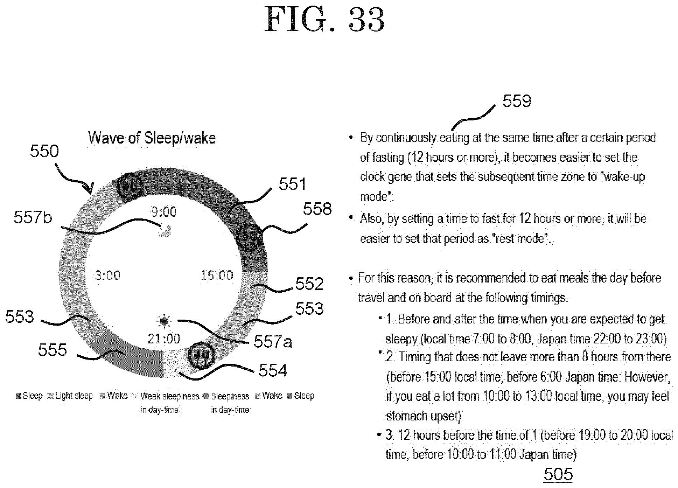

In an embodiment, these displays may be provided, for example, by an interface 505 of the terminal as shown in FIG. 33. The interface 505 includes, for example, a circular indicator 550, and displays a sleep time zone 551 before traveling, a light sleep time zone 552 before traveling, an awaking time zone 553 before traveling, a time zone 554 during which weak sleepiness occurs in the daytime before traveling, and a time zone 555 during which strong sleepiness is likely to occur in the daytime before traveling, by applying them to the time display of the destination. The interface 505 has an icon/illustration 557a, such as the sun, which means the day, an icon/illustration 557b, such as the moon or star, which means the night, and an icon/illustration 558, which indicates the mealtime adjacent to the indicator 550. The interface 505 may display a comment 559 to alert the user of poor performance, sleepiness, or the like. As described above, in the system 100, the user can visually understand the life pattern recommended after traveling based on the optimal life pattern of the user before traveling and the recommended mealtime described above.

In an embodiment, the recommended action presentation unit 112Dd transmits advice to the terminal that if the user ingests caffeine or other arousal substances, the user should ingest them in the first half of the time when the user wants the activity and not in the second half (S1217).

The recommended action presentation unit 112Dd may provide the above-described advice to the user via the terminal even when the user is moving at high speed (e.g., a passenger plane) or midway (e.g., during the stay at a relay station). For example, the recommended action presentation unit 112Dd may present the above-described advice to the user or a person providing the move/stay services to the user (airline staff, hotel staff, etc.), so that the user maintains the condition of advice described above even while the user is moving. In particular, advice regarding the advised light environment, advised meal timing, advised timing of avoidance of arousal substances such as caffeine may be provided to the user or the service provider.

In an embodiment, in the case where the calculated recommended wake-up time/bedtime (the physiological sleep time zone recommended according to the biological clock of the user) deviates from the time at which a user can rest at the destination (for example, travel between regions with a large time difference), the recommended action presentation unit 112Dd may advise the user to rest using the time zone suitable for taking a nap according to the body time shown in the embodiment described above. The recommended action presentation unit 112Dd may also present the "time estimated to be difficult to fall asleep (Sleep Forbidden Zone)" shown in the embodiment described above.

In the case where the calculated recommended wake-up time/bedtime (the physiological sleep time zone recommended according to the biological clock of the user) largely overlaps with the time at which the user can rest at the destination (e.g., traveling between regions with little time difference, traveling westward by a eveningness person, or traveling eastward by a morningness person), the recommended action presentation unit 112Dd may advise wake-up while remaining within ±2 hours of the recommended bedtime and wake-up time, respectively. The recommended action presentation unit 112Dd may also present the "time estimated to be difficult to fall asleep (Sleep Forbidden Zone)" shown in the embodiment described above.

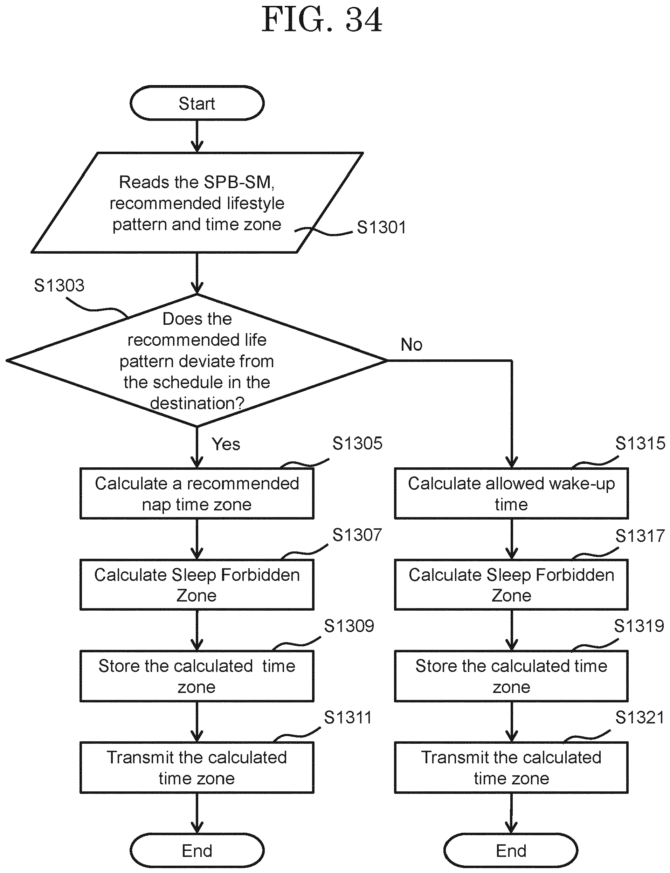

FIG. 34 is a flowchart illustrating presentation processing for a recommended action based on a difference between a recommended wake-up time/bedtime (the physiological sleep time zone recommended according to a biological clock of a user) calculated in the information processing device 110D according to an embodiment of the present invention and the time during which the user can rest at the destination. The recommended action presentation unit 112Dd reads the SPB-SM of the user, the recommended lifestyle pattern, and the data of the time zone at the destination stored in the database 114D of the storage unit 113D (S1301).

The recommended action presentation unit 112Dd determines a deviation between the recommended life pattern and schedules based on the time zone of the destination (S1303). In the case where the deviation is large, for example, in the case where the physiological sleep time zone based on the circadian rhythm does not overlap with the time zone during which resting in the destination is possible, it may be determined that the deviation is large. More specifically, in a case where 0:00 to 8:00 is a natural sleep time zone in Japan, since it is only possible to take a rest at 8:00 to 16:00 Japan time at a destination such as Europe, it may be set for this case to determine that there is a large deviation. The recommended action presentation unit 112Dd calculates the recommended nap time zone described in the above embodiment (S1305). The recommended action presentation unit 112Dd calculates the Sleep Forbidden Zone (S1307). The recommended time-of-day calculation unit 112Dc stores the calculated recommended nap time zone and Sleep Forbidden Zone in storage unit 113D (S1309). The recommended action presentation unit 112Dd may transmit the recommended nap time zone and Sleep Forbidden Zone from the communication unit 115D to the terminal via the network 50 (S1311), and the terminal may present the received recommended nap time zone and Sleep Forbidden Zone to the user.

In a case where the deviation is small, the recommended action presentation unit 112Dd calculates ±2 hours from the recommended bedtime and wake-up time as an allowable bedtime and an allowable wake-up time, respectively (S1315). The recommended action presentation unit 112Dd calculates Sleep Forbidden Zone (S1317). The recommended action presentation unit 112Dd stores the calculated allowable bedtime, allowable wake-up time, and Sleep Forbidden Zone in the storage unit 113D (S1309). The recommended action presentation unit 112Dd may transmit the allowable bedtime, allowable wake-up time, and Sleep Forbidden Zone from the communication unit 115D to the terminal via the network 50 (S1311), and the terminal may present the received allowable bedtime, allowable wake-up time, and Sleep Forbidden Zone to the user.

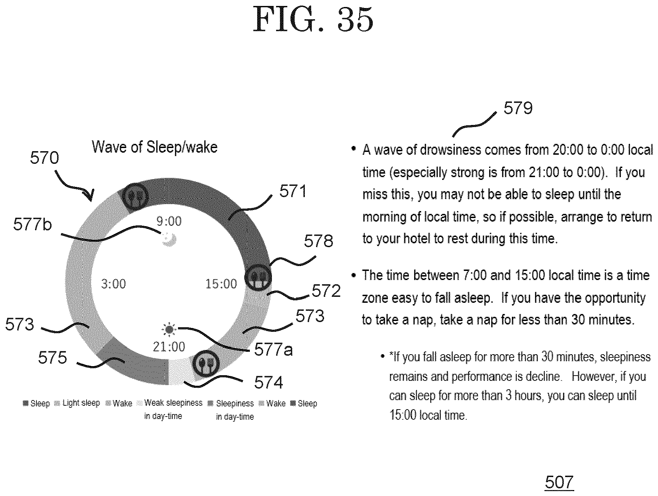

In an embodiment, these displays may be provided, for example, by an interface 507 of the terminal as shown in FIG. 35. The interface 507 includes, for example, a circular indicator 570, and displays a sleep time zone 571 before traveling, a light sleep time zone 572 before traveling, an awaking time zone 573 before traveling, a time zone 574 during which weak sleepiness occurs in the daytime before traveling, and a time zone 575 during which strong sleepiness is likely to occur in the daytime before traveling, by applying them to the time display of the destination. The interface 507 has an icon/illustration 577a, such as the sun, which means the day, an icon/illustration 577b, such as the moon or star, which means the night, and an icon/illustration 578, which indicates the mealtime adjacent to the indicator 570. The interface 505 may display a comment 579 to alert the user to recommend sleep or not to take a nap. As described above, in the system 100, the user can visually understand the life pattern recommended after traveling based on the difference between the calculated recommended wake-up time/bedtime (the physiological sleep time zone recommended according to the biological clock of user) and the time during which the user can rest at the destination.

In the processing described with reference to FIG. 32, in the case where the stay period of the user is 5 days or more, it is important to quickly tune the biological clock to the activity schedule at the destination. For this reason, it is necessary to quickly adapt the biological clock to the time of the destination and quickly recover performance.

FIG. 36 (a) is a flowchart illustrating a method of presenting a recommended time in the case where a stay period of a user is 5 days or more according to an embodiment of the present invention. A connector A of FIG. 36 (a) corresponds to the connector A shown in FIG. 32. That is, the processing S1401 of FIG. 36 (a) shows processing in the case where it is determined in the determination S1203 of FIG. 32 that the travel is a long-term travel of 5 days or more. The recommended action presentation unit 112Dd calculates the time at which the user desires to start an activity as the mealtime, and calculates the time just before the user goes to bed as the time when meals are not recommended (S1401). The recommended action presentation unit 112Dd stores the calculated recommended mealtime in the storage unit 113D (S1403). The recommended action presentation unit 112Dd transmits the recommended mealtime from the communication unit 115D to the terminal via the network 50. In addition, since providing a fasting time of approximately 12 hours or more increases the possibility of falling asleep after the next day at that time, the recommended action presentation unit 112Dd transmits advice to the terminal to extend the meal interval before and after the time zone during which the user desires to rest and sleep at the destination and to make the meal interval 12 hours or more if feasible (S1405). As described above, the recommended action presentation unit 112Dd may transmit advice to the terminal that if the user ingests caffeine or other arousal substances, the user should ingest them in the first half of the time when the user wants the activity and not in the second half.

When the chronotype or SPB-SM of the user is unknown, or when a simple countermeasure is selected, the recommended action presentation unit 112Dd may advise the user or the service provider to imitate the light environment at the destination before departure and on the move. FIG. 36 (b) is a flowchart illustrating a method of presenting a recommended time in the case where a stay period of a user is approximately 5 days or more according to an embodiment. A connector A in FIG. 36 (b) corresponds to the connector A shown in FIG. 32. That is, the processing S1411 of FIG. 36 (b) shows processing in the case where it is determined in the determination S1203 of FIG. 32 that the travel is a long-term of 5 days or more. For example, if it is assumed that a London-based user travels to New York for 5 days or more, the recommended action presentation unit 112Dd reads the sunrise and sunset time of New York (S1411), transmits the time recommended to be exposed to light from the communication unit 115D to the terminal via the network 50, and advises the user to be exposed to light in accordance with the sunrise and sunset at the time of the destination before the departure, and to be exposed to light in accordance with the sunrise and sunset at the time of the destination while the user is moving. The recommended action presentation unit 112Dd may be connected to a lighting device via the network 50 and operated to automatically turn off or dim lights or blue lights used by the user to match the light environment at the destination. As described above, the lighting control system can be configured by connecting the system 100 for presenting a daily life schedule based on the individual circadian rhythm to the lighting equipment.

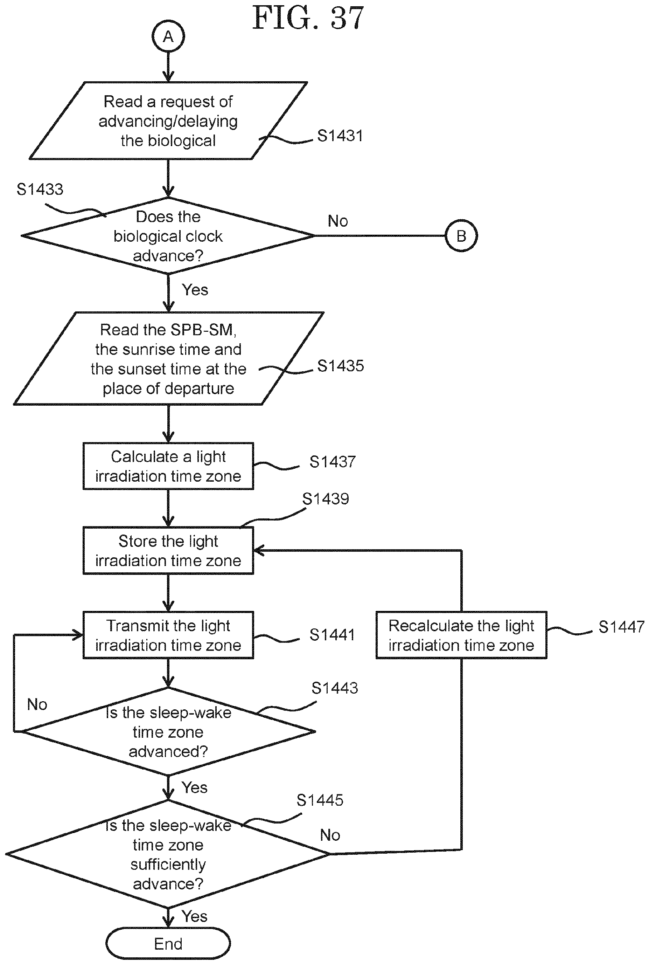

In an embodiment, in the processing method described in FIG. 32, in the case where the stay period of the user is 5 days or more, it is preferable to adjust the biological clock to the schedule at the destination more quickly according to the biological clock of the user, and to minimize the mental and physical disorder due to desynchronizing. FIG. 37 is a flowchart illustrating presentation processing for a recommended action after traveling in the case where a stay period of a user is 5 days or more according to an embodiment of the present invention. A connector A in FIG. 37 corresponds to the connector A shown in FIG. 32. That is, the processing S1431 of FIG. 37 shows processing in the case where it is determined in the determination S1203 of FIG. 32 that the travel is a long-term travel of 5 days or more.

The recommended action presentation unit 112Dd causes the user or the service provider to select via the terminal (A) whether the user wants to adapt to the destination by advancing the cycle of the biological clock earlier than 24 hours and advancing the sleep-wake schedule, or (B) whether the user wants to adapt to the destination by delaying the cycle of the biological clock later than 24 hours and retracting the sleep-wake schedule. The information processing device 110D may transmit questions regarding life patterns, stay periods, and the time zone of the destination to the terminal, and may receive the answer of the user from the terminal. The recommended action presentation unit 112Dd may read the answer of the user stored in the database 114D of the storage unit 113D in the case where the answer of the user is stored in the database 114D of the storage unit 113D (S1431).

(A) Adaptation to a destination by advancing the cycle of the biological clock earlier than 24 hours and advancing the sleep-awake schedule is recommended in the case where there is a very short eastward time lag, in the case where a morningness person (SPB-SM is about 3:00 or earlier) travels eastward, in the case where it is physically good to advance the sleep-wake schedule, or in the case where the sleep time zone desired at the destination is a short time ahead of the sleep time zone recommended by SPB-SM. (B) Adaptation to a destination by delaying the cycle of the biological clock later than 24 hours and retracting the sleep-wake schedule is recommended in the case where the travel is westward, in the case where a eveningness person (SPB-SM approximately 5:00 or later) travels eastward with a strong time lag, in the case where it is physically good to delay the sleep-wake schedule, or in the case where the sleep time zone desired at the destination is delayed compared to the sleep time zone recommended by SPB-SM. In an embodiment, the system 100 may display the recommended sentence of pattern (A) or pattern (B) on the terminal, or if the information is obtained, may set the pattern as the default based on the information.

The recommended action presentation unit 112Dd determines whether the user has selected the pattern (A) or the pattern (B) in the read answer. For example, the recommended action presentation unit 112Dd determines whether the user advances the biological clock, that is, selects the pattern (A) in the read answer (S1433). In the case where the user chooses to (A) adapt to the destination by advancing the sleep-wake schedule, the phase of the biological clock can be advanced quickly by being exposed to light at a time earlier than the sunrise time at the place of departure and later than SPB-SM, thereby synchronizing quickly to the destination time. Therefore, the recommended action presentation unit 112Dd reads the SPB-SM of the user stored in the database 114D of the storage unit 113D and reads the sunrise time and the sunset time at the place of departure via the network 50 (S1435). The recommended action presentation unit 112Dd calculates a light irradiation time zone recommended to the user based on the read SPB-SM of the user and the sunrise time and the sunset time of the place of departure (S1437). The recommended action presentation unit 112Dd stores the calculated light irradiation time zone in the storage unit 113D (S1439).

The recommended action presentation unit 112Dd transmits the light irradiation time zone from the communication unit 115D to the terminal via the network 50 (S1441), and the terminal displays the received light irradiation time zone recommended to the user. For example, the recommended action presentation unit 112Dd may transmit advice to the user or the service provider to "keep the user's surroundings bright before the sunrise time at the place of departure, but after the time of SPB-SM has elapsed". At this time, the information processing device 110D may be connected to the lighting device used by the user via the network 50 and operated to automatically turn on the light or blue light at a time earlier than the sunrise time at the place of departure and at a time later than SPB-SM. As described above, the lighting control system can be configured by connecting the system 100 for presenting a daily life schedule based on the individual circadian rhythm to the lighting equipment. The light stimulation at this time may be continuous light or may be pulsed light (irradiation only for a short time once or a plurality of times).

Further, the recommended action presentation unit 112Dd determines whether the wake-up time (sleep-wake time zone) of the user is actually advancing by subjective morningness illumination (S1443). The wake-up time of the user after presenting the light irradiation time zone may be input by the user via the terminal, or by detection by the wearable terminal or alarm stop operation time of the mobile terminal may be received by the information processing device 110D via the network 50. In the case where the sleep-wake time zone of the user is not actually advancing, the recommended action presentation unit 112Dd transmits the same light irradiation time zone to the terminal and displays it (S1441). In the case where the sleep-wake time zone of the user is actually advancing, the recommended action presentation unit 112Dd determines whether the sleep-wake time zone is sufficiently advancing to the extent that it can correspond to the schedule of the destination (S1445). In the case where the sleep-wake time zone of the user is not sufficiently advanced, the recommended action presentation unit 112Dd recalculates the SPB-SM of the user based on the advanced sleep-wake time zone, and recalculates the light irradiation time zone recommended to the user based on the recalculated SPB-SM of the user, the sunrise time at the place of departure, and the sunset time at the place of departure (S1447). The recommended action presentation unit 112Dd stores the recalculated light irradiation time zone in the storage unit 113D (S1439). By repeating processing S1439 to processing S1447, the recommended action presentation unit 112Dd can make the user adapt to the destination by advancing the sleep-wake schedule. For example, in the case where adjustments are made more than 2 to 3 days before the travel, if the sleep-wake time is actually advancing after 2 to 3 days from the start of adjustment, the timing of the light irradiation can be advanced beyond SPB-SM within the range of the advance.

Exposure to light at a time later than the sunset time at the place of departure prevents the phase advancement of the biological clock and delays the phase advancement of the biological clock. For this reason, the recommended action presentation unit 112Dd may transmit advice to the user or the service provider "not to be exposed to light after the sunset time at the place of departure". At this time, the information processing device 110D may be connected to the lighting device used by the user via the network 50 and operate to automatically turn off or dim lights or blue lights at the time of the sunset at the place of departure. As described above, the lighting control system can be configured by connecting the system 100 for presenting a daily life schedule based on the individual circadian rhythm to the lighting equipment. In addition, shading in the afternoon (after the mid-south time) at the place of departure facilitates the phase of the biological clock to advance. For this reason, the recommended action presentation unit 112Dd may transmit advice to the user or the service provider "not to be exposed to light later than the afternoon at the place of departure". The information processing device 110D may be connected to a lighting device used by the user via the network 50 and operate to automatically turn off or dim lights or blue lights at that time. As described above, the lighting control system can be configured by connecting the system 100 for presenting a daily life schedule based on the individual circadian rhythm to the lighting equipment.

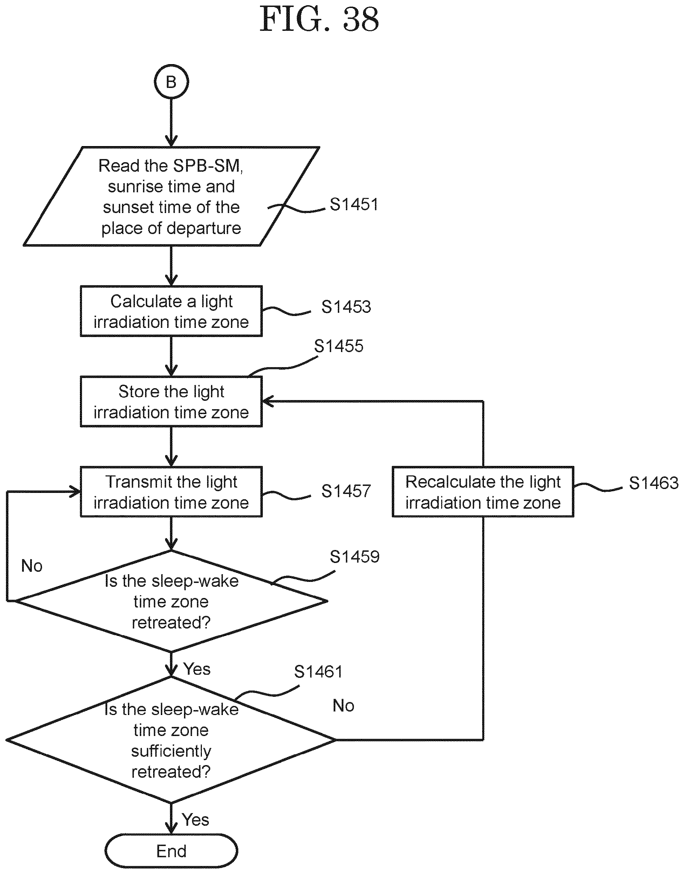

(B) Processing in the case where the biological clock is temporally extended to more than 24 hours and the sleep-wake schedule is delayed to adapt to the destination will be described. FIG. 38 is a flowchart illustrating processing according to an embodiment of the present invention in the case of delaying a biological clock cycle from 24 hours and delaying a sleep-wake schedule. A connector B in FIG. 38 corresponds to the connector B shown in FIG. 37. That is, the processing S1451 of FIG. 38 shows processing in the case where it is determined in the determination S1433 of FIG. 37 that the user has advanced the biological clock, that is, the pattern (A) has not been selected.

In the case of choosing that (B) the biological clock is temporally extended to more than 24 hours and delaying the sleep-wake schedule to adapt to the destination, the phase of the biological clock is prompted to delay by shading from SPB-SM to the mid-south time (in the morning) and by irradiating light from the sunset to SPB-SM (in the first half of the night). Therefore, the recommended action presentation unit 112Dd reads the SPB-SM of user stored in the database 114D of the storage unit 113D and reads the sunrise time and the sunset time of the place of departure via the network 50 (S1451). The recommended action presentation unit 112Dd calculates the light irradiation time zone recommended to the user based on the read SPB-SM of the user, the sunrise time at the place of departure, and the sunset time at the place of departure (S1453). The recommended action presentation unit 112Dd stores the calculated light irradiation time zone in the storage unit 113D (S1455).

The recommended action presentation unit 112Dd transmits the light irradiation time zone from the communication unit 115D to the terminal via the network 50 (S1457), and the terminal displays the received light irradiation time zone recommended to the user. For example, the recommended action presentation unit 112Dd advises the user or service provider to shade and to be exposed to light at times from SPB-SM to the mid-south time and from after sunset to SPB-SM. At this time, the information processing device 110D may be connected to the lighting device used by the user via the network 50, and may operate to automatically turn on, turn off, and dim lights or blue lights at the times from SPB-SM to the mid-south time and from after the sunset to SPB-SM. As described above, the lighting control system can be configured by connecting the system 100 for presenting a daily life schedule based on the individual circadian rhythm to the lighting equipment.

Further, the recommended action presentation unit 112Dd determines whether the wake-up time (sleep-wake time zone) of the user actually delays (S1459). The wake-up time of the user after presenting the light irradiation time zone may be input by the user via the terminal, or by detection by the wearable terminal or alarm stop operation time may be received by the information processing device 110D via the network 50. In the case where the sleep-wake time zone of the user is not actually delaying, the recommended action presentation unit 112Dd transmits the same light irradiation time zone to the terminal and display it (S1457). In the case where the sleep-wake time zone of the user is actually delaying, the recommended action presentation unit 112Dd determines whether the sleep-wake time zone is sufficiently delaying to the extent that it can correspond to the schedule of the destination (S1461). In the case where the sleep-wake time zone of the user is not sufficiently delayed, the recommended action presentation unit 112Dd recalculates the SPB-SM of the user based on the delayed sleep-wake time zone, and recalculates the light irradiation time zone recommended to the user based on the recalculated SPB-SM of the user, the sunrise time of the place of departure, and the sunset time of the place of departure (S1463). The recommended action presentation unit 112Dd stores the recalculated light irradiation time zone in the storage unit 113D (S1455). By repeating processing S1455 to processing S1463, the recommended action presentation unit 112Dd can make the user adapt to the destination by delaying the sleep-wake schedule. For example, in the case where adjustments are made more than 2 to 3 days before travel, if the sleep-wake time is actually delaying after 2 to 3 days from the start of the adjustment, the timing of the light irradiation can be delayed beyond SPB-SM within the range of delay.

### [Method for presenting optimal eating time for preventing obesity and hyperglycemia based on individual circadian rhythm].

Hypertension, hyperlipidemia, abnormal glucose tolerance, and diabetes mellitus, myocardial infarction, and cerebral infarction caused by obesity and metabolic syndrome are major public health problems. On the other hand, it is known that circadian rhythm exists in lipocyte and adipose tissue and insulin secretion capacity and glucose tolerance. For example, it is known that lipocytes have higher synthetic capacity and less insulin-secretion at night, and conversely, in the morning, lipocytes have less activity and higher insulin-secretion. It is also known that disturbance of wake-up rhythm leads to obesity and glucose intolerance. For example, see, the following references.

GIMBLE, Jeffrey M., et al. Circadian rhythms in adipose tissue: an update. Current Opinion in Clinical Nutrition & Metabolic Care, 2011, 14.6: 554-561.

KIEHN, Jana - Thabea, et al. Circadian rhythms in adipose tissue physiology. Comprehensive Physiology, 2011, 7.2: 383-427.

BODEN, Guenther, et al. Evidence for a circadian rhythm of insulin secretion. American Journal of Physiology-Endocrinology And Metabolism, 1996, 271.2: E246-E252.

Shi, S. Q., Ansari, T. S., McGuinness, O. P., Wasserman, D. H., & Johnson, C. H. (2013). Circadian disruption leads to insulin resistance and obesity. Current biology : CB, 23(5), 372-81.

ROENNEBERG, Till, et al. Social jetlag and obesity. Current Biology, 2012, 22.10: 939-943.

On the other hand, although existing studies have roughly shown that "eating early in the morning during the day is unlikely to cause obesity" and "eating late at night during the day is likely to cause obesity," it is not clear at all what time it is and how much there is a difference between individuals.

The inventor of the present invention investigated the body weight variation according to the circadian rhythm and the eating time based on the circadian rhythm by the cohort study, and revealed that there are different mealtimes which are likely to lead to body weight variation and an increase/decrease of body fat depending on the individual.

Also, it was clarified that there exists time in which the blood glucose level is difficult to rise and time in which it is easy to rise respectively by the individual by carrying out a carbide load of a fixed quantity for the individual in which the subjective midnight time differed to measure the blood glucose level fluctuation.

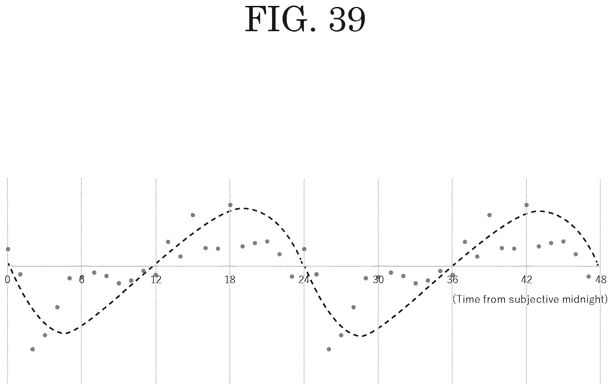

FIG. 39 is a diagram showing an analysis result in which the influence of a normal subjective mealtime on body weight fluctuation is adjusted by covariance structure analysis for the correlation between mealtimes. In FIG. 39, the values of the standardization coefficient adjusted for the correlation of each variable by covariance structure analysis were plotted. The covariance structural analysis revealed that R square of the heavy-correlation coefficients of weight gain level was 14%, which was significantly higher than 7% based on the absolute time, and that the effects of mealtime on weight gain were not absolute time but relative time.

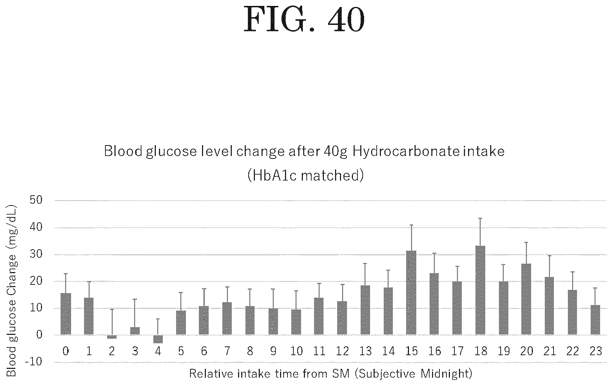

FIG. 40 is a diagram showing the difference between the blood glucose level at the time of ingestion and the blood glucose level 1 hour after ingestion of 40 g of carbohydrate (such as rice ball) at any time in one day (the horizontal axis represents the relative time at the time of ingestion). Also in FIG. 40, although there was no significant difference (p < 0.05) when the analysis was performed at the absolute time, a significant difference was found as a result of analysis at a relative time based on the subjective midnight.

As described above, it became clear that obesity, weight gain, increase in body fat, and hyperglycemia are strongly influenced by "when the person eats meals in light of the biological clock". Investigations by the inventor have revealed that irregularities in the time of meal (variations in eating time and standard deviation of time in a day) also cause obesity, weight gain, increased body fat, and hyperglycemia. In an embodiment, a method for presenting an optimal eating time for preventing obesity, hyperglycemia, weight gain, and body fat weight gain is provided.

Social Jet Lag (difference between sleep time zone on weekdays and holidays) is known to cause obesity and hyperglycemia (ROENNEBERG, Till, et al. Social jetlag and obesity. Current Biology, 2012, 22.10: 939-943.). Furthermore, the investigations by the inventor have shown that a sleep-wake schedule deviating from the original internal rhythm of the person also risks weight gain and diabetes development. Thus, in an embodiment, the system 100 for presenting a daily life schedule based on the individual circadian rhythm also provides advice regarding sleep time zones.

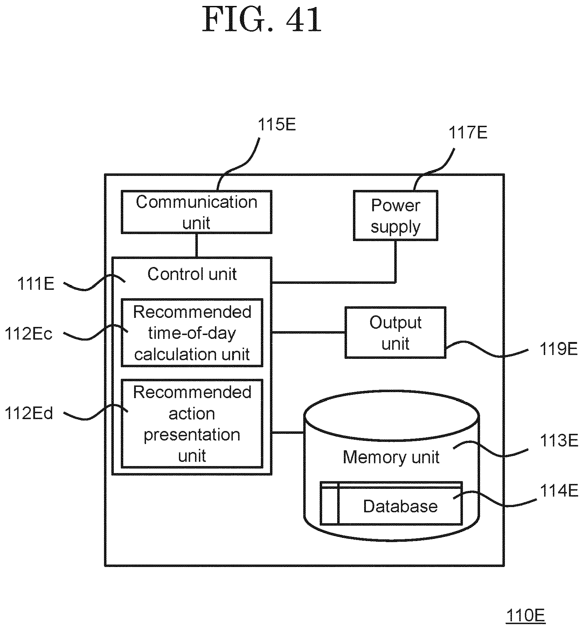

FIG. 41 is a block diagram illustrating an information processing device 110E according to an embodiment of the present invention. In the present embodiment, the system 100 includes the information processing device 110E in place of the information processing device 110 to present an optimal eating time for preventing obesity and hyperglycemia based on the individual circadian rhythm. The information processing device 110E includes, for example, a control unit 111E, a storage unit 113E, a communication unit 115E and a power supply 117E, and may further include an output unit 119E. The basic configuration of control unit 111E is similar to that of the control unit 111, but differs from that of the control unit 111 in that it includes a recommended time-of-day calculation unit 112Ec for presenting an optimal eating time for preventing obesity and hyperglycemia based on the individual circadian rhythm, and a recommended action presentation unit 112Ed for presenting an optimal eating time for preventing obesity and hyperglycemia based on the individual circadian rhythm. The configurations of the storage unit 113E, the communication unit 115E, and the output unit 119E may be the same as those of the storage unit 113, the communication unit 115, and the output unit 119, respectively, and detailed descriptions thereof are omitted. In addition, the information processing device 110E may include various types of electronic devices included in a known information processing device.

The recommended time-of-day calculation unit 112Ec is composed of an application program or module or the like that calculates an optimal eating time for preventing obesity and hyperglycemia of the user, based on the SPB-SM of the user or the intermediate time of the original physiological sleep time zone (hereinafter, also referred to as SPB-SM for convenience) obtained by other methods. The recommended time-of-day calculation unit 112Ec may be, for example, an application program or module stored in the storage unit 113E and executed by the control unit 111E. The recommended time-of-day calculation unit 112Ec may calculate the optimal eating time for preventing obesity and hyperglycemia of the user by reading SPB-SM of the user stored in the storage unit 113E or the intermediate time of the original physiological sleep time zone obtained by other methods, or may calculate SPB-SM of the user as in the above-described SPB-SM calculation unit 112b.

The recommended action presentation unit 112Ed is composed of an application program or module or the like that presents an optimal action time for preventing obesity and hyperglycemia based on the individual circadian rhythm. The recommended action presentation unit 112Ed may be, for example, an application program or module stored in the storage unit 113E and executed by the control unit 111E.

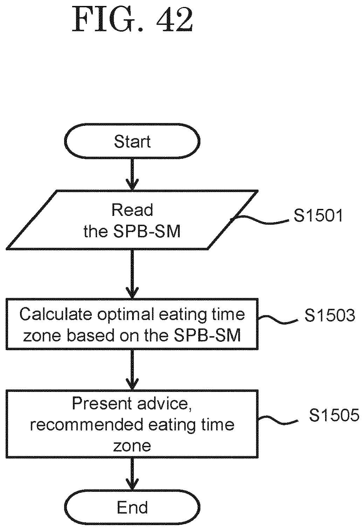

FIG. 42 is a flowchart illustrating calculation processing for an optimal eating time in the information processing device 110E according to an embodiment of the present invention. The recommended time-of-day calculation unit 112Ec reads the SPB-SM of the user stored in a database 114E of the storage unit 113E (S1501). The recommended time-of-day calculation unit 112Ec may calculate SPB-SM of the user as in the SPB-SM calculation unit 112b.

The recommended time-of-day calculation unit 112Ec calculates a first recommended eating time zone during which the influence on weight gain is small and an increase in the blood glucose level is relatively suppressed by adding a ninth reference value having a predetermined time range to SPB-SM of the user. The ninth reference value is in a range of 1 to 12 hours, and the first recommended eating zone is calculated as SPB-SM +1 to 12 hours. A first non-recommended eating time zone during which eating significantly increases body weight and body fat and tends to raise the blood glucose level to a higher degree is calculated by adding a tenth reference value in a time range later than the ninth reference value to SPB-SM (S1503). The tenth reference value is in a range of 13 to 24 hours, and the first non-recommended eating time zone is calculated as SPB-SM +13 to 24 hours. The recommended time-of-day calculation unit 112Ec advises that eating should be carried out between SPB-SM +1 to 12 hours, and that eating should be avoided or conserved between SPB-SM +13 to 24 hours (1505).

Even during the first non-recommended eating time zone, particularly, eating between SPB-SM +2 to 4 hours, has minimal effect on body weight gain and produces little or no excessive increase in blood glucose levels. Therefore, the recommended time-of-day calculation unit 112Ec may present SPB-SM +2 to 4 hours as a second recommended eating time zone which is optimal for the first meal in the day by making the range of 2 to 4 hours as an eleventh reference value. This time is often equivalent to breakfast.

Since eating at any time at SPB-SM +13 to 24 hours significantly affects weight gain and a blood glucose level increase, the recommended time-of-day calculation unit 112Ec may present SPB-SM +13 to 24 hours as "mealtime requiring attention for weight gain and blood glucose level increase". On the other hand, there are times when the effect on the body weight gain, body fat weight gain, and blood sugar level increase is rather small in SPB-SM +16 to 17 hours, SPB-SM +19 hours, and SPB-SM +23 hours. Therefore, the recommended time-of-day calculation unit 112Ec may present SPB-SM +16 to 17 hours, SPB-SM +19 hours, and SPB-SM +23 hours as the "time zone during which the effect on obesity and hyperglycemia is relatively weak when it is unavoidable to eat" (a third recommended eating time zone) using the ranges of 16 to 17 hours, 19 hours, and 23 hours as a twelfth reference value.

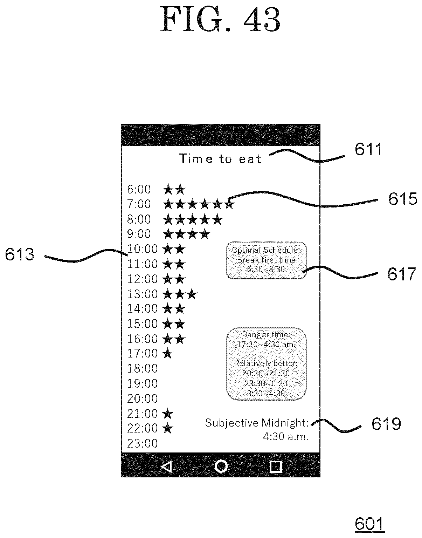

In an embodiment, these displays may be provided, for example, by an interface 601 as shown in FIG. 43. The interface 601 may include, for example, a title 611, a time display 613, an icon 615, a display for calling attention 617, and an SPB-SM display 619. The title 611 may clarify that the interface 601 presents the user with the optimal eating time for obesity and hyperglycemia prevention. The time display 613 and the icon 615 visually display the first eating recommended time zone, the second eating recommended time zone, the third eating recommended time zone, and the first non-recommended eating time zone calculated by the recommended time-of-day calculation unit 112Ec by the number of icons 615 arranged for the time display 613. The time display 613 and the icon 615 may be realized by other known display means such as a bar graph with respect to the time axis. The display for calling attention 617 may display, for example, a time zone during which eating significantly increases body weight and blood glucose levels also tend to increase to a higher degree to alert the user. The SPB-SM display 619 displays SPB-SM of the user used to calculate the recommended eating time zone. The interface 601 may include other known displays for presenting to the user the recommended eating time zone.

Glucose and lipids have a major effect on body weight gain, and glucose has a major effect on blood glucose levels. Therefore, in a case where the user needs to eat at a time other than SPB+1 to 12 hours, SPB-SM +16 to 17 hours, SPB-SM +19 hours, or SPB-SM +23 hours, the recommended time-of-day calculation unit 112Ec may present that carbohydrates and lipids should be conserved and that proteins and vegetables and the like are preferably ingested.

In addition to the relative time of eating, variations in the time of eating also significantly affects weight gain and increased blood glucose levels. Therefore, the recommended time-of-day calculation unit 112Ec preferable presents advice to eat at the same time every day, even if the user can or cannot select the mealtime from among the times presented above.

A person who provides meals to customers as a business, such as hospitals, accommodation facilities, or transportation facilities can provide meals to the users at optimal timings by displaying a list of mealtimes being desirable for the users by the recommended time-of-day calculation unit 112Ec.

Social Jet Lag (difference between sleep time zone on weekdays and holidays) and the sleep-wake schedule deviating from the person's original physiological sleep time zone themselves cause obesity and hyperglycemia. Therefore, the recommended action presentation unit 112Ed presents an optimal sleep time zone based on the individual circadian rhythm described in the above embodiment, and when it is desired to prevent weight gain and hyperglycemia, the recommended action presentation unit 112Ed can present to follow the optimal sleep time zone or sleep and wake-up at a certain time every day as much as possible even if the optimal sleep time zone deviates.

### REFERENCE SIGNS LIST

1: questionnaire, 2: sensor mat, 11: server, 13: personal computer (PC), 15: mobile phone, 17: tablet terminal, 19: wearable device, 50: network, 100: system, 110: information processing device, 110A: information processing device, 110B: information processing device, 110C: information processing device, 110D: information processing device, 110E: information processing device, 111: control unit, 111A: control unit, 111B: control unit, 111C: control unit, 111D: control unit, 111E: control unit, 112a: data exclusion unit, 112Ac: recommended time-of-day calculation unit, 112b: SPB-SM calculation unit, 112Bc: recommended time-of-day calculation unit, 112c: recommended time-of-day calculation unit, 112Cc: recommended time-of-day calculation unit, 112Dc: recommended time-of-day calculation unit, 112Dd: recommended action presentation unit, 112Ec: recommended time-of-day calculation unit, 112Ed: recommended action presentation unit, 113: storage unit, 113A: storage unit, 113B: storage unit, 113C: storage unit, 113D: storage unit, 113E: storage unit, 114: database, 114A: database, 114B: database, 114C: database, 114D: database, 114E: database, 115: communication unit, 115A: communication unit, 115B: communication unit, 115C: communication unit, 115C: communication unit, 115D: communication unit, 115E: communication unit, 117: power supply, 117A: power supply, 117B: power supply, 117C: power supply, 117D: power supply, 117D: power supply, 117E: power supply, 119: output unit, 119A: output unit, 119B: output unit, 119C: output unit, 119C: output unit, 119D: output unit, 119E: output unit, 301: interface, 303: interface, 305: interface, 307: interface, 310: indicator, 311: slider, 311a: icon, 311b: icon, 313a: icon/illustration, 313b: icon/illustration, 330: indicator, 331: slider, 331a: icon, 331b: icon, 333a: icon/illustration, 333b: icon/illustration, 350: indicator, 351: slider, 351a: button and icon, 353: icon/illustration, 355: icon/illustration, 370: indicator, 371: bar, 371a: mark, 371b: mark, 373a: icon/illustration, 373b: icon/illustration, 401: interface, 403: interface, 411: title, 413: title display, 415: icon, 417: display for calling attention, 419: SPB-SM display, 431: title, 433: time display, 435: icon, 437: recommended time slot display, 439: non-recommended time slot display, 501: interface, 503: interface, 505: interface, 507: interface, 510: indicator, 511: sleep time zone, 512: sleep time zone, 513: awaking time zone, 514: time zone, 515: time zone, 517a: icon/illustration, 517b: icon/illustration, 518: icon/illustration, 519: comment, 530: indicator, 531: sleep time zone, 532: sleep time zone, 533: awaking time zone, 534: time zone, 535: time zone, 537a: icon/illustration, 537b: icon/illustration, 538: icon/illustration, 539: comment, 550: indicator, 551: sleep time zone, 552: sleep time zone, 553: awaking time zone, 554: time zone, 555: time zone, 557a: icon/illustration, 557b: icon/illustration, 558: icon/illustration, 559: comment, 570: indicator, 571: sleep time zone, 572: sleep time zone, 573: sleep time zone, 574: time zone, 575: time zone, 577a: icon/illustration, 577b: icon/illustration, 579: comment, 601: interface, 611: title, 613: time display, 615: icon, 617: display for calling attention, 619: SPB-SM display

## Claims

1. A system for presenting a daily life schedule based on circadian rhythm comprising:
an information processing device having a subjective midnight calculation unit, the subjective midnight calculation unit calculating a subjective midnight;
wherein the information processing device determines, on the basis of an answer to a question based on a sleep time zone, or measured data, whether time-of-day data included in the answer or the measured data includes time-of-day data to be excluded;
the information processing device calculates a subjective midnight on the basis of the sleep time zone based on the time-of-day data included in the answer or measured data, if the time-of-day data to be excluded is not included; and
the information processing device calculates a subjective midnight on the basis of the sleep time zone based on the time-of-day data included in the answer or measured data in which the time-of-day to be excluded has been excluded from the time-of-day data included in the answer or measured data, if the time-of-day data to be excluded is included.

2. The system for presenting the daily life schedule based on the circadian rhythm according to claim 1,
wherein the information processing device sets a flag if exception data is included in the answer or measured data, and
the information processing device calculates the subjective midnight on the basis of the time-of-day data included in the answer or measured data which includes the exception data.

3. The system for presenting the daily life schedule based on the circadian rhythm according to claim 1,
wherein the information processing device determines whether a time-of-day at which the least number of people fall asleep corresponding to the user's age is included in a range of the answer to the question based on a sleeping time zone, and
the information processing device issues a warning if the information processing device determines that the time-of-day at which the least number of people fall asleep is included in the answer.

4. The system for presenting the daily life schedule based on the circadian rhythm according to claim 3,
wherein the information processing device calculates and updates the time-of-data at which the least number of people fall asleep in the answer by performing aggregation using the answer or measured data.

5. The system for presenting the daily life schedule based on the circadian rhythm according to claim 1,
wherein the information processing device determines which condition the answer for conditions corresponding to a plurality of calculation methods for calculating a required sleeping time applies to, and
the information processing device calculates the required sleeping time on the basis of the calculation method corresponding to the conditions applied to the answer.

6. The system for presenting the daily life schedule based on the circadian rhythm according to claim 5, comprising:
calculating a recommended wake up time by adding half of the required sleeping time to the subjective midnight;
calculating a recommended bedtime by subtracting the required sleeping time from the recommended wake up time; and
calculating a sleep forbidden zone by adding a predetermined time to the subjective midnight.

7. The system for presenting the daily life schedule based on the circadian rhythm according to claim 6, comprising:
calculating a recommended activity time for taking action for promoting falling asleep by adding or subtracting a predetermined reference value using the subjective midnight, the required sleeping time, the recommended wake up time, or the recommended bedtime.

8. The system for presenting the daily life schedule based on the circadian rhythm according to claim 7,
wherein the information processing device calculates the predetermined reference value by performing aggregation or regression analysis using the answer or measured data.

9. The system for presenting the daily life schedule based on the circadian rhythm according to claim 1,
wherein the information processing device calculates a first sleepiness time zone in which sleepiness may occur by adding a first reference value having a predetermined time range at the subjective midnight,
the information processing device calculates a second sleepiness time zone in which strong sleepiness may occur by adding a second reference value having a narrower time range than the first reference time to the subjective midnight, and
the information processing device calculates a third sleepiness time zone which is likely to be a peak of sleepiness by adding a third reference value having a narrower time range than the second reference time to the subjective midnight.

10. The system for presenting the daily life schedule based on the circadian rhythm according to claim 9,
wherein the information processing device calls attention if sleep deprivation, agrypnia, or hypersomnia is determined based on the answer or measured data.

11. The system for presenting the daily life schedule based on the circadian rhythm according to claim 9,
wherein the information processing device calculates and updates the first reference value, the second reference value, and the third reference value by performing aggregation or regression analysis using the answer or measured data.

12. The system for presenting the daily life schedule based on the circadian rhythm according to claim 9,
wherein the information processing device calculates a time zone from the time-of-day of the subjective midnight to a predetermined time as a recommended nap time zone.

13. The system for presenting the daily life schedule based on the circadian rhythm according to claim 1,
wherein the information processing device determines whether an activity time zone is fixed to a general activity time zone,
the information processing device calculates a first activity time zone at which physical and mental performance improves in a first half of a day by adding a fourth reference value having a predetermined time range to the subjective midnight, if the activity time zone is fixed to the general activity time zone, and
the information processing device calculates a second activity time zone at which physical and mental performance improves in a second half of the day by adding a fifth reference value which is set at a late time in a predetermined time range from the fourth reference value to the subjective midnight, and
the information processing device calculates a time zone between the first activity time zone and the second activity time zone as a recommended break time zone.

14. The system for presenting the daily life schedule based on the circadian rhythm according to claim 13,
wherein the information processing device calculates a recommended start time at which the physical and mental performance improves by adding a sixth reference value having a predetermined time range to the subjective midnight, if the activity time zone is not fixed to a general activity time zone.

15. The system for presenting the daily life schedule based on the circadian rhythm according to claim 1,
wherein the information processing device calculates a time zone in which a high efficacy rate of a sedative-hypnotic psychotropic drug is expected by adding a seventh reference value having a predetermined time range to the subjective midnight,
the information processing device calculates a time zone of taking medicine with few side effects by adding an eighth reference value that is set in a narrow time range within a predetermined time range from the seventh reference value to the subjective midnight, and
the information processing device calculates a first recommended time zone based on the time zone in which the high efficacy rate is expected and the time zone of taking medicine with few side effects.

16. The system for presenting the daily life schedule based on the circadian rhythm according to claim 15,
wherein the information processing device determines whether the sedative-hypnotic psychotropic drug is an immediate release tablet,
the information processing device determines whether the sedative-hypnotic psychotropic drug is a delayed-action preparation, if the sedative-hypnotic psychotropic drug is not the immediate release tablet,
the information processing device calculates a second recommended time zone by subtracting a duration until the blood concentration or brain concentration of the drug being taken reaches an effective range or time until sleepiness actually appears after taking the drug from the first recommended time zone, if the sedative-hypnotic psychotropic drug is the delayed-action preparation, and
the information processing device calculates the second recommended time zone by subtracting a duration until the blood concentration or brain concentration of the drug being taken reaches an effective range or time until sleepiness actually appears after taking the drug from the first recommended time zone, if the sedative-hypnotic psychotropic drug is not the delayed-action preparation.

17. The system for presenting the daily life schedule based on the circadian rhythm according to claim 6,
wherein the information processing device determines whether a stay period to an area with a time difference is less than 5 days,
the information processing device determines whether the recommended wake up time and the recommended bedtime before making a voyage are earlier than a wakeup time and a bedtime when returning to a home country, if the stay period is less than 5 days,
the information processing device provides advice that the light environment at the destination may be earlier than that at a place of departure, if the recommended wake up time and the recommended bedtime before making a voyage are earlier than the wake-up time and the bedtime when returning to the home country, and
the information processing device provides advice that the light environment at the destination may be later than that at the place of departure, if the recommended wake up time and the recommended bedtime before making a voyage are later than the wake-up time and the bedtime when returning to the home country.

18. The system for presenting the daily life schedule based on the circadian rhythm according to claim 17,
wherein the information processing device calculates a time to desire to start an activity as mealtime, calculates a time just before bedtime as a time when meals are not recommended, and sets a certain fasting time in the time zone to desire to sleep.

19. The system for presenting the daily life schedule based on the circadian rhythm according to claim 17,
wherein the information processing device determines whether there is a large deviation between the recommended wake up time and the recommended bedtime before making a voyage and the recommended wake up time and the recommended bedtime at the destination,
the information processing device calculates a first sleepiness time zone by adding a first reference value having a predetermined time range to the subjective midnight, if the deviation is determined to be large,
the information processing device calculates a time from the start time of the first sleepiness time zone to a predetermined time as a recommended nap time zone, and
the information processing device calculates a sleep forbidden zone by adding a predetermined time to the subjective midnight.

20. The system for presenting the daily life schedule based on the circadian rhythm according to claim 19,
wherein the information processing device calculates a time in a range ±2 hours from each of the recommended bedtime and wake up time as an allowable bedtime and wake up time, if the deviation is determined to be small.

21. The system for presenting the daily life schedule based on the circadian rhythm according to claim 17,
wherein the information processing device inquires to a user whether it makes the body clock cycle shorter than 24 hours, if the stay period is 5 days or more,
the information processing device calculates a recommended light irradiation time zone based on the subjective midnight and sunrise and sunset times at the place of departure, if it is determined that making the body clock cycle shorter than 24 hours is selected,
the information processing device determines whether an actual sleep-wake time zone is advanced by the light irradiation of a subjective morningness,
the information processing device determines whether the sleep-wake time zone is advanced enough to a range which can accommodate a schedule at the destination, if the sleep-wake time zone is actually advanced,
the information processing device re-calculates the subjective midnight based on the advanced sleep-wake time zone until the sleep-wake time zone is advanced enough, and
the information processing device re-calculates the recommended light irradiation time zone based on the re-calculated subjective midnight and the sunrise and sunset times at the place of departure.

22. The system for presenting the daily life schedule based on the circadian rhythm according to claim 21,
wherein the information processing device calculates the recommended light irradiation time zone based on the subjective midnight and the sunrise and sunset times at the place of departure to recommend shading from the subjective midnight to sun meridian transit time and light irradiation until the subjective midnight after the sunset, if it is determined that the body clock cycle is longer than 24 hours;
information processing device determines whether the actual sleep-wake time zone is delayed;
the information processing device determines whether the sleep-wake time zone is delayed enough to the range which can accommodate the schedule at the destination, if the sleep-wake time zone is actually delayed;
the information processing device re-calculates the subjective midnight based on the delayed sleep-wake time zone until the sleep-wake time zone is delayed enough; and
the information processing device re-calculates the recommended light irradiation time zone based on the re-calculated subjective midnight and the sunrise and sunset times at the place of departure.

23. The system for presenting the daily life schedule based on the circadian rhythm according to claim 1,
wherein the information processing device calculates a first recommended eating time zone which has little effect on body weight increase and relatively suppresses an increase in blood glucose levels by adding a ninth reference value having a predetermined time range to the subjective midnight; and
the information processing device calculates a first non-recommended eating time zone which significantly increases body weight and tends to increase blood glucose levels to a higher degree by adding a tenth reference value of a later time range than the ninth reference value to the subjective midnight.

24. The system for presenting the daily life schedule based on the circadian rhythm according to claim 23,
wherein the information processing device calculates a second recommended eating time zone which minimizes effects on body weight increase and almost no excessive increase in blood glucose levels by adding an eleventh reference value having a predetermined time range to the subjective midnight.

25. The system for presenting the daily life schedule based on the circadian rhythm according to claim 23,
wherein the information processing device calculates a third recommended eating time zone in which eating has little effect on body weight increase and increase in blood glucose levels by adding a twelfth reference value having a predetermined time range to the subjective midnight.
